# EUROPÄISCHE PATENTSCHRIFT

(11) **EP 1 003 736 B1**
(45) Veröffentlichungstag und Bekanntmachung des Hinweises auf die Patenterteilung: **12.04.2006**
(21) Anmeldenummer: 98942572.3
(22) Anmeldetag: 20.07.1998
(51) Int. Cl.: C07D 401/10, A01N 43/56, A01N 43/74, A01N 43/08, A01N 43/40, C07D 405/10, C07D 213/30, C07D 307/42, C07D 307/58, C07D 413/10, C07D 261/08

(54) **SUBSTITUIERTE 4-BENZOYL-PYRAZOLE**
SUBSTITUTED 4-BENZOYL-PYRAZOLES
4-BENZOYL-PYRAZOLES SUBSTITUES

(30) Priorität: 07.08.1997 DE 19734186
(43) Veröffentlichungstag der Anmeldung: 31.05.2000
(73) Patentinhaber: BASF Aktiengesellschaft, 67056 Ludwigshafen (DE)
(72) Erfinder: ENGEL, Stefan, D-55286 Wörrstadt (DE); RHEINHEIMER, Joachim, D-67063 Ludwigshafen (DE); BAUMANN, Ernst, D-67373 Dudenhofen (DE); VON DEYN, Wolfgang, D-67435 Neustadt (DE); HILL, Regina, Luise, D-67346 Speyer (DE); MAYER, Guido, D-67433 Neustadt (DE); MISSLITZ, Ulf, D-67433 Neustadt (DE); WAGNER, Oliver, D-67061 Ludwigshafen (DE); WITSCHEL, Matthias, D-67061 Ludwigshafen (DE); OTTEN, Martina, D-67069 Ludwigshafen (DE); WALTER, Helmut, D-67283 Obrigheim (DE); WESTPHALEN, Karl-Otto, D-67346 Speyer (DE)
(86) Internationale Anmeldenummer: PCT/EP1998/004481
(87) Internationale Veröffentlichungsnummer: WO 1999/007697

(56) Entgegenhaltungen:
- EP-A- 0 282 944
- WO-A-96/26206
- FR-A- 2 053 017
- BOHLMANN F ET AL: "Synthesis of naturally occuring hydroxyacetophenone derivatives" CHEM. BER. (CHBEAM);72; VOL.105 (3); PP.863-73, XP002088289 Tech. Univ. Berlin;Org.-Chem. Inst.; Berlin; Ger.
- CHEMICAL ABSTRACTS, vol. 076, no. 15, 1972 Columbus, Ohio, US; abstract no. 085658, SAMULA K: "Condensation of acetylbenzene derivatives with pyridine aldehydes" Seite 405; Spalte 1; XP002088291 & ROCZ. CHEM. (ROCHAC);71; VOL.45 (11); PP.1833-40, Inst. Farm.;Warsaw; Pol.
- MIYANO S ET AL: "Nucleophilic addition of phenols to N-(2-pyridylmethylene)aniline" TETRAHEDRON LETT. (TELEAY);70; (22); PP.1909-12, XP002088290 Fukuoka Univ.;Dep. Pharm. Sci.; Fukuoka; Japan

## Beschreibung

Die vorliegende Erfindung betrifft substituierte 4-Benzoylpyrazole der Formel I 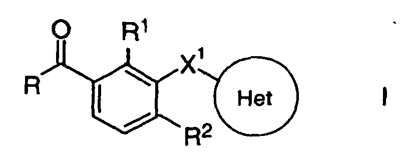 in der die Substituenten folgende Bedeutung haben:
- R¹: Chlor oder Methyl;
- R²: Chlor, Methylsulfonyl oder Trifluormethyl;
- Q: ein in 4-Stellung verknüpftes Pyrazol der Formel II, 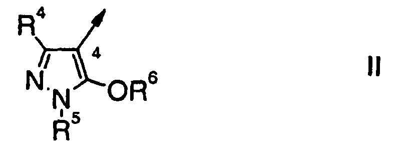 wobei
R⁴ für Wasserstoff;
R⁵ für Methyl, Ethyl oder n-Propyl;
R⁶ für Wasserstoff, Methyl, Ethyl, Methylcarbonyl, Ethylcarbonyl, Methylsulfonyl, Ethylsulfonyl oder 4-Methylphenylsulfonyl; stehen;
- X¹: CH₂, CHCH₃, CHOH, CHOCH₃, CHOCOCH₃, CHOSO₂CH₃, CH₂-CH₂ oder -C≡C-;
- Het: Oxiranyl, 3-Methyl-2-oxiranyl, 2-Oxetanyl, 3-Hydroxy-3-methyl-2-oxetanyl, 3-Hydroxy-3-ethyl-2-oxetanyl, 3-Hydroxy-3-propyl-2-oxetanyl, 3-Hydroxy-3-buthyl-2-oxetanyl, 3-Methoxy-3-methyl-2-oxetanyl, 3-Methoxy-3-ethyl-2-oxetanyl, 3-Methoxy-3-propyl-2-oxetanyl, 3-Methoxy-3-butyl-2-oxetanyl, 3-Trimethylsilyloxy-3-me- thyl-2-oxetanyl, 3-Trimethylsilyloxy-3-ethyl-2-oxetanyl, 3-Trimethyl-silyloxy-3-propyl-2-oxetanyl, 3-Trimethylsilyloxy-3-butyl-2-oxetanyl, 3-Oxetanyl, 2-Furyl, 4,5-Dihydro-2-furyl, 2,3-Dihydro-2-furyl, 3-Furyl, 4,5-Dihydro-3-furyl, 2,3-Dihydro-3-furyl, 2-Thienyl, 4,5-Dihydro-2-thienyl, 2,3-Dihydro-2-thienyl, 5-Chlor-2-thienyl, 5-Methyl-2-thienyl, 3-Thienyl, 4,5-Dihydro-3-thienyl, 2,3-Dihydro-3-thienyl, 2-Pyrrolyl, 2,5-Dihydro-2-pyrrolyl, 3-Pyrrol, 2,5-Dihydro-3-pyrrolyl, 3-Isoxazolyl, 4-Methyl-3-isoxazolyl, 5-Methyl-3-isoxazolyl, 4,5-Dimethyl-3-isoxazolyl, 4,5-Dihydro-3-isoxazolyl, 4-Methyl-4,5-dihydro-3-isoxazolyl, 5-Methyl-4,5-dihydro-3-isoxazolyl, 4,5-Dimethyl-4,5-dihydro-3-isoxazolyl, 4-Isoxazolyl, 3-Methyl-4-isoxazolyl, 5-Methyl-4-isoxazolyl, 5-Cyclopropyl-4-isoxazolyl, 5-Phenyl-4-isoxazolyl, 3,5-Dimethyl-4-isoxazolyl, 4,5-Dihydro-4-isoxazolyl, 3-Methyl-4,5-dihydro-4-isoxazolyl, 5-Methyl-4,5-dihydro-4-isoxazolyl, 3,5-Dimethyl-4,5-dihydro-4-isoxazolyl, 5-Isoxazolyl, 3-Methyl-5-isoxazolyl, 4-Methyl-5-isoxazolyl, 3,4-Dimethyl-5-isoxazolyl, 4,5-Dihydro-5-isoxazolyl, 3-Methyl-4,5-dihydro-5-isoxazolyl, 4-Methyl-4,5-dihydro-5-isoxazolyl, 3,4-Dimethyl-4,5-dihydro-5-isoxazolyl, 3-Isothiazolyl, 4-Methyl-3-isothiazolyl, 5-Methyl-3-isothiazolyl, 4-Isothiazolyl, 3-Methyl-4-isothiazolyl, 5-Methyl-4-isothiazolyl, 5-Isothiazolyl, 3-Methyl-5-isothiazolyl, 4-Methyl-5-isothiazolyl, 2-Oxazolyl, 4-Oxazolyl, 5-Oxazolyl, 2-Thiazolyl, 4-Thiazolyl, 5-Thiazolyl, 3-Pyrazolyl, 4-Pyrazolyl, 1-Methyl-3-pyrazolyl, 1-Methyl-4-pyrazolyl, 1-Methyl-5-pyrazolyl, 2-Imidazolyl, 1-Methyl-2-imidazolyl, 5-Methyl-[1,3,4]-2-oxadiazolyl, 5-Methyl-[1,2,4]-3-oxadiazolyl, 5-Methyl-[1,3,4]-2-thiadiazolyl, 5-Methyl-[1,2,4]-3-thiadiazolyl, [1,2,4]-3-Triazolyl, [1,2,3]-4-Triazolyl, 2-Pyridyl, 6-Chlor-2-pyridyl, 6-Methoxy-2-pyridyl, 6-Trifluormethyl-2-pyridyl, 3-Pyridyl, 2-Chlor-3-pyridyl, 2-Methoxy-3-pyridyl, 4-Pyridyl, 2-Chlor-4-pyridyl, 2-Methoxy-4-pyridyl, 2-Ethoxy-4-pyridyl, 2-Methylthio-4-pyridyl, 2-Trifluormethyl-5-pyridyl, 2-Pyrimidinyl, 3-Pyrimidinyl, 4-Pyrimidinyl, 2-Pyrazinyl, 3-Pyridazinyl, 4-Pyridazinyl, 2-(2*H*-1,3-Oxazinyl), 2-(6*H*-1,3-Oxazinyl), 4-(6*H*-1,3-Oxazinyl), 6-(6*H*-1,3-Oxazinyl), [1,3,5]-2-Triazinyl, [1,2,4]-3-Triazinyl, [1,2,4]-5-Triazinyl, [1,2,4]-6-Triazinyl;
sowie deren landwirtschaftlich brauchbaren Salze.

Außerdem betrifft die Erfindung Verfahren und Zwischenprodukte zur Herstellung von Verbindungen der Formel I, Mittel welche diese enthalten, sowie die Verwendung der Verbindungen der Formel I und diese enthaltende Mittel zur Schadpflanzenbekämpfung.

Aus der Literatur, beispielsweise aus EP-A 282 944 sind 4-Benzoyl-pyrazole bekannt.

In FR 2053 017 werden Salicylsäurederivate, welche para zur Hydroxygruppe einen methylenverbrückten cyclischen Rest tragen, offenbart.In Bohlmann et al., Chem. Ber. 105(3), 1972, 863-873 und K. Samula, Rocz. Chem. 45(11), 1971, 1833-1840 (= CA Vol 76, Nr. 15, 1972, abstr. 085658) wird die Synthese von Hydroxyacetophenonderivaten beschrieben.
Aus WO 96/26206 sind 4-Benzoylpyrazole, welche in 3-Position am zentralen Phenylring direkt mit einem Heterocyclus verknüpft sind, bekannt.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die erfindungsgemäßen 4-Benzoyl-pyrazole der Formel I sowie deren herbizide Wirkung gefunden.

Ferner wurden herbizide Mittel gefunden, die die Verbindungen I enthalten und eine sehr gute herbizide Wirkung besitzen. Darüber hinaus wurden Verfahren zur Herstellung dieser Mittel und Verfahren zur Bekämpfung von unerwünschtem Pflanzenwuchs mit den Verbindungen I gefunden.

Gegenstand der vorliegenden Erfindung sind auch Stereoisomere der Verbindungen der Formel I. Es werden sowohl reine Stereoisomere als auch Gemische hiervon erfaßt.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl oder Hydroxy-C₁-C₄-alkyl und/oder ein Phenyl oder Benzyl ersetzt sein können, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)-sulfonium und Sulfoxoniumionen, vorzugsweise Tri(C₁-C₄-alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

### Verfahren A:

Umsetzungen von Pyrazolen der Formel II (mit R⁶ = H) mit einer aktivierten Carbonsäure IIIa oder einer Carbonsäure IIIb, die vorzugsweise in situ aktiviert wird, zu dem Acylierungsprodukt V und anschließende Umlagerung zu den erfindungsgemäßen Verbindungen der Formel I.

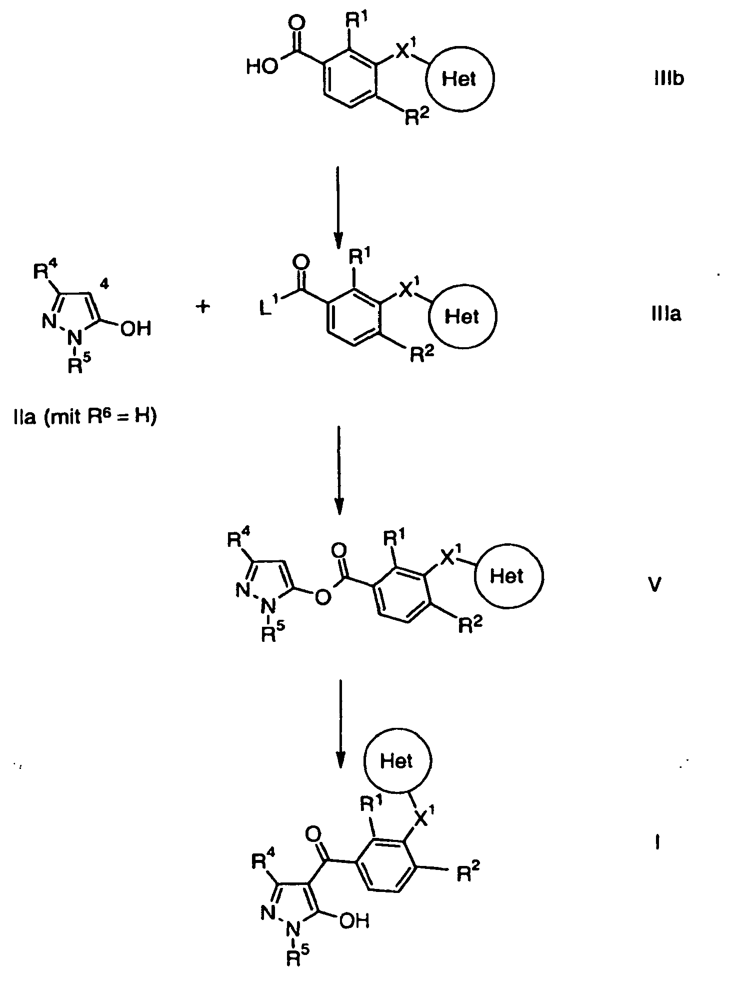

L¹ steht für eine nucleophil austauschbare Abgangsgruppe, wie Halogen z.B. Brom, Chlor, Hetaryl, z.B. Imidazolyl, Pyridyl, Carboxylat, z.B. Acetat, Trifluoracetat etc.

Die aktivierte Carbonsäure kann direkt eingesetzt werden, wie im Fall der Carbonsäurehalogenide oder in situ erzeugt werden, z.B. mit Dicyclohexylcarbodiimid, Triphenylphosphin/Azodicarbonsäureester, 2-Pyridindisulfit/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Carbonsäurehalogenide als aktivierte Carbonsäurekomponente eingesetzt, so kann es zweckmäßig sein, bei Zugabe dieses Reaktionspartners die Reaktionsmischung auf 0 bis 10°C abzukühlen. Anschließend rührt man bei 20 bis 100°C, vorzugsweise bei 25 bis 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels wird der rohe Enolester der Formel V vorzugsweise durch Chromatographie gereinigt. Es ist aber auch möglich, den rohen Enolester der Formel V ohne weitere Reinigung zur Umlagerung einzusetzen.

Die Umlagerung der Enolester der Formel V zu den Verbindungen der Formel I erfolgt zweckmäßigerweise bei Temperaturen von 20 bis 40°C in einem Lösungsmittel und in Gegenwart einer Base sowie gegebenenfalls in Gegenwart einer Cyanoverbindung.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonate verwendet.

Als Cyanoverbindungen kommen anorganische Cyanide, wie Natriumcyanid, Kaliumcyanid und organische Cyanoverbindungen, wie Acetoncyanhydrin, Trimethylsilylcyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Besonders bevorzugt werden Alkalicarbonate, wie Kaliumcarbonat, in Acetonitril oder Dioxan eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid, Essigsäureethylester extrahiert. Der organische Extrakt kann mit 5 bis 10 %iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt.

(Beispiele für die Darstellung von Estern von Hydroxypyrazolen und für die Umlagerung der Ester sind z.B. in EP-A 282 944 oder US 4 643 757 genannt).

### Verfahren B:

Umsetzungen von 4-Benzoyl-pyrazolen der Formel I (mit R⁶ = H) mit einer Verbindung der Formel IV (mit R6 ≠ H):

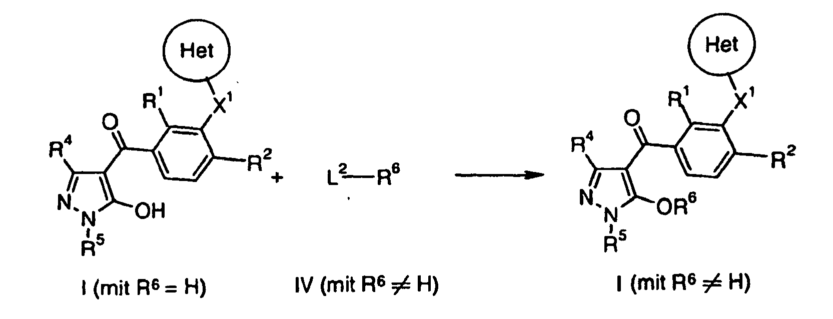

L² steht für eine nucleophil austauschbare Abgangsgruppe, wie Halogen, z.B. Brom, Chlor, Hetaryl, z.B. Imidazolyl, Pyridyl, Carboxylat, z.B. Acetat, Trifluoracetat, Sulfonat, z.B. Mesylat, Triflat etc.

Die Verbindungen der Formel IV können direkt eingesetzt werden, wie z.B. im Fall der Alkylhalogenide, Carbonsäurehalogenide, Sulfonsäurehalogenide, Carbonsäureanhydride und Sulfonsäureanhydride oder in situ erzeugt werden, z.B. aktivierte Carbonsäuren (mittels Carbonsäure und Dicyclohexylcarbodiimid, Carbonyldiimidazol etc.).

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuß der Hilfsbase z.B. 1,5 bis 3 Moläquivalente, bezogen auf Ia, kann unter Umständen vorteilhaft sein.

Als Hilfsbasen eignen sich tertiäre Alkylamine, wie Triethylamin, Pyridin, Alkalimetallcarbonate, z.B. Natriumcarbonat, Kaliumcarbonat und Alkalimetallhydride, z.B. Natriumhydrid. Bevorzugt verwendet werden Triethylamin, Pyridin und Kaliumcarbonat.

Als Lösungsmittel kommen z.B. chlorierte Kohlenwasserstoffe, wie Methylenchlorid, 1,2-Dichlorethan, aromatische Kohlenwasserstoffe, z.B. Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise erfolgen.

Die Benzoesäuren der Formel III sind neu, 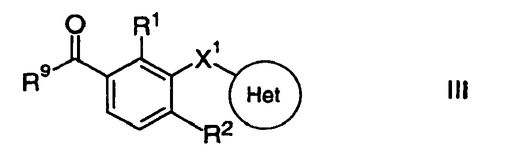 wobei die Variablen folgende Bedeutung haben:
- R¹: Chlor oder Methyl;
- R²: Chlor, Methylsulfonyl oder Trifluormethyl;
- X¹: CH₂, CHCH₃, CHOH, CHOCH₃, CHOCOCH₃, CHOSO₂CH₃, CH₂-CH₂ oder ―C≡C―;
- Het: Oxiranyl, 3-Methyl-2-oxiranyl, 2-Oxetanyl, 3-Hydroxy-3-methyl-2-oxetanyl, 3-Hydroxy-3-ethyl-2-oxetanyl, 3-Hydroxy-3-propyl-2-oxetanyl, 3-Hydroxy-3-buthyl-2-oxetanyl, 3-Methoxy-3-methyl-2-oxetanyl, 3-Methoxy-3-ethyl-2-oxetanyl, 3-Methoxy-3-propyl-2-oxetanyl, 3-Methoxy-3-butyl-2-oxetanyl, 3-Trimethylsilyloxy-3-methyl-2-oxetanyl, 3-Trimethylsilyloxy-3-ethyl-2-oxetanyl, 3-Trimethyl-silyloxy-3-propyl-2-oxetanyl, 3-Trimethylsilyloxy-3-butyl-2-oxetanyl, 3-Oxetanyl, 2-Furyl, 4,5-Dihydro-2-furyl, 2,3-Dihydro-2-furyl, 3-Furyl, 4,5-Dihydro-3-furyl, 2,3-Dihydro-3-furyl, 2-Thienyl, 4,5-Dihydro-2-thienyl, 2,3-Dihydro-2-thienyl, 5-Chlor-2-thienyl, 5-Methyl-2-thienyl, 3-Thienyl, 4,5-Dihydro-3-thienyl, 2,3-Dihydro-3-thienyl, 2-Pyrrolyl, 2,5-Dihydro-2-pyrrolyl, 3-Pyrrol, 2,5-Dihydro-3-pyrrolyl, 3-Isoxazolyl, 4-Methyl-3-isoxazolyl, 5-Methyl-3-isoxazolyl, 4,5-Dimethyl-3-isoxazolyl, 4,5-Dihydro-3-isoxazolyl, 4-Methyl-4,5-dihydro-3-isoxazolyl, 5-Methyl-4,5-dihydro-3-isoxazolyl, 4,5-Dimethyl-4,5-dihydro-3-isoxazolyl, 4-Isoxazolyl, 3-Methyl-4-isoxazolyl, 5-Methyl-4-isoxazolyl, 5-Cyclopropyl-4-isoxazolyl, 5-Phenyl-4-isoxazolyl, 3,5-Dimethyl-4-isoxazolyl, 4,5-Dihydro-4-isoxazolyl, 3-Methyl-4,5-dihydro-4-isoxazolyl, 5-Methyl-4,5-dihydro-4-isoxazolyl, 3,5-Dimethyl-4,5-dihydro-4-isoxazolyl, 5-Isoxazolyl, 3-Methyl-5-isoxazolyl, 4-Methyl-5-isoxazolyl, 3,4-Dimethyl-5-isoxazolyl, 4,5-Di- hydro-5-isoxazolyl, 3-Methyl-4,5-dihydro-5-isoxazolyl, 4-Methyl-4,5-dihydro-5-isoxazolyl, 3,4-Dimethyl-4,5-dihydro-5-isoxazolyl, 3-Isothiazolyl, 4-Methyl-3-isothiazolyl, 5-Methyl-3-isothiazolyl, 4-Isothiazolyl, 3-Methyl-4-isothiazolyl, 5-Methyl-4-isothiazolyl, 5-Isothiazolyl, 3-Methyl-5-isothiazolyl, 4-Methyl-5-isothiazolyl, 2-Oxazolyl, 4-Oxazolyl, 5-Oxazolyl, 2-Thiazolyl, 4-Thiazolyl, 5-Thiazolyl, 3-Pyrazolyl, 4-Pyrazolyl, 1-Methyl-3-pyrazolyl, 1-Methyl-4-pyrazolyl, 1-Methyl-5-pyrazolyl, 2-Imidazolyl, 1-Methyl-2-imidazolyl, 5-Methyl-[1,3,4]-2-oxadiazolyl, 5-Methyl-[1,2,4]-3-oxadiazolyl, 5-Methyl-[1,3,4]-2-thiadiazolyl, 5-Methyl-[1,2,4]-3-thiadiazolyl, [1,2,4]-3-Triazolyl, [1,2,3]-4-Triazolyl, 2-Pyridyl, 6-Chlor-2-pyridyl, 6-Methoxy-2-pyridyl, 6-Trifluormethyl-2-pyridyl, 3-Pyridyl, 2-Chlor-3-pyridyl, 2-Methoxy-3-pyridyl, 4-Pyridyl, 2-Chlor-4-pyridyl, 2-Methoxy-4-pyridyl, 2-Ethoxy-4-pyridyl, 2-Methylthio-4-pyridyl, 2-Trifluormethyl-5-pyridyl, 2-Pyrimidinyl, 3-Pyrimidinyl, 4-Pyrimidinyl, 2-Pyrazinyl, 3-Pyridazinyl, 4-Pyridazinyl, 2-(2*H*-1,3-Oxazinyl), 2-(6*H*-1,3-Oxazinyl), 4-(6*H*-1,3-Oxazinyl), 6-(6*H*-1,3-Oxazinyl), [1,3,5]-2-Triazinyl, [1,2,4]-3-Triazinyl, [1,2,4]-5-Triazinyl, [1,2,4]-6-Triazinyl;
- R⁹: Hydroxy oder ein hydrolysierbarer Rest.

Beispiele für hydrolysierbare Reste sind Alkoxy-, Phenoxy-, Alkylthio-, Phenylthioreste, die substituiert sein können, Halogenide, Hetarylreste, die über Stickstoff gebunden sind, Amino-, Iminoreste, die substituiert sein können, etc.

Bevorzugt sind Benzoesäurehalogenide IIIa mit L¹ = Halogen (≙ III mit R⁹ = Halogen), 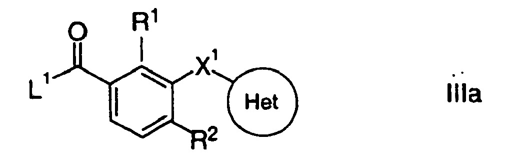 wobei die Variablen R¹, R², X¹ und Het die unter Formel III genannte Bedeutung haben und
- L¹: Halogen, insbesondere Chlor oder Brom, bedeuten.

Ebenso bevorzugt sind Benzoesäuren der Formel IIIb (≙ III mit R⁹ = Hydroxy), 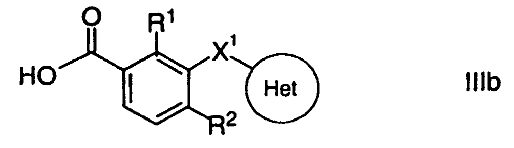 wobei die Variablen R¹, R², X¹ und Het die unter Formel III genannte Bedeutung haben.

Ebenso bevorzugt sind Benzoesäureester der Formel IIIc (≙ III mit R⁹ = C₁-C₆-Alkoxy), 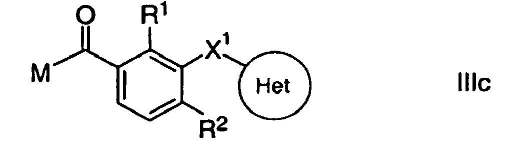 wobei die Variablen R¹, R², X¹ und Het die unter Formel III genannte Bedeutung haben und
- M: C₁-C₆-Alkoxy
bedeutet.

Die Verbindungen der Formel IIIa (mit L¹ = Halogen) können in Analogie zu literaturbekannten Methoden (vgl. L.G. Fieser, M. Fieser "Reagents for Organic Synthesis", Bd. I, S. 767-769 (1967)) durch Umsetzung von Benzoesäuren der Formel IIIb mit Halogenierungsreagentien wie Thionylchlorid, Thionylbromid, Phosgen, Diphosgen, Triphosgen, Oxalylchlorid, Oxalylbromid dargestellt werden.

Die Benzoesäuren der Formel IIIb können u. a. durch Verseifung der Benzoesäureester der Formel IIIc (mit M = C₁-C₆-Alkoxy) erhalten werden.

Die erfindungsgemäßen Benzoesäureester der Formel IIIc sind nach verschiedenen literaturbekannten Methoden (z.B. a. G. Dittus in Houben-Weyl, Methoden der Organischen Chemie, Band VI/3, Sauerstoff-Verbindungen I, 4. Aufl., S. 493 ff., Georg Thieme Verlag, 1965; b. T. L. Gilchrist, Heterocyclenchemie, 2. Aufl., Verlag Chemie, 1995) darstellbar, wie in den nachfolgenden Beispielen illustriert.

### Verfahren C:

1,3-dipolare Cycloaddition der Benzoesäureester IIId mit gegebenenfalls substituierten Olefinen oder Acetylenen unter dehydratisierenden Bedingungen liefert die erfindungsgemäßen Benzoesäureester, wie beispielsweise IIIe. 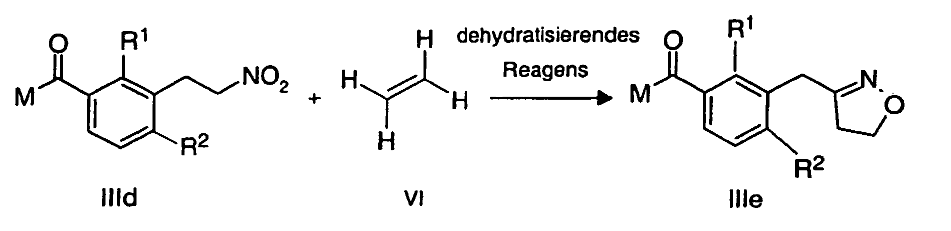 Zur Dehydratisierung nach der Methode von Mukaiyama werden bevorzugt aromatische Isocyanate (z.B. T. Mukaiyama *et al., J. Am. Chem. Soc.* 1960, 82, 5339), wie z.B. Phenylisocyanat oder 4-Chlorphenylisocyanat eingesetzt.

In der Variante nach Shimizu eignen sich ebenfalls aliphatische Chlorameisensäureester (z.B. T. Shimizu et al., *Bull. Chem. Soc. Jpn.* 1986, 59, 2827), bevorzugt Ethylchloroformat.

Neuere Entwicklungen zeigen, daß z.B. auch *N,N*-Diethylaminoschwefeltrifluorid (DAST), (Methoxycarbonylsulfamoyl)-triethylammoniumhydroxid (Burgess Reagens), Phosphorylchlorid (z.B. C. Mioskowski *et al., Tetrahedron Letters* 1997, 38, 1547) oder auch eine Kombination aus Di-*tert*-butyl-dicarbonat (Boc₂O) und 4-Dimethylaminopyridin (DMAP) (A. Hassner *et al., Synthesis* 1997, 309) erfolgreich als dehydratisierende Reagenzien zur Erzeugung von Nitriloxiden eingesetzt werden können.

Die auf diese Weise in situ gebildeten Nitriloxide können bei Raumtemperatur bis zur Siedetemperatur des verwendeten Lösungsmittels mit beliebig substituierten Olefinen oder Acetylen zu den erfindungsgemäßen Benzoesäureestern IIIc umgesetzt werden, wobei hier beispielsweise X¹ eine Methylengruppe und Het einen gegebenenfalls substituiertes Isoxazol- oder Isoxazolingerüst darstellt.

Die Durchführung der Cycloaddition erfolgt in inerten Lösungsmitteln, wie beispielsweise Toluol, Chloroform oder Acetonitril.

Der Benzoesäureester IIId kann durch Reduktion nach literaturbekannten Methoden aus IIIg erhalten werden, der durch Nitroolefinierung (z.B. a. V. V. Perekalin *et al.,* Nitroalkenes, John Wiley & Sons Ltd., New York 1994, b. A. G. M. Barrett *et al., Chem. Rev.* 1986, 86, 751) des entsprechenden Aldehyds IIIf hergestellt werden kann.

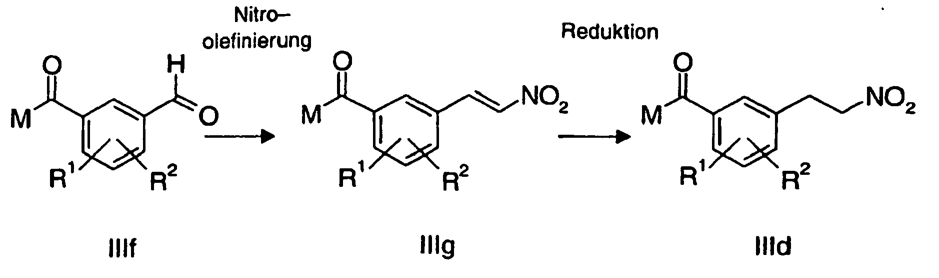

Die für die Substituenten R¹, R⁴, R⁵, R⁶ oder als Reste an Phenyl- und Hetarylringen genannten organischen Molekülteile stellen Sammelbegriffe für individuelle Aufzählungen der einzelnen Gruppenmitglieder dar. Sämtliche Kohlenwasserstoffketten, also alle Alkyl- und Alkoxy-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl: Methyl, Ethyl, n-Propyl, 1-Methylethyl, Butyl, 1-Methylpropyl, 2-Methylpropyl und 1,1-Dimethylethyl;
- C₁-C₄-Alkoxy: Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy: C₁-C₄-Alkoxy wie voranstehend genannt, sowie Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- Hetaryl: aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thia diazol-5-yl, 1,2,4-Triazol-3-yl, 1,3,4-Oxadiazol- 2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4,5-Tetrazin-3-yl, sowie die entsprechenden benzokondensierten Derivate.

Alle Phenyl- und Hetarylringe sind vorzugsweise unsubstituiert oder tragen ein bis drei Halogenatome und/oder einen oder zwei Reste aus folgender Gruppe:
Nitro, Cyano, Methyl, Trifluormethyl, Methoxy, Trifluormethoxy oder Methoxycarbonyl.

Außerordentlich bevorzugt sind die Verbindungen der Formel Ia, in der die Substituenten R¹ und R² und Q und Het die oben genannte Bedeutung haben und X¹ für eine C₁-C₂-Alkylen- oder eine C₂-Alkinylenkette steht.

Darüber hinaus sind die erfindungsgemäßen Verbindungen der Formel Ia außerordentlich bevorzugt, in der die Substituenten R¹, R², Q und X¹ die oben genannte Bedeutung haben und Het für 2-Furyl, 4,5-Dihydro-2-furyl, 2,3-Dihydro-2-furyl, 3-Furyl, 4,5-Dihydro-3-furyl, 2,3-Dihydro-3-furyl, 2-Thienyl, 4,5-Dihydro-2-thienyl, 2,3-Dihydro-2-thienyl, 5-Chlor-2-thienyl, 5-Methyl-2-thienyl, 3-Thienyl, 4,5-Dihydro-3-thienyl, 2,3-Dihydro-3-thienyl, 2-Pyrrolyl, 2,5-Dihydro-2-pyrrolyl, 3-Pyrrolyl, 2,5-Dihydro-3-pyrrolyl, 3-Isoxazolyl, 4-Methyl-3-isoxazolyl, 5-Methyl-3-isoxazolyl, 4,5-Dimethyl-3-isoxazolyl, 4,5-Dihydro-3-isoxazolyl, 4-Methyl-4,5-dihydro-3-isoxazolyl, 5-Methyl-4,5-dihydro-3-isoxazolyl, 4,5-Dimethyl-4,5-dihydro-3-isoxazolyl, 4-Isoxazolyl, 3-Methyl-4-isoxazolyl, 5-Methyl-4-isoxazolyl, 5-Cyclopropyl-4-isoxazolyl, 5-Phenyl-4-isoxazolyl, 3,5-Dimethyl-4-isoxazolyl, 4,5-Dihydro-4-isoxazolyl, 3-Methyl-4,5-dihydro-4-isoxazolyl, 5-Methyl-4,5-dihydro-4-isoxazolyl, 3,5-Dimethyl-4,5-dihydro-4-isoxazolyl, 5-Isoxazolyl, 3-Methyl-5-isoxazolyl, 4-Methyl-5-isoxazolyl, 3,4-Dimethyl-5-isoxazolyl, 4,5-Dihydro-5-isoxazolyl, 3-Methyl-4,5-dihydro-5-isoxazolyl, 4-Methyl-4,5-dihydro-5-isoxazolyl, 3,4-Dimethyl-4,5-dihydro-5-isoxazolyl, 3-isothiazolyl, 4-Methyl-3-isothiazolyl, 5-Methyl-3-isothiazolyl, 4-Isothiazolyl, 3-Methyl-4-isothiazolyl, 5-Methyl-4-isothiazolyl, 5-Isothiazolyl, 3-Methyl-5-isothiazolyl, 4-Methyl-5-isothiazolyl, 2-Oxazolyl, 4-Oxazolyl, 5-Oxazolyl, 2-Thiazolyl, 4-Thiazolyl, 5-Thiazolyl, 3-Pyrazolyl, 4-Pyrazolyl, 1-Methyl-3-pyrazolyl, 1-Methyl-4-pyrazolyl, 1-Methyl-5-pyrazolyl, 2-Imidazolyl, 1-Methyl-2-imidazolyl, 5-Methyl-[1,3,4]-2-oxadiazolyl, 5-Methyl-[1,2,4]-3-oxadiazolyl, 5-Methyl-[1,3,4]-2-thiadiazolyl, 5-Methyl-[1,2,4]-3-thiadiazolyl, [1,2,4]-3-Triazolyl, [1,2,3]-4-Triazolyl, 2-Pyridyl, 6-Chlor-2-pyridyl, 6-Methoxy-2-pyridyl, 6-Trifluormethyl-2-pyridyl, 3-Pyridyl, 2-Chlor-3-pyridyl, 2-Methoxy-3-pyridyl, 4-Pyridyl, 2-Chlor-4-pyridyl, 2-Methoxy-4-pyridyl, 2-Ethoxy-4-pyridyl, 2-Methylthio-4-pyridyl, 2-Trifluormethyl-5-pyridyl, 2-Pyrimidinyl, 3-Pyrimidinyl, 4-Pyrimidinyl, 2-Pyrazinyl, 3-Pyridazinyl, 4-Pyridazinyl, 2-(2H-1,3-Oxazinyl), 2-(6H-1,3-Oxazinyl), 4-(6H-1,3-Oxazinyl), 6-(6H-1,3-Oxazinyl), [1,3,5]-2-Triazinyl, [1,2,4]-3-Triazinyl, [1,2,4]-5-Triazinyl, [1,2,4]-6-Triazinyl;
steht.

Insbesondere bevorzugt sind die Verbindungen Ib der Tabellen 1 bis 144.

**Tabelle A**

| **Nr.** | **X**^{**1**} | **Het** |
|---|---|---|
| 1 | CH₂ | Oxiranyl |
| 2 | CH₂ | 3-Methyl-2-oxiranyl |
| 3 | CH₂ | 2-Oxetanyl |
| 4 | CH₂ | 3-Hydroxy-3-methyl-2-oxetanyl |
| 5 | CH₂ | 3-Hydroxy-3-ethyl-2-oxetanyl |
| 6 | CH₂ | 3-Hydroxy-3-propyl-2-oxetanyl |
| 7 | CH₂ | 3-Hydroxy-3-butyl-2-oxetanyl |
| 8 | CH₂ | 3-Methoxy-3-methyl-2-oxetanyl |
| 9 | CH₂ | 3-Methoxy-3-ethyl-2-oxetanyl |
| 10 | CH₂ | 3-Methoxy-3-propyl-2-oxetanyl |
| 11 | CH₂ | 3-Methoxy-3-butyl-2-oxetanyl |
| 12 | CH₂ | 3-Trimethylsilyloxy-3-methyl-2-oxetanyl |
| 13 | CH₂ | 3-Trimethylsilyloxy-3-ethyl-2-oxetanyl |
| 14 | CH₂ | 3-Trimethylsilyloxy-3-propyl-2-oxetanyl |
| 15 | CH₂ | 3-Trimethylsilyloxy-3-butyl-2-oxetanyl |
| 16 | CH₂ | 3-Oxetanyl |
| 17 | CH₂ | 2-Furyl |
| 18 | CH₂ | 4,5-Dihydro-2-furyl |
| 19 | CH₂ | 2,3-Dihydro-2-furyl |
| 20 | CH₂ | 3-Furyl |
| 21 | CH₂ | 4,5-Dihydro-3-furyl |
| 22 | CH₂ | 2,3-Dihydro-3-furyl |
| 23 | CH₂ | 2-Thienyl |
| 24 | CH₂ | 4,5-Dihydro-2-thienyl |
| 25 | CH₂ | 2,3-Dihydro-2-thienyl |
| 26 | CH₂ | 5-Chlor-2-thienyl |
| 27 | CH₂ | 5-Methyl-2-thienyl |
| 28 | CH₂ | 3-Thienyl |
| 29 | CH₂ | 4,5-Dihydro-3-thienyl |
| 30 | CH₂ | 2,3-Dihydro-3-thienyl |
| 31 | CH₂ | 2-Pyrrolyl |
| 32 | CH₂ | 2,5-Dihydro-2-pyrrolyl |
| 33 | CH₂ | 3-Pyrrol |
| 34 | CH₂ | 2,5-Dihydro-3-pyrrolyl |
| 35 | CH₂ | 3-Isoxazolyl |
| 36 | CH₂ | 4-Methyl-3-isoxazolyl |
| 37 | CH₂ | 5-Methyl-3-isoxazolyl |
| 38 | CH₂ | 4,5-Dimethyl-3-isoxazolyl |
| 39 | CH₂ | 4,5-Dihydro-3-isoxazolyl |
| 40 | CH₂ | 4-Methyl-4,5-dihydro-3-isoxazolyl |
| 41 | CH₂ | 5-Methyl-4,5-dihydro-3-isoxazolyl |
| 42 | CH₂ | 4,5-Dimethyl-4,5-dihydro-3-isoxazolyl |
| 43 | CH₂ | 4-Isoxazolyl |
| 44 | CH₂ | 3-Methyl-4-isoxazolyl |
| 45 | CH₂ | 5-Methyl-4-isoxazolyl |
| 46 | CH₂ | 5-Cyclopropyl-4-isoxazolyl |
| 47 | CH₂ | 5-Phenyl-4-isoxazolyl |
| 48 | CH₂ | 3,5-Dimethyl-4-isoxazolyl |
| 49 | CH₂ | 4,5-Dihydro-4-isoxazolyl |
| 50 | CH₂ | 3-Methyl-4,5-dihydro-4-isoxazolyl |
| 51 | CH₂ | 5-Methyl-4,5-dihydro-4-isoxazolyl |
| 52 | CH₂ | 3,5-Dimethyl-4,5-dihydro-4-isoxazolyl |
| 53 | CH₂ | 5-Isoxazolyl |
| 54 | CH₂ | 3-Methyl-5-isoxazolyl |
| 55 | CH₂ | 4-Methyl-5-isoxazolyl |
| 56 | CH₂ | 3,4-Dimethyl-5-isoxazolyl |
| 57 | CH₂ | 4,5-Dihydro-5-isoxazolyl |
| 58 | CH₂ | 3-Methyl-4,5-dihydro-5-isoxazolyl |
| 59 | CH₂ | 4-Methyl-4,5-dihydro-5-isoxazolyl |
| 60 | CH₂ | 3,4-Dimethyl-4,5-dihydro-5-isoxazolyl |
| 61 | CH₂ | 3-Isothiazolyl |
| 62 | CH₂ | 4-Methyl-3-isothiazolyl |
| 63 | CH₂ | 5-Methyl-3-isothiazolyl |
| 64 | CH₂ | 4-Isothiazolyl |
| 65 | CH₂ | 3-Methyl4-isothiazolyl |
| 66 | CH₂ | 5-Methyl-4-isothiazolyl |
| 67 | CH₂ | 5-Isothiazolyl |
| 68 | CH₂ | 3-Methyl-5-isothiazolyl |
| 69 | CH₂ | 4-Methyl-5-isothiazolyl |
| 70 | CH₂ | 2-Oxazolyl |
| 71 | CH₂ | 4-Oxazolyl |
| 72 | CH₂ | 5-Oxazolyl |
| 73 | CH₂ | 2-Thiazolyl |
| 74 | CH₂ | 4-Thiazolyl |
| 75 | CH₂ | 5-Thiazolyl |
| 76 | CH₂ | 3-Pyrazolyl |
| 77 | CH₂ | 4-Pyrazolyl |
| 78 | CH₂ | 1-Methyl-3-pyrazolyl |
| 79 | CH₂ | 1-Methyl-4-pyrazolyl |
| 80 | CH₂ | 1-Methyl-5-pyrazolyl |
| 81 | CH₂ | 2-Imidazolyl |
| 82 | CH₂ | 1-Methyl-2-imidazolyl |
| 83 | CH₂ | 5-Methyl-[1,3,4]-2-oxadiazolyl |
| 84 | CH₂ | 5-Methyl-[1,2,4]-3-oxadiazolyl |
| 85 | CH₂ | 5-Methyl-[1,3,4]-2-thiadiazolyl |
| 86 | CH₂ | 5-Methyl-[1,2,4]-3-thiadiazolyl |
| 87 | CH₂ | [1,2,4]-3-triazolyl |
| 88 | CH₂ | [1,2,3]-4-triazolyl |
| 89 | CH₂ | 2-Pyridyl |
| 90 | CH₂ | 6-Chlor-2-pyridyl |
| 91 | CH₂ | 6-Methoxy-2-pyridyl |
| 92 | CH₂ | 6-Trifluormethyl-2-pyridyl |
| 93 | CH₂ | 3-Pyridyl |
| 94 | CH₂ | 2-Chlor-3-pyridyl |
| 95 | CH₂ | 2-Methoxy-3-pyridyl |
| 96 | CH₂ | 4-Pyridyl |
| 97 | CH₂ | 2-Chlor-4-pyridyl |
| 98 | CH₂ | 2-Methoxy-4-pyridyl |
| 99 | CH₂ | 2-Ethoxy-4-pyridyl |
| 100 | CH₂ | 2-Methylthio-4-pyridyl |
| 101 | CH₂ | 2-Trifluormethyl-5-pyridyl |
| 102 | CH₂ | 2-Pyrimidinyl |
| 103 | CH₂ | 3-Pyrimidinyl |
| 104 | CH₂ | 4-Pyrimidinyl |
| 105 | CH₂ | 2-Pyrazinyl |
| 106 | CH₂ | 3-Pyridazinyl |
| 107 | CH₂ | 4-Pyridazinyl |
| 108 | CH₂ | 2-(2*H*-1,3-oxazinyl) |
| 109 | CH₂ | 2-(6*H*-1,3-oxazinyl) |
| 110 | CH₂ | 4-(6*H*-1,3-oxazinyl) |
| 111 | CH₂ | 6-(6*H*-1,3-oxazinyl) |
| 112 | CH₂ | [1,3,5]-2-Triazinyl |
| 113 | CH₂ | [1,2,4]-3-Triazinyl |
| 114 | CH₂ | [1,2,4]-5-Triazinyl |
| 115 | CH₂ | [1,2,4]-6-Triazinyl |
| 116 | CHCH₃ | Oxiranyl |
| 117 | CHCH₃ | 3-Methyl-2-oxiranyl |
| 118 | CHCH₃ | 2-Oxetanyl |
| 119 | CHCH₃ | 3-Hydroxy-3-methyl-2-oxetanyl |
| 120 | CHCH₃ | 3-Hydroxy-3-ethyl-3-oxetanyl |
| 121 | CHCH₃ | 3-Hydroxy-3-propyl-2-oxetanyl |
| 122 | CHCH₃ | 3-Hydroxy-3-butyl-2-oxetanyl |
| 123 | CHCH₃ | 3-Methoxy-3-methyl-2-oxetanyl |
| 124 | CHCH₃ | 3-Methoxy-3-ethyl-2-oxetanyl |
| 125 | CHCH₃ | 3-Methoxy-3-propyt-2-oxetanyl |
| 126 | CHCH₃ | 3-Methoxy-3-butyl-2-oxetanyl |
| 127 | CHCH₃ | 3-Trimethylsilylox-3-methyl-2-oxetanyl |
| 128 | CHCH₃ | 3-Trimethylsilyloxy-3-ethyl-2-oxetanyl |
| 129 | CHCH₃ | 3-Trimethylsilyloxy-3-propyl-2-oxetanyl |
| 130 | CHCH₃ | 3-Trimethylsilyloxy-3-butyl-2-oxetanyl |
| 131 | CHCH₃ | 3-Oxetanyl |
| 132 | CHCH₃ | 2-Furyl |
| 133 | CHCH₃ | 4,5-Dihydro-2-furyl |
| 134 | CHCH₃ | 2,3-Dihydro-2-furyl |
| 135 | CHCH₃ | 3-Furyl |
| 136 | CHCH₃ | 4,5-Dihydro-3-furyl |
| 137 | CHCH₃ | 2,3-Dihydro-3-furyl |
| 138 | CHCH₃ | 2-Thienyl |
| 139 | CHCH₃ | 4,5-Dihydro-2-thienyl |
| 140 | CHCH₃ | 2,3-Dihydro-2-thienyl |
| 141 | CHCH₃ | 5-Chlor-2-thienyl |
| 142 | CHCH₃ | 5-Methyl-2-thienyl |
| 143 | CHCH₃ | 3-Thienyl |
| 144 | CHCH₃ | 4,5-Dihydro-3-thienyl |
| 145 | CHCH₃ | 2,3-Dihydro-3-thienyl |
| 146 | CHCH₃ | 2-Pyrrolyl |
| 147 | CHCH₃ | 2,5-Dihydro-2-pyrrolyl |
| 148 | CHCH₃ | 3-Pyrrol |
| 149 | CHCH₃ | 2,5-Dihydro-3-pyrrolyl |
| 150 | CHCH₃ | 3-Isoxazolyl |
| 151 | CHCH₃ | 4-Methyl-3-isoxazolyl |
| 152 | CHCH₃ | 5-Methyl-3-isoxazolyl |
| 153 | CHCH₃ | 4,5-Dimediyl-3-isoxazolyl |
| 154 | CHCH₃ | 4,5-Dihydro-3-isoxazolyl |
| 155 | CHCH₃ | 4-Methyl-4,5-dihydro-3-isoxazolyl |
| 156 | CHCH₃ | 5-Methyl-4,5-dihydro-3- isoxazolyl |
| 157 | CHCH₃ | 4,5-Dimethyl-4,5-dihydro-3-isoxazolyl |
| 158 | CHCH₃ | 4-Isoxazolyl |
| 159 | CHCH₃ | 3-Methyl-4-isoxazolyl |
| 160 | CHCH₃ | 5-Methyl-4-isoxazolyl |
| 161 | CHCH₃ | 5-Cyclopropyl-4-isoxazolyl |
| 162 | CHCH₃ | 5-Phenyl-4-isoxazolyl |
| 163 | CHCH₃ | 3,5-Dimethyl-4-isoxazolyl |
| 164 | CHCH₃ | 4,5-Dihydro-4-isoxazolyl |
| 165 | CHCH₃ | 3-Methyl-4,5-dihydro-4-isoxazolyl |
| 166 | CHCH₃ | 5-Methyl-4,5-dihydro-4-isoxazolyl |
| 167 | CHCH₃ | 3,5-Dimethyl-4,5-dihydro-4-isoxazolyl |
| 168 | CHCH₃ | 5-Isoxazolyl |
| 169 | CHCH₃ | 3-Methyl-5-isoxazolyl |
| 170 | CHCH₃ | 4-Methyl-5-isoxazolyl |
| 171 | CHCH₃ | 3,4-Dimethyl-5-isoxazolyl |
| 172 | CHCH₃ | 4,5-Dihydro-5-isoxazolyl |
| 173 | CHCH₃ | 3-Methyl-4,5-dihydro-5-isoxazolyl |
| 174 | CHCH₃ | 4-Methyl-4,5-dihydro-5-isoxazolyl |
| 175 | CHCH₃ | 3,4-Dimethyl-4,5-dihydro-5-isoxazolyl |
| 176 | CHCH₃ | 3-Isothiazolyl |
| 177 | CHCH₃ | 4-Methyl-3-isothiazolyl |
| 178 | CHCH₃ | 5-Methyl-3-isothiazolyl |
| 179 | CHCH₃ | 4-Isothiazolyl |
| 180 | CHCH₃ | 3-Methyl-4-isothiazolyl |
| 181 | CHCH₃ | 5-Methyl-4-isothiazolyl |
| 182 | CHCH₃ | 5-Isothiazolyl |
| 183 | CHCH₃ | 3-Methyl-5-isothiazolyl |
| 184 | CHCH₃ | 4-Methyl-5-isothiazolyl |
| 185 | CHCH₃ | 2-Oxazolyl |
| 186 | CHCH₃ | 4-Oxazolyl |
| 187 | CHCH₃ | 5-Oxazolyl |
| 188 | CHCH₃ | 2-Thiazolyl |
| 189 | CHCH₃ | 4-Thiazolyl |
| 190 | CHCH₃ | 5-Thiazolyl |
| 191 | CHCH₃ | 3-Pyrazolyl |
| 192 | CHCH₃ | 4-Pyrazolyl |
| 193 | CHCH₃ | 1-Methyl-3-pyrazolyl |
| 194 | CHCH₃ | 1-Methyl-4-pyrazolyl |
| 195 | CHCH₃ | 1-Methyl-5-pyrazolyl |
| 196 | CHCH₃ | 2-Imidazolyl |
| 197 | CHCH₃ | 1-Methyl-2-imidazolyl |
| 198 | CHCH₃ | 5-Methyl-[1,3,4]-2-oxadiazolyl |
| 199 | CHCH₃ | 5-Methyl-[1,2,4]-3-oxadiazolyl |
| 200 | CHCH₃ | 5-Methyl-[1,3,4]-2-thiadiazolyl |
| 201 | CHCH₃ | 5-Methyl-[1,2,4]-3-thiadiazolyl |
| 202 | CHCH₃ | [1,2,4]-3-triazolyl |
| 203 | CHCH₃ | [1,2,3]-4-triazolyl |
| 204 | CHCH₃ | 2-Pyridyl |
| 205 | CHCH₃ | 6-Chlor-2-pyridyl |
| 206 | CHCH₃ | 6-Methoxy-2-pyridyl |
| 207 | CHCH₃ | 6-Trifluormethyl-2-pyridyl |
| 208 | CHCH₃ | 3-Pyridyl |
| 209 | CHCH₃ | 2-Chlor-3-pyridyl |
| 210 | CHCH₃ | 2-Methoxy-3-Pyridyl |
| 211 | CHCH₃ | 4-Pyridyl |
| 212 | CHCH₃ | 2-Chlor-4-pyridyl |
| 213 | CHCH₃ | 2-Methoxy-4-pyridyl |
| 214 | CHCH₃ | 2-Ethoxy-4-pyridyl |
| 215 | CHCH₃ | 2-Methylthio-4-pyridyl |
| 216 | CHCH₃ | 2-Trifluormethyl-5-pyridyl |
| 217 | CHCH₃ | 2-Pyrimidinyl |
| 218 | CHCH₃ | 3-Pyrimidinyl |
| 219 | CHCH₃ | 4-Pyrimidinyl |
| 220 | CHCH₃ | 2-Pyrazinyl |
| 221 | CHCH₃ | 3-Pyridazinyl |
| 222 | CHCH₃ | 4-pyridazinyl |
| 223 | CHCH₃ | 2-(2*H*-1,3-oxazinyl) |
| 224 | CHCH₃ | 2-(6*H*-1,3-oxazinyl) |
| 225 | CHCH₃ | 4-(6*H*-1,3-oxazinyl) |
| 226 | CHCH₃ | 6-(6*H*-1,3-oxazinyl) |
| 227 | CHCH₃ | [1,3,5]-2-Triazinyl |
| 228 | CHCH₃ | [1,2,4]-3-Triazinyl |
| 229 | CHCH₃ | [1,2,4]-5-Triazinyl |
| 230 | CHCH₃ | [1,2,4]-6-Triazinyl |
| 231 | CHOH | Oxiranyl |
| 232 | CHOH | 3-Methyl-2-oxiranyl |
| 233 | CHOH | 2-Oxetanyl |
| 234 | CHOH | 3-Hydroxy-3-methyl-2-oxetanyl |
| 235 | CHOH | 3-Hydroxy-3-ethyl-2-oxetanyl |
| 236 | CHOH | 3-Hydroxy-3-propyl-2-oxetanyl |
| 237 | CHOH | 3-Hydroxy-3-butyl-2-oxetanyl |
| 238 | CHOH | 3-Methoxy-3-methyl-2-oxetanyl |
| 239 | CHOH | 3-Methoxy-3-ethyl-2-oxetanyl |
| 240 | CHOH | 3-Methoxy-3-propyl-2-oxetanyl |
| 241 | CHOH | 3-Methoxy-3-butyl-2-oxetanyl |
| 242 | CHOH | 3-Trimethylsilyloxy-3-methyl-2-oxetanyl |
| 243 | CHOH | 3-Trimethylsilyloxy-3-ethyl-2-oxetanyl |
| 244 | CHOH | 3-Trimethylsilyloxy-3-propyl-2-oxetanyl |
| 245 | CHOH | 3-Trimethylsilyloxy-3-butyl-2-oxetanyl |
| 246 | CHOH | 3-Oxetanyl |
| 247 | CHOH | 2-Furyl |
| 248 | CHOH | 4,5-Dihydro-2-furyl |
| 249 | CHOH | 2,3-Dihydro-2-furyl |
| 250 | CHOH | 3-Furyl |
| 251 | CHOH | 4,5-Dibydro-3-furyl |
| 252 | CHOH | 2,3-Dihydro-3-furyl |
| 253 | CHOH | 2-Thienyl |
| 254 | CHOH | 4,5-Dihydro-2-thienyl |
| 255 | CHOH | 2,3-Dihydro-2-thienyl |
| 256 | CHOH | 5-Chlor-2-thienyl |
| 257 | CHOH | 5-Methyl-2-thienyl |
| 258 | CHOH | 3-Thienyl |
| 259 | CHOH | 4,5-Dihydro-3-thienyl |
| 260 | CHOH | 2,3-Dihydro-3-thienyl |
| 261 | CHOH | 2-Pyrrolyl |
| 262 | CHOH | 2,5-Dihydro-2-pyrrolyl |
| 263 | CHOH | 3-Pyrrol |
| 264 | CHOH | 2,5-Dihydro-3-pyrrolyl |
| 265 | CHOH | 3-Isoxazolyl |
| 266 | CHOH | 4-Methyl-3-isoxazolyl |
| 267 | CHOH | 5-Methyl-3-isoxazolyl |
| 268 | CHOH | 4,5-Dimethyl-3-isoxazolyl |
| 269 | CHOH | 4,5-Dihydro-3-isoxazolyl |
| 270 | CHOH | 4-Methyl-4,5-dihydro-3-isoxazolyl |
| 271 | CHOH | 5-Methyl-4,5-dihydro 3-isoxazolyl |
| 272 | CHOH | 4,5-Dimethyl-4,5-dihydro-3-isoxazolyl |
| 273 | CHOH | 4-Isoxazolyl |
| 274 | CHOH | 3-Methyl-4-isoxazolyl |
| 275 | CHOH | 5-Methyl-4-isoxazolyl |
| 276 | CHOH | 5-Cyclopropyl-4-isoxazolyl |
| 277 | CHOH | 5-Phenyl-4-isoxazolyl |
| 278 | CHOH | 3,5-Dimethyl-4-isoxazolyl |
| 279 | CHOH | 4,5-Dihydro-4-isoxazolyl |
| 280 | CHOH | 3-Methyl-4,5-dihydro-4-isoxazolyl |
| 281 | CHOH | 5-Methyl-4,5-dihydro-4-isoxazolyl |
| 282 | CHOH | 3,5-Dimethyl-4,5-dihydro-4-isoxazolyl |
| 283 | CHOH | 5-Isoxazolyl |
| 284 | CHOH | 3-Methyl-5-isoxazolyl |
| 285 | CHOH | 4-Methyl-5-isoxazolyl |
| 286 | CHOH | 3,4-Dimethyl-5-isoxazolyl |
| 287 | CHOH | 4,5-Dihydro-5-isoxazolyl |
| 288 | CHOH | 3-Methyl-4,5-dihydro-5-isoxazolyl |
| 289 | CHOH | 4-Methyl-4,5-dihydro-5-isoxazolyl |
| 290 | CHOH | 3,4-Dimethyl-4,5-dihydro-5-isoxazolyl |
| 291 | CHOH | 3-Isothiazolyl |
| 292 | CHOH | 4-Methyl-3-isothiazolyl |
| 293 | CHOH | 5-Methyl-3-isothiazolyl |
| 294 | CHOH | 4-Isothiazolyl |
| 295 | CHOH | 3-Methyl-4-isothiazolyl |
| 296 | CHOH | 5-Methyl-4-isothiazolyl |
| 297 | CHOH | 5-Isothiazolyl |
| 298 | CHOH | 3-Methyl-5-isothiazolyl |
| 299 | CHOH | 4-Methyl-5-isothiazolyl |
| 300 | CHOH | 2-Oxazolyl |
| 301 | CHOH | 4-Oxazolyl |
| 302 | CHOH | 5-Oxazolyl |
| 303 | CHOH | 2-Thiazolyl |
| 304 | CHOH | 4-Thiazolyl |
| 305 | CHOH | 5-Thiazolyl |
| 306 | CHOH | 3-Pyrazolyl |
| 307 | CHOH | 4-Pyrazolyl |
| 308 | CHOH | 1-Methyl-3-pyrazolyl |
| 309 | CHOH | 1-Methyl-4-pyrazolyl |
| 310 | CHOH | 1-Methyl-5-pyrazolyl |
| 311 | CHOH | 2-Imidazolyl |
| 312 | CHOH | 1-Methyl-2-imidazolyl |
| 313 | CHOH | 5-Methyl-[1,3,4]-2-oxadiazolyl |
| 314 | CHOH | 5-Methyl-[1,2,4]-3-oxadiazolyl |
| 315 | CHOH | 5-Methyl-[1,3,4]-2-thiadiazolyl |
| 316 | CHOH | 5-Methyl-[1,2,4]-3-thiadiazolyl |
| 317 | CHOH | [1,2,4]-3-triazolyl |
| 318 | CHOH | [1,2,3]-4-triazolyl |
| 319 | CHOH | 2-Pyridyl |
| 320 | CHOH | 6-Chlor-2-pyridyl |
| 321 | CHOH | 6-Methoxy-2-pyridyl |
| 322 | CHOH | 6-Trifluormethyl-2-pyridyl |
| 323 | CHOH | 3-Pyridyl |
| 324 | CHOH | 2-Chlor-3-pyridyl |
| 325 | CHOH | 2-Methoxy-3-pyridyl |
| 326 | CHOH | 4-Pyridyl |
| 327 | CHOH | 2-Chlor-4-pyridyl |
| 328 | CHOH | 2-Methoxy-4-pyridyl |
| 329 | CHOH | 2-Ethoxy-4-pyridyl |
| 330 | CHOH | 2-Methylthio-4-pyridyl |
| 331 | CHOH | 2-Trifluormethyl-5-pyridyl |
| 332 | CHOH | 2-Pyrimidinyl |
| 333 | CHOH | 3-Pyrimidinyl |
| 334 | CHOH | 4-Pyrimidinyl |
| 335 | CHOH | 2-Pyrazinyl |
| 336 | CHOH | 3-Pyridazinyl |
| 337 | CHOH | 4-Pyridazinyl |
| 338 | CHOH | 2-(2*H*-1,3-oxazinyl) |
| 339 | CHOH | 2-(6*H*-1,3-oxazinyl) |
| 340 | CHOH | 4-(6*H*-1,3-oxazinyl) |
| 341 | CHOH | 6-(6*H*-1,3-oxazinyl) |
| 342 | CHOH | [1,3,5]-2-Trazinyl |
| 343 | CHOH | [1,2,4]-3-Triazinyl |
| 344 | CHOH | [1,2,4]-5-Triazinyl |
| 345 | CHOH | [1,2,4]-6-Triazinyl |
| 346 | CHOCH₃ | Oxiranyl |
| 347 | CHOCH₃ | 3-Methyl-2-oxiranyl |
| 348 | CHOCH₃ | 2-Oxetanyl |
| 349 | CHOCH₃ | 3-Hydroxy-3-methyl-2-oxetanyl |
| 350 | CHOCH₃ | 3-Hydroxy-3-ethyl-2-oxetanyl |
| 351 | CHOCH₃ | 3-Hydroxy-3-propyl-2-oxetanyl |
| 352 | CHOCH₃ | 3-Hydroxy-3-butyl-2-oxetanyl |
| 353 | CHOCH₃ | 3-Methoxy-3-methyl-2-oxetanyl |
| 354 | CHOCH₃ | 3-Methoxy-3-ethyl-2-oxetanyl |
| 355 | CHOCH₃ | 3-Methoxy-3-propyl-2-oxetanyl |
| 356 | CHOCH₃ | 3-Methoxy-3-butyl-2-oxetanyl |
| 357 | CHOCH₃ | 3-Trimethylsilyloxy-3-methyl-2-oxetanyl |
| 358 | CHOCH₃ | 3-Trimethylsilyloxy-3-ethyl-2-oxetanyl |
| 359 | CHOCH₃ | 3-Trimethylsilyloxy-3-propyl-2-oxetanyl |
| 360 | CHOCH₃ | 3-Trimethylsilyloxy-3-butyl-2-oxetanyl |
| 361 | CHOCH₃ | 3-Oxetanyl |
| 362 | CHOCH₃ | 2-Furyl |
| 363 | CHOCH₃ | 4,5-Dihydro-2-fluryl |
| 364 | CHOCH₃ | 2,3-Dihydro-2-furyl |
| 365 | CHOCH₃ | 3-Furyl |
| 366 | CHOCH₃ | 4,5-Dihydro-3-furyl |
| 367 | CHOCH₃ | 2,3-Dihydro-3-furyl |
| 368 | CHOCH₃ | 2-Thienyl |
| 369 | CHOCH₃ | 4,5-Dihydro-2-thienyl |
| 370 | CHOCH₃ | 2,3-Dihydro-2-thienyl |
| 371 | CHOCH₃ | 5-Chlor-2-thienyl |
| 372 | CHOCH₃ | 5-Methyl-2-thienyl |
| 373 | CHOCH₃ | 3-Thienyl |
| 374 | CHOCH₃ | 4,5-Dihydro-3-thienyl |
| 375 | CHOCH₃ | 2,3-Dihydro-3-thienyl |
| 376 | CHOCH₃ | 2-Pyrrolyl |
| 377 | CHOCH₃ | 2,5-Dihydro-2-pyrrolyl |
| 378 | CHOCH₃ | 3-Pyrrol |
| 379 | CHOCH₃ | 2,5-Dihydro-3-pyrrolyl |
| 380 | CHOCH₃ | 3-Isoxazolyl |
| 381 | CHOCH₃ | 4-Methyl-3-isoxazolyl |
| 382 | CHOCH₃ | 5-Methyl-3-isoxazolyl |
| 383 | CHOCH₃ | 4,5-Dimethyl-3-isoxazolyl |
| 384 | CHOCH₃ | 4,5-Dihydro-3-isoxazolyl |
| 385 | CHOCH₃ | 4-Methyl-4,5-dihydro-3-isoxazolyl |
| 386 | CHOCH₃ | 5-Methyl-4,5-dihydro-3-isoxazolyl |
| 387 | CHOCH₃ | 4,5-Dimethyl-4,5-dihydro-3-isoxazolyl |
| 388 | CHOCH₃ | 4-Isoxazolyl |
| 389 | CHOCH₃ | 3-Methyl-4 isoxazolyl |
| 390 | CHOCH₃ | 5-Methyl-4-isoxazolyl |
| 391 | CHOCH₃ | 5-Cyclopropyl-4-isoxazolyl |
| 392 | CHOCH₃ | 5-Phenyl-4-isoxazolyl |
| 393 | CHOCH₃ | 3,5-Dimethyl-4-isoxazolyl |
| 394 | CHOCH₃ | 4,5-Dihydro-4-isoxazolyl |
| 395 | CHOCH₃ | 3-Methyl-4,5-dihydro-4-isoxazolyl |
| 396 | CHOCH₃ | 5-Methyl-4,5-dihydro-4-isoxazolyl |
| 397 | CHOCH₃ | 3,5-Dimethyl-4,5-dihydro-4-isoxazolyl |
| 398 | CHOCH₃ | 5-Isoxazolyl |
| 399 | CHOCH₃ | 3-Methyl-5-isoxazolyl |
| 400 | CHOCH₃ | 4-Methyl-5-isoxazolyl |
| 401 | CHOCH₃ | 3,4-Dimethyl-5-isoxazolyl |
| 402 | CHOCH₃ | 4,5-Dihydro-5-isoxazolyl |
| 403 | CHOCH₃ | 3-Methyl-4,5-dihydro-5-isoxazolyl |
| 404 | CHOCH₃ | 4-Methyl-4,5-dihydro-5-isoxazolyl |
| 405 | CHOCH₃ | 3,4-Dimethyl-4,5-dihydro-5-isoxazolyl |
| 406 | CHOCH₃ | 3-Isothiazolyl |
| 407 | CHOCH₃ | 4-Methyl-3-isothiazolyl |
| 408 | CHOCH₃ | 5-Methyl-3-isothiazolyl |
| 409 | CHOCH₃ | 4-Isothiazolyl |
| 410 | CHOCH₃ | 3-Methyl-4-isothiazolyl |
| 411 | CHOCH₃ | 5-Methyl-4-isothiazolyl |
| 412 | CHOCH₃ | 5-Isothiazolyl |
| 413 | CHOCH₃ | 3-Methyl-5-isothiazolyl |
| 414 | CHOCH₃ | 4-Methyl-5-isothiazolyl |
| 415 | CHOCH₃ | 2-Oxazolyl |
| 416 | CHOCH₃ | 4-Oxazolyl |
| 417 | CHOCH₃ | 5-Oxazolyl |
| 418 | CHOCH₃ | 2-Thiazolyl |
| 419 | CHOCH₃ | 4-Thiazolyl |
| 420 | CHOCH₃ | 5-Thiazolyl |
| 421 | CHOCH₃ | 3-Pyrazolyl |
| 422 | CHOCH₃ | 4-Pyrazolyl |
| 423 | CHOCH₃ | 1-Methyl-3-pyrazolyl |
| 424 | CHOCH₃ | 1-Methyl-4-pyrazolyl |
| 425 | CHOCH₃ | 1-Methyl-5-pyrazolyl |
| 426 | CHOCH₃ | 2-Imidazolyl |
| 427 | CHOCH₃ | 1-Methyl-2-imidazolyl |
| 428 | CHOCH₃ | 5-Methyl-[1,3,4]-2-oxadiazolyl |
| 429 | CHOCH₃ | 5-Methyl-[1,2,4]-3-oxadiazolyl |
| 430 | CHOCH₃ | 5-Methyl-[1,3,4]-2-thiadiazolyl |
| 431 | CHOCH₃ | 5-Methyl-[1,2,4]-3-thiadiazolyl |
| 432 | CHOCH₃ | [1,2,4]-3-triazolyl |
| 433 | CHOCH₃ | [1,2,3]-4-triazolyl |
| 434 | CHOCH₃ | 2-Pyridyl |
| 435 | CHOCH₃ | 6-Chlor-2-pyridyl |
| 436 | CHOCH₃ | 6-Methoxy-2-pyridyl |
| 437 | CHOCH₃ | 6-Trifluorinethyl-2-pyridyl |
| 438 | CHOCH₃ | 3-Pyridyl |
| 439 | CHOCH₃ | 2-Chlor-3-pyridyl |
| 440 | CHOCH₃ | 2-Methoxy-3-pyridyl |
| 441 | CHOCH₃ | 4-Pyridyl |
| 442 | CHOCH₃ | 2-Chlor-4-pyridyl |
| 443 | CHOCH₃ | 2-Methoxy-4-pyridyl |
| 444 | CHOCH₃ | 2-Ethoxy-4-pyridyl |
| 445 | CHOCH₃ | 2-Methylthio-4-pyridyl |
| 446 | CHOCH₃ | 2-Trifluormethyl-5-pyridyl |
| 447 | CHOCH₃ | 2-Pyrimidinyl |
| 448 | CHOCH₃ | 3-Pyrimidinyl |
| 449 | CHOCH₃ | 4-Pyrimidinyl |
| 450 | CHOCH₃ | 2-Pyrazinyl |
| 451 | CHOCH₃ | 3-Pyridazinyl |
| 452 | CHOCH₃ | 4-Pyridazinyl |
| 453 | CHOCH₃ | 2-(2*H*-1,3-oxazinyl) |
| 454 | CHOCH₃ | 2-(6*H*-1,3-oxazinyl) |
| 455 | CHOCH₃ | 4-(6*H*-1,3-oxazinyl) |
| 456 | CHOCH₃ | 6-(6*H*-1,3-oxazinyl) |
| 457 | CHOCH₃ | [1,3,5]-2-Triazinyl |
| 458 | CHOCH₃ | [1,2,4]-3-Triazinyl |
| 459 | CHOCH₃ | [1,2,4]-5-Triazinyl |
| 460 | CHOCH₃ | [1,2,4]-6-Triazinyl |
| 461 | CHOCOCH₃ | Oxiranyl |
| 462 | CHOCOCH₃ | 3-Methyl-2-oxiranyl |
| 463 | CHOCOCH₃ | 2-Oxetanyl |
| 464 | CHOCOCH₃ | 3-Hydroxy-3-methyl-2-oxetanyl |
| 465 | CHOCOCH₃ | 3-Hydroxy-3-ethyl-2-oxetanyl |
| 466 | CHOCOCH₃ | 3-Hydroxy-3-propyl-2-oxetanyl |
| 467 | CHOCOCH₃ | 3-Hydroxy-3-butyl-2-oxetanyl |
| 468 | CHOCOCH₃ | 3-Methoxy-3-methyl-2-oxetanyl |
| 469 | CHOCOCH₃ | 3-Methoxy-3-ethyl-2-oxetanyl |
| 470 | CHOCOCH₃ | 3-Methoxy-3-propyl-2-oxetanyl |
| 471 | CHOCOCH₃ | 3-Methoxy-3-butyl-2-oxetanyl |
| 472 | CHOCOCH₃ | 3-Trimethylsilyloxy-3-methyl-2-oxetanyl |
| 473 | CHOCOCH₃ | 3-Trimethylsilyloxy-3-ethyl-2-oxetanyl |
| 474 | CHOCOCH₃ | 3-Trimethylsilyloxy-3-propyl-2-oxetanyl |
| 475 | CHOCOCH₃ | 3-Trimethylsilyloxy-3-butyl-2-oxetanyl |
| 476 | CHOCOCH₃ | 3-Oxetanyl |
| 477 | CHOCOCH₃ | 2-Furyl |
| 478 | CHOCOCH₃ | 4,5-Dihydro-2 furyl |
| 479 | CHOCOCH₃ | 2,3-Dihydro-2-furyl |
| 480 | CHOCOCH₃ | 3-furyl |
| 481 | CHOCOCH₃ | 4,5-Dihydro-3-furyl |
| 482 | CHOCOCH₃ | 2,3-Dihydro-3-furyl |
| 483 | CHOCOCH₃ | 2-Thienyl |
| 484 | CHOCOCH₃ | 4,5-Dihydro-2-thienyl |
| 485 | CHOCOCH₃ | 2,3-Dihydro-2-thienyl |
| 486 | CHOCOCH₃ | 5-Chlor-2-thienyl |
| 487 | CHOCOCH₃ | 5-Methyl-2-thienyl |
| 488 | CHOCOCH₃ | 3-Thienyl |
| 489 | CHOCOCH₃ | 4,5-Dihydro-3-thienyl |
| 490 | CHOCOCH₃ | 2,3-Dihydro-3-thienyl |
| 491 | CHOCOCH₃ | 2-Pyrrolyl |
| 492 | CHOCOCH₃ | 2,5-Dihydro-2-pyrrolyl |
| 493 | CHOCOCH₃ | 3-Pyrrol |
| 494 | CHOCOCH₃ | 2,5-Dihydro-3-pyrrolyl |
| 495 | CHOCOCH₃ | 3-Isoxazolyl |
| 496 | CHOCOCH₃ | 4-Methyl-3-isoxazolyl |
| 497 | CHOCOCH₃ | 5-Methyl-3-isoxazolyl |
| 498 | CHOCOCH₃ | 4,5-Dimethyl-3-isoxazolyl |
| 499 | CHOCOCH₃ | 4,5-Dihydro-3-isoxazolyl |
| 500 | CHOCOCH₃ | 4-Methyl-4,5-dihydro-3-isoxazolyl |
| 501 | CHOCOCH₃ | 5-Methyl-4,5-dihydro-3-isoxazolyl |
| 502 | CHOCOCH₃ | 4,5-Dimethyl-4,5-dihydro-3-isoxazolyl |
| 503 | CHOCOCH₃ | 4-Isoxazolyl |
| 504 | CHOCOCH₃ | 3-Methyl-4-isoxazolyl |
| 505 | CHOCOCH₃ | 5-Methyl-4-isoxazolyl |
| 506 | CHOCOCH₃ | 5-Cyclopropyl-4-isoxazolyl |
| 507 | CHOCOCH₃ | 5-Phenyl-4-isoxazolyl |
| 508 | CHOCOCH₃ | 3,5-Dimethyl-4-isoxazolyl |
| 509 | CHOCOCH₃ | 4,5-Dihydro-4-isoxazolyl |
| 510 | CHOCOCH₃ | 3-Methyl-4,5-dihydro-4-isoxazolyl |
| 511 | CHOCOCH₃ | 5-Methyl-4,5-dihydro-4-isoxazolyl |
| 512 | CHOCOCH₃ | 3,5-Dimethyl-4,5-dihydro-4-isoxazolyl |
| 513 | CHOCOCH₃ | 5-Isoxazolyl |
| 514 | CHOCOCH₃ | 3-Methyl-5-isoxazolyl |
| 515 | CHOCOCH₃ | 4-Methyl-5-isoxazolyl |
| 516 | CHOCOCH₃ | 3,4-Dimethyl-5-isoxazolyl |
| 517 | CHOCOCH₃ | 4,5-Dihydro-5-isoxazolyl |
| 518 | CHOCOCH₃ | 3-Methyl-4,5-dihydro-5-isoxazolyl |
| 519 | CHOCOCH₃ | 4-Methyl-4,5-dihydro-5-isoxazolyl |
| 520 | CHOCOCH₃ | 3,4-Dimethyl-4,5-dihydro-5-isoxazolyl |
| 521 | CHOCOCH₃ | 3-Isothiazolyl |
| 522 | CHOCOCH₃ | 4-Methyl-3-isothiazolyl |
| 523 | CHOCOCH₃ | 5-Methyl-3-isothiazolyl |
| 524 | CHOCOCH₃ | 4-Isothiazolyl |
| 525 | CHOCOCH₃ | 3-Methyl-4-isothiazolyl |
| 526 | CHOCOCH₃ | 5-Methyl-4-isothiazolyl |
| 527 | CHOCOCH₃ | 5-Isothiazolyl |
| 528 | CHOCOCH₃ | 3-Methyl-5-isothiazolyl |
| 529 | CHOCOCH₃ | 4-Methyl-5-isothiazolyl |
| 530 | CHOCOCH₃ | 2-Oxazolyl |
| 531 | CHOCOCH₃ | 4-Oxazolyl |
| 532 | CHOCOCH₃ | 5-Oxazolyl |
| 533 | CHOCOCH₃ | 2-Thiazolyl |
| 534 | CHOCOCH₃ | 4-Thiazolyl |
| 535 | CHOCOCH₃ | 5-Thiazolyl |
| 536 | CHOCOCH₃ | 3 Pyrazolyl |
| 537 | CHOCOCH₃ | 4-Pyrazolyl |
| 538 | CHOCOCH₃ | 1-Methyl-3-pyrazolyl |
| 539 | CHOCOCH₃ | 1-Methyl-4-pyrazolyl |
| 540 | CHOCOCH₃ | 1-Methyl-5-pyrazolyl |
| 541 | CHOCOCH₃ | 2-Imidazolyl |
| 542 | CHOCOCH₃ | 1-Methyl-2-imidazolyl |
| 543 | CHOCOCH₃ | 5-Methyl-[1,3,4]-2-oxadiazolyl |
| 544 | CHOCOCH₃ | 5-Methyl-[1,2,4]-3-oxadiazolyl |
| 545 | CHOCOCH₃ | 5-Methyl-[1,3,4]-2-thiadiazolyl |
| 546 | CHOCOCH₃ | 5-Methyl-[1,2,4]-3-thiadiazolyl |
| 547 | CHOCOCH₃ | [1,2,4]-3-triazolyl |
| 548 | CHOCOCH₃ | [1,2,3]-4-triazolyl |
| 549 | CHOCOCH₃ | 2-Pyridyl |
| 550 | CHOCOCH₃ | 6-Chlor-2-pyridyl |
| 551 | CHOCOCH₃ | 6-Methoxy-2-pyridyl |
| 552 | CHOCOCH₃ | 6-Trifluormethyl-2-pyridyl |
| 553 | CHOCOCH₃ | 3-Pyridyl |
| 554 | CHOCOCH₃ | 2-Chlor-3-pyridyl |
| 555 | CHOCOCH₃ | 2-Methoxy-3-pyridyl |
| 556 | CHOCOCH₃ | 4-Pyridyl |
| 557 | CHOCOCH₃ | 2-Chlor-4-pyridyl |
| 558 | CHOCOCH₃ | 2-Methoxy-4-pyridyl |
| 559 | CHOCOCH₃ | 2-Ethoxy-4-pyridyl |
| 560 | CHOCOCH₃ | 2-Methylthio-4-pyridyl |
| 561 | CHOCOCH₃ | 2-Trifluormethyl-5-pyridyl |
| 562 | CHOCOCH₃ | 2-Pyrimidinyl |
| 563 | CHOCOCH₃ | 3-Pyrimidinyl |
| 564 | CHOCOCH₃ | 4-Pyrimidinyl |
| 565 | CHOCOCH₃ | 2-Pyrazinyl |
| 566 | CHOCOCH₃ | 3-Pyridazinyl |
| 567 | CHOCOCH₃ | 4-Pyridazinyl |
| 568 | CHOCOCH₃ | 2-(2*H*-1,3-oxazinyl) |
| 569 | CHOCOCH₃ | 2-(6*H*-1,3-oxazinyl) |
| 570 | CHOCOCH₃ | 4-(6*H*-1,3-oxazinyl) |
| 571 | CHOCOCH₃ | 6-(6*H*-1,3-oxazinyl) |
| 572 | CHOCOCH₃ | [1,3,5]-2-Triazinyl |
| 573 | CHOCOCH₃ | [1,2,4]-3-Triazinyl |
| 574 | CHOCOCH₃ | [1,2,4]-5-Triazinyl |
| 575 | CHOCOCH₃ | [1,2,4]-6-Triazinyl |
| 576 | CHOSO₂CH₃ | Oxiranyl |
| 577 | CHOSO₂CH₃ | 3-Methyl-2-oxiranyl |
| 578 | CHOSO₂CH₃ | 2-Oxetanyl |
| 579 | CHOSO₂CH₃ | 3-Hydroxy-3-methyl-2-oxetanyl |
| 580 | CHOSO₂CH₃ | 3-Hydroxy-3-ethyl-2-oxetanyl |
| 581 | CHOSO₂CH₃ | 3-Hydroxy-3-propyl-2-oxetanyl |
| 582 | CHOSO₂CH₃ | 3-Hydroxy-3-butyl-2-oxetanyl |
| 583 | CHOSO₂CH₃ | 3-Methoxy-3-methyl-2-oxetanyl |
| 584 | CHOSO₂CH₃ | 3-Methoxy-3-ethyl-2-oxetanyl |
| 585 | CHOSO₂CH₃ | 3-Methoxy-3-propyl-2-oxetanyl |
| 586 | CHOSO₂CH₃ | 3-Methoxy-3-butyl-2-oxetanyl |
| 587 | CHOSO₂CH₃ | 3-Trimethylsilyloxy-3-methyl-2-oxetanyl |
| 588 | CHOSO₂CH₃ | 3-Trimethylsilyloxy-3-ethyl-2-oxetanyl |
| 589 | CHOSO₂CH₃ | 3-Trimethylsilyloxy-3-propyl-2-oxetanyl |
| 590 | CHOSO₂CH₃ | 3-Trimethylsilyloxy-3-butyl-2-oxetanyl |
| 591 | CHOSO₂CH₃ | 3-Oxetanyl |
| 592 | CHOSO₂CH₃ | 2-Furyl |
| 593 | CHOSO₂CH₃ | 4,5-Dihydro-2-furyl |
| 594 | CHOSO₂CH₃ | 2,3-Dihydro-2-furyl |
| 595 | CHOSO₂CH₃ | 3-Furyl |
| 596 | CHOSO₂CH₃ | 4,5-Dihydro-3-furyl |
| 597 | CHOSO₂CH₃ | 2,3-Dihydro-3-furyl |
| 598 | CHOSO₂CH₃ | 2-Thienyl |
| 599 | CHOSO₂CH₃ | 4,5-Dihydro-2-thienyl |
| 600 | CHOSO₂CH₃ | 2,3-Dihydro-2-thienyl |
| 601 | CHOSO₂CH₃ | 5-Chlor-2-thienyl |
| 602 | CHOSO₂CH₃ | 5-Methyl-2-thienyl |
| 603 | CHOSO₂CH₃ | 3-Thienyl |
| 604 | CHOSO₂CH₃ | 4,5-Dihydro-3-thienyl |
| 605 | CHOSO₂CH₃ | 2,3-Dihydro-3-thienyl |
| 606 | CHOSO₂CH₃ | 2-Pyrrolyl |
| 607 | CHOSO₂CH₃ | 2,5-Dihydro-2-pyrrolyl |
| 608 | CHOSO₂CH₃ | 3-Pyrrol |
| 609 | CHOSO₂CH₃ | 2,5-Dihydro-3-pyrolyl |
| 610 | CHOSO₂CH₃ | 3-Isoxazolyl |
| 611 | CHOSO₂CH₃ | 4-Methyl-3-isoxazolyl |
| 612 | CHOSO₂CH₃ | 5-Methyl-3-isoxazolyl |
| 613 | CHOSO₂CH₃ | 4,5-Dimethyl-3-isoxazolyl |
| 614 | CHOSO₂CH₃ | 4,5-Dihydro-3-isoxazolyl |
| 615 | CHOSO₂CH₃ | 4-Methyl-4,5-dihydro-3-isoxazolyl |
| 616 | CHOSO₂CH₃ | 5-Methyl-4,5-dihydro-3-isoxazolyl |
| 617 | CHOS0₂CH₃ | 4,5-Dimethyl-4,5-dihydro-3-isoxmlyl |
| 618 | CHOSO₂CH₃ | 4-Isoxazolyl |
| 619 | CHOSO₂CH₃ | 3-Methyl-4-isoxazolyl |
| 620 | CHOSO₂CH₃ | 5-Methyl-4-isoxazolyl |
| 621 | CHOSO₂CH₃ | 5-Cyclopropyl-4-isoxazolyl |
| 622 | CHOSO₂CH₃ | 5-Phenyl-4-isoxazolyl |
| 623 | CHOSO₂CH₃ | 3,5-Dimethyl-4-isoxazolyl |
| 624 | CHOSO₂CH₃ | 4,5-Dihydro-4-isoxazolyl |
| 625 | CHOSO₂CH₃ | 3-Methyl-4,5-dihydro-4-isoxazolyl |
| 626 | CHOSO₂CH₃ | 5-Methyl-4,5-dihydro-4-isoxazolyl |
| 627 | CHOSO₂CH₃ | 3,5-Dimethyl-4,5-dihydro-4-isoxazolyl |
| 628 | CHOSO₂CH₃ | 5-Isoxazolyl |
| 629 | CHOSO₂CH₃ | 3-Methyl-5-isoxazolyl |
| 630 | CHOSO₂CH₃ | 4-Methyl-5-isoxazolyl |
| 631 | CHOSO₂CH₃ | 3,4-Dimethyl-5-isoxazolyl |
| 632 | CHOSO₂CH₃ | 4,5-Dihydro-5-isoxazolyl |
| 633 | CHOSO₂CH₃ | 3-Methyl-4,5-dihydro-5-isoxazolyl |
| 634 | CHOSO₂CH₃ | 4-Methyl-4,5-dihydro-5-isoxazolyl |
| 635 | CHOSO₂CH₃ | 3,4-Dimethyl-4,5-dihydro-5-isoxazolyl |
| 636 | CHOSO₂CH₃ | 3-Isothiazolyl |
| 637 | CHOSO₂CH₃ | 4-Methyl-3-isothiazolyl |
| 638 | CHOSO₂CH₃ | 5-Methyl-3-isothiazolyl |
| 639 | CHOSO₂CH₃ | 4-Isothiazolyl |
| 640 | CHOSO₂CH₃ | 3-Methyl-4-isothiazolyl |
| 641 | CHOSO₂CH₃ | 5-Methyl-4-isothiazolyl |
| 642 | CHOSO₂CH₃ | 5-Isothiazolyl |
| 643 | CHOSO₂CH₃ | 3-Methyl-5-isothiazolyl |
| 644 | CHOSO₂CH₃ | 4-Methyl-5-isothiazolyl |
| 645 | CHOSO₂CH₃ | 2-Oxazolyl |
| 646 | CHOSO₂CH₃ | 4-Oxazolyl |
| 647 | CHOSO₂CH₃ | 5-Oxazolyl |
| 648 | CHOSO₂CH₃ | 2-Thiazolyl |
| 649 | CHOSO₂CH₃ | 4-Thiazolyl |
| 650 | CHOSO₂CH₃ | 5-Thiazolyl |
| 651 | CHOSO₂CH₃ | 3-Pyrazolyl |
| 652 | CHOSO₂CH₃ | 4-Pyrazolyl |
| 653 | CHOSO₂CH₃ | 1-Methyt-3-pyrazolyl |
| 654 | CHOSO₂CH₃ | 1-Methyl-4-pyrazolyl |
| 655 | CHOSO₂CH₃ | 1-Methyl-5-pyrazolyl |
| 656 | CHOSO₂CH₃ | 2-Imidazolyl |
| 657 | CHOSO₂CH₃ | 1-Methyl-2-imidazolyl |
| 658 | CHOSO₂CH₃ | 5-Methyl-[1,3,4]-2-oxadiazolyl |
| 659 | CHOSO₂CH₃ | 5-Methyl-[1,2,4]-3-oxadiazolyl |
| 660 | CHOSO₂CH₃ | 5-Methyl-[1,3,4]-2-thiadiazolyl |
| 661 | CHOSO₂CH₃ | 5-Methyl-[1,2,4]-3-thiadiazolyl |
| 662 | CHOSO₂CH₃ | [1,2,4]-3-triazolyl |
| 663 | CHOSO₂CH₃ | [1,2,3]-4-triazolyl |
| 664 | CHOSO₂CH₃ | 2-Pyridyl |
| 665 | CHOSO₂CH₃ | 6-Chlor-2-pyridyl |
| 666 | CHOSO₂CH₃ | 6-Methoxy-2-pyridyl |
| 667 | CHOSO₂CH₃ | 6-Trifluormethyl-2-pyridyl |
| 668 | CHOSO₂CH₃ | 3-Pyridyl |
| 669 | CHOSO₂CH₃ | 2-Chlor-3-pyridyl |
| 670 | CHOSO₂CH₃ | 2-Methoxy-3-pyridyl |
| 671 | CHOSO₂CH₃ | 4-Pyridyl |
| 672 | CHOSO₂CH₃ | 2-Chlor-4-pyridyl |
| 673 | CHOSO₂CH₃ | 2-Methoxy-4-pyridyl |
| 674 | CHOSO₂CH₃ | 2-Ethoxy-4-pyridyl |
| 675 | CHOSO₂CH₃ | 2-Methylthio-4-pyridyl |
| 676 | CHOSO₂CH₃ | 2-Trifluormethyl-5-pyridyl |
| 677 | CHOSO₂CH₃ | 2-Pyrimidinyl |
| 678 | CHOSO₂CH₃ | 3-Pyrimidinyl |
| 679 | CHOSO₂CH₃ | 4-Pyrimidinyl |
| 680 | CHOSO₂CH₃ | 2-Pyrazinyl |
| 681 | CHOSO₂CH₃ | 3-Pyridazinyl |
| 682 | CHOSO₂CH₃ | 4-Pyridazinyl |
| 683 | CHOSO₂CH₃ | 2-(2*H*-1,3-oxazinyl) |
| 684 | CHOSO₂CH₃ | 2-(6*H*-1,3-oxazinyl) |
| 685 | CHOSO₂CH₃ | 4-(6*H*-1,3-oxazinyl) |
| 686 | CHOSO₂CH₃ | 6-(6*H*-1,3-oxazinyl) |
| 687 | CHOSO₂CH₃ | [1,3,5]-2-Triazinyl |
| 688 | CHOSO₂CH₃ | [1,2,4]-3-Triazinyl |
| 689 | CHOSO₂CH₃ | [1,2,4]-5-Triazinyl |
| 690 | CHOSO₂CH₃ | [1,2,4]-6-Triazinyl \| |
| 691 | CH₂CH₂ | Oxiranyl |
| 692 | CH₂CH₂ | 3-Methyl-2-oxiranyl |
| 693 | CH₂CH₂ | 2-Oxetanyl |
| 694 | CH₂CH₂ | 3-Hydroxy-3-methyl-2-oxetanyl |
| 695 | CH₂CH₂ | 3-Hydroxy-3-ethyl-2-oxetanyl |
| 696 | CH₂CH₂ | 3-Hydroxy-3-propyl-2-oxetanyl |
| 697 | CH₂CH₂ | 3-Hydroxy-3-butyl-2-oxetanyl |
| 698 | CH₂CH₂ | 3-Methoxy-3-methyl-2-oxetanyl |
| 699 | CH₂CH₂ | 3-Methoxy-3-ethyl-2-oxetanyl |
| 700 | CH₂CH₂ | 3-Methoxy-3-propyl-2-oxetanyl |
| 701 | CH₂CH₂ | 3-Methoxy-3-butyl-2-oxetanyl |
| 702 | CH₂CH₂ | 3-Trimethylsilyloxy-3-methyl-2-oxetanyl |
| 703 | CH₂CH₂ | 3-Trimethylsilyloxy-3-ethyl-2-oxetanyl |
| 704 | CH₂CH₂ | 3-Trimethylsilyloxy-3-propyl-2-oxetanyl |
| 705 | CH₂CH₂ | 3-Trimethylsilyloxy-3-butyl-2-oxetanyl |
| 706 | CH₂CH₂ | 3-Oxetanyl |
| 707 | CH₂CH₂ | 2-Furyl |
| 708 | CH₂CH₂ | 4,5-Dihydro-2-furyl |
| 709 | CH₂CH₂ | 2,3-Dihydro-2-furyl |
| 710 | CH₂CH₂ | 3-Furyl |
| 711 | CH₂CH₂ | 4,5-Dihydro-3-furyl |
| 712. | CH₂CH₂ | 2,3-Dihydro-3-furyl |
| 713 | CH₂CH₂ | 2-Thienyl |
| 714 | CH₂CH₂ | 4,5-Dihydro-2-thienyl |
| 715 | CH₂CH₂ | 2,3-Dihydro-2-thienyl |
| 716 | CH₂CH₂ | 5-Chlor-2-thienyl |
| 717 | CH₂CH₂ | 5-Methyl-2-thienyl |
| 718 | CH₂CH₂ | 3-Thienyl |
| 719 | CH₂CH₂ | 4,5-Dihydro-3-thienyl |
| 720 | CH₂CH₂ | 2,3-Dihydro-3-thienyl |
| 721 | CH₂CH₂ | 2-Pyrrolyl |
| 722 | CH₂CH₂ | 2,5-Dihydro-2-pyrrolyl |
| 723 | CH₂CH₂ | 3-Pyrrol |
| 724 | CH₂CH₂ | 2,5-Dihydro-3-pyrrolyl |
| 725 | CH₂CH₂ | 3-Isoxazolyl |
| 726 | CH₂CH₂ | 4-Methyl-3-isoxazolyl |
| 727 | CH₂CH₂ | 5-Methyl-3-isoxazolyl |
| 728 | CH₂CH₂ | 4,5-Dimethyl-3-isoxazolyl |
| 729 | CH₂CH₂ | 4,5-Dihydro-3-isoxazolyl |
| 730 | CH₂CH₂ | 4-Methyl-4,5-dihydro-3-isoxazolyl |
| 731 | CH₂CH₂ | 5-Methyt-4,5-dihydro-3-isoxazolyl |
| 732 | CH₂CH₂ | 4,5-Dimethyl-4,5-dihydro-3-isoxazolyl |
| 733 | CH₂CH₂ | 4-Isoxazolyl |
| 734 | CH₂CH₂ | 3-Methyl-4-isoxazolyl |
| 735 | CH₂CH₂ | 5-Methyl-4-isoxazolyl |
| 736 | CH₂CH₂ | 5-Cyclopropyl-4-isoxazolyl |
| 737 | CH₂CH₂ | 5-Phenyl-4-isoxazolyl |
| 738 | CH₂CH₂ | 3,5-Dimethyl-4-isoxazolyl |
| 739 | CH₂CH₂ | 4,5-Dihydro-4-isoxazolyl |
| 740 | CH₂CH₂ | 3-Methyl-4,5-dihydro-4-isoxazolyl |
| 741 | CH₂CH₂ | 5-Methyl-4,5-dihydro-4-isoxazolyl |
| 742 | CH₂CH₂ | 3,5-Dimethyl-4,5-dihydro-4 isoxazolyl |
| 743 | CH₂CH₂ | 5-Isoxazolyl |
| 744 | CH₂CH₂ | 3-Methyl-5-isoxazolyl |
| 745 | CH₂CH₂ | 4-Methyl-5-isoxazolyl |
| 746 | CH₂CH₂ | 3,4-Dimethyl-5-isoxazolyl |
| 747 | CH₂CH₂ | 4,5-Dihydro-5-isoxazolyl |
| 748 | CH₂CH₂ | 3-Methyl-4,5-dihydro-5-isoxazolyl |
| 749 | CH₂CH₂ | 4-Methyl-4,5-dihydro-5-isoxazolyl |
| 750 | CH₂CH₂ | 3,4-Dimethyl-4,5-dihydro-5-isoxazolyl |
| 751 | CH₂CH₂ | 3-isothiazolyl |
| 752 | CH₂CH₂ | 4-Methyl-3-isothiazolyl |
| 753 | CH₂CH₂ | 5-Methyl-3-isothiazolyl |
| 754 | CH₂CH₂ | 4-Isothiazolyl |
| 755 | CH₂CH₂ | 3-Methyl-4-isothiazolyl |
| 756 | CH₂CH₂ | 5-Methyl-4-isothiazolyl |
| 757 | CH₂CH₂ | 5-Isothiazolyl |
| 758 | CH₂CH₂ | 3-Methyl-5-isothiazolyl |
| 759 | CH₂CH₂ | 4-Methyl-5-isothiazolyl |
| 760 | CH₂CH₂ | 2-Oxazolyl |
| 761 | CH₂CH₂ | 4-Oxazolyl |
| 762 | CH₂CH₂ | 5-Oxazolyl |
| 763 | CH₂CH₂ | 2-Thiazolyl |
| 764 | CH₂CH₂ | 4-Thiazolyl |
| 765 | CH₂CH₂ | 5-Thiazolyl |
| 766 | CH₂CH₂ | 3-Pyrazolyl |
| 767 | CH₂CH₂ | 4-Pyrazolyl |
| 768 | CH₂CH₂ | 1-Methyl-3-pyrazolyl |
| 769 | CH₂CH₂ | 1-Methyl-4-pyrazolyl |
| 770 | CH₂CH₂ | 1-Methyl-5-pyrazolyl |
| 771 | CH₂CH₂ | 2-Imidazolyl |
| 772 | CH₂CH₂ | 1-Methyl-2-imidazolyl |
| 773 | CH₂CH₂ | 5-Methyl-[1,3,4]-2-oxadiazolyl |
| 774 | CH₂CH₂ | 5-Methyl-[1,2,4]-3-oxadiazolyl |
| 775 | CH₂CH₂ | 5-Methyl-[1,3,4]-2-thiadiazolyl |
| 776 | CH₂CH₂ | 5-Methyl-[1,2,4]-3-thiadiazolyl |
| 777 | CH₂CH₂ | [1.2,4]-3-triazolyl |
| 778 | CH₂CH₂ | [1,2,3]-4-triazolyl |
| 779 | CH₂CH₂ | 2-Pyridyl |
| 780 | CH₂CH₂ | 6-Chlor-2-pyridyl |
| 781 | CH₂CH₂ | 6-Methoxy-2-pyridyl |
| 782 | CH₂CH₂ | 6-Trifluormethyl-2-pyridyl |
| 783 | CH₂CH₂ | 3-Pyridyl |
| 784 | CH₂CH₂ | 2-Chlor-3-pyridyl |
| 785 | CH₂CH₂ | 2-Methoxy-3-pyridyl |
| 786 | CH₂CH₂ | 4-Pyridyl |
| 787 | CH₂CH₂ | 2-Chlor-4-pyridyl |
| 788 | CH₂CH₂ | 2-Methoxy-4-pyridyl |
| 789 | CH₂CH₂ | 2-Ethoxy-4-pyridyl |
| 790 | CH₂CH₂ | 2-Methylthio-4-pyridyl |
| 791 | CH₂CH₂ | 2-Trifluormethyl-5-pyridyl |
| 792 | CH₂CH₂ | 2-Pyrimidinyl |
| 793 | CH₂CH₂ | 3-Pyrimidinyl |
| 794 | CH₂CH₂ | 4-Pyrimidinyl |
| 795 | CH₂CH₂ | 2-Pyrazinyl |
| 796 | CH₂CH₂ | 3-Pyridazinyl |
| 797 | CH₂CH₂ | 4-Pyridazinyl |
| 798 | CH₂CH₂ | 2-(2*H*-1,3-oxazinyl) |
| 799 | CH₂CH₂ | 2-(6*H*-1,3-oxazinyl) |
| 800 | CH₂CH₂ | 4-(6*H*-1,3-oxazinyl) |
| 801 | CH₂CH₂ | 6-(6*H*-1,3-oxazinyl) |
| 802 | CH₂CH₂ | [1,3,5]-2-Triazinyl |
| 803 | CH₂CH₂ | [1,2,4]-3-Triazinyl |
| 804 | CH₂CH₂ | [1,2,4]-5-Triazinyl |
| 805 | CH₂CH₂ | [1,2,4]-6-Triazinyl |
| 806 | ―C≡C― | Oxiranyl |
| 807 | ―C≡C― | 3-Methyl-2-oxiranyl |
| 808 | ―C≡C― | 2-Oxetanyl |
| 809 | ―C≡C― | 3-Hydroxy-3-methyl-2-oxetanyl |
| 810 | ―C≡C― | 3-Hydroxy-3-ethyl-2-oxetanyl |
| 811 | ―C≡C― | 3-Hydroxy-3-propyl-2-oxetanyl |
| 812 | ―C≡C― | 3-Hydroxy-3-butyl-2-oxetanyl |
| 813 | ―C≡C― | 3-Methoxy-3-methyl-2-oxetanyl |
| 814 | ―C≡C― | 3-Methoxy-3-ethyl-2-oxetanyl |
| 815 | ―C≡C― | 3-Methoxy-3-propyl-2-oxetanyl |
| 816 | ―C≡C― | 3-Methoxy-3-butyl-2-oxetanyl |
| 817 | ―C≡C― | 3-Trimethylsilyloxy-3-methyl-2-oxetanyl |
| 818 | ―C≡C― | 3-Trimethylsilyloxy-3-ethyl-2-oxetanyl |
| 819 | ―C≡C― | 3-Trimethylsilyloxy-3-propyl-2-oxetanyl |
| 820 | ―C≡C― | 3-Trimethylsilyloxy-3-butyl-2-oxetanyl |
| 821 | ―C≡C― | 3-Oxetanyl |
| 822 | ―C≡C― | 2-Furyl |
| 823 | ―C≡C― | 4,5-Dihydro-2-furyl |
| 824 | ―C≡C― | 2,3-Dihydro-2-furyl |
| 825 | ―C≡C― | 3-Furyl |
| 826 | ―C≡C― | 4,5-Dihydro-3-furyl |
| 827 | ―C≡C― | 2,3-Dihydro-3-furyl |
| 828 | ―C≡C― | 2-Thienyl |
| 829 | ―C≡C― | 4,5-Dihydro-2-thienyl |
| 830 | ―C≡C― | 2,3-Dihydro-2-thienyl |
| 831 | ―C≡C― | 5-Chlor-2-thienyl |
| 832 | ―C≡C― | 5-Methyl-2-thienyl |
| 833 | ―C≡C― | 3-Thienyl |
| 834 | ―C≡C― | 4,5-Dihydro-3-thienyl |
| 835 | ―C≡C― | 2,3-Dihydro-3-thienyl |
| 836 | ―C≡C― | 2-Pyrrolyl |
| 837 | ―C≡C― | 2,5-Dihydro-2-pyrrolyl |
| 838 | ―C≡C― | 3-Pyrrol |
| 839 | ―C≡C― | 2,5-Dihydro-3-pyrrolyl |
| 840 | ―C≡C― | 3-Isoxazolyl |
| 841 | ―C≡C― | 4-Methyl-3-isoxazolyl |
| 842 | ―C≡C― | 5-Methyl-3-isoxazolyl |
| 843 | ―C≡C― | 4,5-Dimethyl-3-isoxazolyl |
| 844 | ―C≡C― | 4,5-Dihydro-3-isoxazolyl |
| 845 | ―C≡C― | 4-Methyl-4,5-dihydro-3-isoxazolyl |
| 846 | ―C≡C― | 5-Methyl-4,5-dihydro-3-isoxazolyl |
| 847 | ―C≡C― | 4,5-Dimethyl-4,5-dihydro-3-isoxazolyl |
| 848 | ―C≡C― | 4-Isoxazolyl |
| 849 | ―C≡C― | 3-Methyl-4-isoxazolyl |
| 850 | ―C≡C― | 5-Methyl-4-isoxazolyl |
| 851 | ―C≡C― | 5-Cyclopropyl-4-isoxazolyl |
| 852 | ―C≡C― | 5-Phenyl-4-isoxazolyl |
| 853 | ―C≡C― | 3,5-Dimethyl-4-isoxazolyl |
| 854 | ―C≡C― | 4,5-Dihydro-4-isoxazolyl |
| 855 | ―C≡C― | 3-Methyl-4,5-dihydro-4-isoxazolyl |
| 856 | ―C≡C― | 5-Methyl-4,5-dihydro-4-isoxazolyl |
| 857 | ―C≡C― | 3,5-Dimethyl-4,5-dihydro-4-isoxazolyl |
| 858 | ―C≡C― | 5-Isoxazolyl |
| 859 | ―C≡C― | 3-Methyl-5-isoxazolyl |
| 860 | ―C≡C― | 4-Methyl-5-isoxazolyl |
| 861 | ―C≡C― | 3,4-Dimethyl-5-isoxazolyl |
| 862 | ―C≡C― | 4,5-Dihydro-5-isoxazolyl |
| 863 | ―C≡C― | 3-Methyl-4,5-dihydro-5-isoxazoly |
| 864 | ―C≡C― | 4-Methyl-4,5-dihydro-5-isoxazolyl |
| 865 | ―C≡C― | 3,4-Dimethyl-4,5-dihydro-5-isoxazolyl |
| 866 | ―C≡C― | 3-Isothiazolyl |
| 867 | ―C≡C― | 4-Methyl-3-isothiazolyl |
| 868 | ―C≡C― | 5-Methyl-3-isothiazolyl |
| 869 | ―C≡C― | 4-Isothiazolyl |
| 870 | ―C≡C― | 3-Methyl-4-isothiazolyl |
| 871 | ―C≡C― | 5-Methyl-4-isothiazolyl |
| 872 | ―C≡C― | 5-Isothiazolyl |
| 873 | ―C≡C― | 3-Methyl-5-isothiazolyl |
| **874** | ―C≡C― | 4-Methyl-5-isothiazolyl |
| **875** | ―C≡C― | 2-Oxazolyl |
| **876** | ―C≡C― | 4-Oxazolyl |
| **877** | ―C≡C― | 5-Oxazolyl |
| **878** | ―C≡C― | 2-Thiazolyl |
| **879** | ―C≡C― | 4-Thiazolyl |
| **880** | ―C≡C― | 5-Thiazolyl |
| **881** | ―C≡C― | 3-Pyrazolyl |
| **882** | ―C≡C― | 4-Pyrazolyl |
| 883 | ―C≡C― | 1-Methyl-3-pyrazolyl |
| 884 | ―C≡C― | 1-Methyl-4-pyrazolyl |
| 885 | ―C≡C― | 1-Methyl-5-pyrazolyl |
| 886 | ―C≡C― | 2-Imidazolyl |
| 887 | ―C≡C― | 1-Methyl-2-imidazolyl |
| 888 | ―C≡C― | 5-Methyl-[1,3,4]-2-oxadiazolyl |
| 889 | ―C≡C― | 5-Methyl-[1,2,4]-3-oxadiazolyl |
| 890 | ―C≡C― | 5-Methyl-[1,3,4]-2-thiadiazolyl |
| 891 | ―C≡C― | 5-Methyl-[1,2,4]-3-thiadiazolyl |
| 892 | ―C≡C― | [1,2,4]-3-triazolyl |
| 893 | ―C≡C― | [1,2,3]-4-triazolyl |
| 894 | ―C≡C― | 2-Pyridyl |
| 895 | ―C≡C― | 6-Chlor-2-pyridyl |
| 896 | ―C≡C― | 6-Methoxy-2-pyridyl |
| 897 | ―C≡C― | 6-Trifluormethyl-2-pyridyl |
| 898 | ―C≡C― | 3-Pyridyl |
| 899 | ―C≡C― | 2-Chlor-3-pyridyl |
| 900 | ―C≡C― | 2-Methoxy-3-pyridyl |
| 901 | ―C≡C― | 4-Pyridyl |
| 902 | ―C≡C― | 2-Chlor-4-pyridyl |
| 903 | ―C≡C― | 2-Methoxy-4-pyridyl |
| 904 | ―C≡C― | 2-Ethoxy-4-pyridyl |
| 905 | ―C≡C― | 2-Methylthio-4-pyridyl |
| 906 | ―C≡C― | 2-Trifluormethyl-5-pyridyl |
| 907 | ―C≡C― | 2-Pyrimidinyl |
| 908 | ―C≡C― | 3-Pyrimidinyl |
| 909 | ―C≡C― | 4-Pyrimidinyl |
| 910 | ―C≡C― | 2-Pyrazinyl |
| 911 | ―C≡C― | 3-Pyridazinyl |
| 912 | ―C=C― | 4-Pyridazinyl |
| 913 | ―C≡C― | 2-(2*H*-1,3-oxazinyl) |
| 914 | ―C≡C― | 2-(6*H*-1,3-oxazinyl) |
| 915 | ―C≡C― | 4-(6*H*-1,3-oxazinyl) |
| 916 | ―C≡C― | 6-(6*H*-1,3-oxazinyl) |
| 917 | ―C≡C― | [1,3,5]-2-Triazinyl |
| 918 | ―C≡C― | [1,2,4]-3-Triazinyl |
| 919 | ―C≡C― | [1,2,4]-5-Triazinyl |
| 920 | ―C≡C― | [1,2,4]-6-Tnazinyl |

Die folgenden Tabellen 1 - 144 basieren auf den 4-Benzoyl-pyrazolen der Formel Ib.

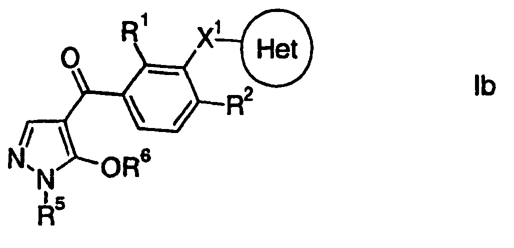

### Tabelle 1: Verbindungen 1.1 - 1.920

Verbindungen der allgemeinen Formel Ib, in der R¹ Chlor und R² Chlor, R⁵ Methyl und R⁶ Wasserstoff bedeutet und die Substituente X¹ und Het für jede einzelne Verbindung jeweils einer Zeile der Tabelle A entsprechen.

### Tabelle 2: Verbindungen 2.1 - 2.920

Verbindungen der allgemeinen Formel Ib, in der R¹ Chlor und R² Chlor, R⁵ Ethyl und R⁶ Wasserstoff bedeutet und die Substituente X¹ und Het für jede einzelne Verbindung jeweils einer Zeile der Tabelle A entsprechen.

### Tabelle 3: Verbindungen 3.1 - 3.920

Verbindungen der allgemeinen Formel Ib, in der R¹ Chlor und R² Chlor, R⁵ *n*-Propyl und R⁶ Wasserstoff bedeutet und die Substituente X¹ und Het für jede einzelne Verbindung jeweils einer Zeile der Tabelle A entsprechen.

### Tabelle 4: Verbindungen 4.1 - 4.920

Verbindungen der allgemeinen Formel Ib, in der R¹ Chlor und R² Chlor, R⁵ Methyl und R⁶ Methyl bedeutet und die Substituente X¹ und Het für jede einzelne Verbindung jeweils einer Zeile der Tabelle A entsprechen.

### Tabelle 5: Verbindungen 5.1 - 5.920

Verbindungen der allgemeinen Formel Ib, in der R¹ Chlor und R² Chlor, R⁵ Ethyl und R⁶ Methyl bedeutet und die Substituente X¹ und Het für jede einzelne Verbindung jeweils einer Zeile der Tabelle A entsprechen.

### Tabelle 6: Verbindungen 6.1 - 6.920

Verbindungen der allgemeinen Formel Ib, in der R¹ Chlor und R² Chlor, R⁵ *n*-Propyl und R⁶ Methyl bedeutet und die Substituente X¹ und Het für jede einzelne Verbindung jeweils einer Zeile der Tabelle A entsprechen.

### Tabelle 7: Verbindungen 7.1 - 7.920

Verbindungen der allgemeinen Formel Ib, in der R¹ Chlor und R² Chlor, R⁵ Methyl und R⁶ Ethyl bedeutet und die Substituente X¹ und Het für jede einzelne Verbindung jeweils einer Zeile der Tabelle A entsprechen.

### Tabelle 8: Verbindungen 8.1 - 8.920

Verbindungen der allgemeinen Formel Ib, in der R¹ Chlor und R² Chlor, R⁵ Ethyl und R⁶ Ethyl bedeutet und die Substituente X¹ und Het für jede einzelne Verbindung jeweils einer Zeile der Tabelle A entsprechen.

### Tabelle 9: Verbindungen 9.1 - 9.920

Verbindungen der allgemeinen Formel Ib, in der R¹ Chlor und R² Chlor, R⁵ n-Propyl und R⁶ Ethyl bedeutet und die Substituente X¹ und Het für jede einzelne Verbindung jeweils einer Zeile der Tabelle A entsprechen.

### Tabelle 10: Verbindungen 10.1 - 10.920

Verbindungen der allgemeinen Formel Ib, in der R¹ Chlor und R² Chlor, R⁵ Methyl und R⁶ Methylcarbonyl bedeutet und die Substituente X¹ und Het für jede einzelne Verbindung jeweils einer Zeile der Tabelle A entsprechen.

### Tabelle 11: Verbindungen 11.1 - 11.920

Verbindungen der allgemeinen Formel Ib, in der R¹ Chlor und R² Chlor, R⁵ Ethyl und R⁶ Methylcarbonyl bedeutet und die Substituente X¹ und Het für jede einzelne Verbindung jeweils einer Zeile der Tabelle A entsprechen.

### Tabelle 12: Verbindungen 12.1 - 12.920

Verbindungen der allgemeinen Formel Ib, in der R¹ Chlor und R² Chlor, R⁵ *n*-Propyl und R⁶ Methylcarbonyl bedeutet und die Substituente X¹ und Het für jede einzelne Verbindung jeweils einer Zeile der Tabelle A entsprechen.

### Tabelle 13: Verbindungen 13.1 - 13.920

Verbindungen der allgemeinen Formel Ib, in der R¹ Chlor und R² Chlor, R⁵ Methyl und R⁶ Ethylcarbonyl bedeutet und die Substituente X¹ und Het für jede einzelne Verbindung jeweils einer Zeile der Tabelle A entsprechen.

### Tabelle 14: Verbindungen 14.1 - 14.920

Verbindungen der allgemeinen Formel Ib, in der R¹ Chlor und R² Chlor, R⁵ Ethyl und R⁶ Ethylcarbonyl bedeutet und die Substituente X¹ und Het für jede einzelne Verbindung jeweils einer Zeile der Tabelle A entsprechen.

### Tabelle 15: Verbindungen 15.1 - 15.920

Verbindungen der allgemeinen Formel Ib, in der R¹ Chlor und R² Chlor, R⁵ n-Propyl und R⁶ Ethylcarbonyl bedeutet und die Substituente X¹ und Het für jede einzelne Verbindung jeweils einer Zeile der Tabelle A entsprechen.

### Tabelle 16: Verbindungen 16.1 - 16.920

Verbindungen der allgemeinen Formel Ib, in der R¹ Chlor und R² Chlor, R⁵ Methyl und R⁶ Methylsulfonyl bedeutet und die Substituente X¹ und Het für jede einzelne Verbindung jeweils einer Zeile der Tabelle A entsprechen.

### Tabelle 17: Verbindungen 17.1 - 17.920

Verbindungen der allgemeinen Formel Ib, in der R¹ Chlor und R² Chlor, R⁵ Ethyl und R⁶ Methylsulfonyl bedeutet und die Substituente X¹ und Het für jede einzelne Verbindung jeweils einer Zeile der Tabelle A entsprechen.

### Tabelle 18: Verbindungen 18.1 - 18.920

Verbindungen der allgemeinen Formel Ib, in der R¹ Chlor und R² Chlor, R⁵ n-Propyl und R⁶ Methylsulfonyl bedeutet und die Substituente X¹ und Het für jede einzelne Verbindung jeweils einer Zeile der Tabelle A entsprechen.

### Tabelle 19: Verbindungen 19.1 - 19.920

Verbindungen der allgemeinen Formel Ib, in der R¹ Chlor und R² Chlor, R⁵ Methyl und R⁶ Ethylsulfonyl bedeutet und die Substituente X¹ und Het für jede einzelne Verbindung jeweils einer Zeile der Tabelle A entsprechen.

### Tabelle 20: Verbindungen 20.1 - 20.920

Verbindungen der allgemeinen Formel Ib, in der R¹ Chlor und R² Chlor, R⁵ Ethyl und R⁶ Ethylsulfonyl bedeutet und die Substituente X¹ und Het für jede einzelne Verbindung jeweils einer Zeile der Tabelle A entsprechen.

### Tabelle 21: Verbindungen 21.1 - 21.920

Verbindungen der allgemeinen Formel Ib, in der R¹ Chlor und R² Chlor, R⁵ n-Propyl und R⁶ Ethylsulfonyl bedeutet und die Substituente X¹ und Het für jede einzelne Verbindung jeweils einer Zeile der Tabelle A entsprechen.

### Tabelle 22: Verbindungen 22.1 - 22.920

Verbindungen der allgemeinen Formel Ib, in der R¹ Chlor und R² Chlor, R⁵ Methyl und R⁶ 4-Methylphenylsulfonyl bedeutet und die Substituente X¹ und Het für jede einzelne Verbindung jeweils einer Zeile der Tabelle A entsprechen.

### Tabelle 23: Verbindungen 23.1 - 23.920

Verbindungen der allgemeinen Formel Ib, in der R¹ Chlor und R² Chlor, R⁵ Ethyl und R⁶ 4-Methylphenylsulfonyl bedeutet und die Substituente X¹ und Het für jede einzelne Verbindung jeweils einer Zeile der Tabelle A entsprechen.

### Tabelle 24: Verbindungen 24.1 - 24.920

Verbindungen der allgemeinen Formel Ib, in der R¹ Chlor und R² Chlor, R⁵ n-Propyl und R⁶ 4-Methylphenylsulfonyl bedeutet und die Substituente X¹ und Het für jede einzelne Verbindung jeweils einer Zeile der Tabelle A entsprechen.

### Tabelle 25: Verbindungen 25.1 - 25.920

Verbindungen der allgemeinen Formel Ib, in der R¹ Chlor und R² Methylsulfonyl, R⁵ Methyl und R⁶ Wasserstoff bedeutet und die Substituente X¹ und Het für jede einzelne Verbindung jeweils einer Zeile der Tabelle A entsprechen.

### Tabelle 26: Verbindungen 26.1 - 26.920

Verbindungen der allgemeinen Formel Ib, in der R¹ Chlor und R² Methylsulfonyl, R⁵ Ethyl und R⁶ Wasserstoff bedeutet und die Substituente X¹ und Het für jede einzelne Verbindung jeweils einer Zeile der Tabelle A entsprechen.

### Tabelle 27: Verbindungen 27.1 - 27.920

Verbindungen der allgemeinen Formel Ib, in der R¹ Chlor und R² Methylsulfonyl, R⁵ *n*-Propyl und R⁶ Wasserstoff bedeutet und die Substituente X¹ und Het für jede einzelne Verbindung jeweils einer Zeile der Tabelle A entsprechen.

### Tabelle 28: Verbindungen 28.1 - 28.920

Verbindungen der allgemeinen Formel Ib, in der R¹ Chlor und R² Methylsulfonyl, R⁵ Methyl und R⁶ Methyl bedeutet und die Substituente X¹ und Het für jede einzelne Verbindung jeweils einer Zeile der Tabelle A entsprechen.

### Tabelle 29: Verbindungen 29.1 - 29.920

Verbindungen der allgemeinen Formel Ib, in der R¹ Chlor und R² Methylsulfonyl, R⁵ Ethyl und R⁶ Methyl bedeutet und die Substituente X¹ und Het für jede einzelne Verbindung jeweils einer Zeile der Tabelle A entsprechen.

### Tabelle 30: Verbindungen 30.1 - 30.920

Verbindungen der allgemeinen Formel Ib, in der R¹ Chlor und R² Methylsulfonyl, R⁵ n-Propyl und R⁶ Methyl bedeutet und die Substituente X¹ und Het für jede einzelne Verbindung jeweils einer Zeile der Tabelle A entsprechen.

### Tabelle 31: Verbindungen 31.1 - 31.920

Verbindungen der allgemeinen Formel Ib, in der R¹ Chlor und R² Methylsulfonyl, R⁵ Methyl und R⁶ Ethyl bedeutet und die Substituente X¹ und Het für jede einzelne Verbindung jeweils einer Zeile der Tabelle A entsprechen.

### Tabelle 32: Verbindungen 32.1 - 32.920

Verbindungen der allgemeinen Formel Ib, in der R¹ Chlor und R² Methylsulfonyl, R⁵ Ethyl und R⁶ Ethyl bedeutet und die Substituente X¹ und Het für jede einzelne Verbindung jeweils einer Zeile der Tabelle A entsprechen.

### Tabelle 33: Verbindungen 33.1 - 33.920

Verbindungen der allgemeinen Formel Ib, in der R¹ Chlor und R² Methylsulfonyl, R⁵ n-Propyl und R⁶ Ethyl bedeutet und die Substituente X¹ und Het für jede einzelne Verbindung jeweils einer Zeile der Tabelle A entsprechen.

### Tabelle 34: Verbindungen 34.1 - 34.920

Verbindungen der allgemeinen Formel Ib, in der R¹ Chlor und R² Methylsulfonyl, R⁵ Methyl und R⁶ Methylcarbonyl bedeutet und die Substituente X¹ und Het für jede einzelne Verbindung jeweils einer Zeile der Tabelle A entsprechen.

### Tabelle 35: Verbindungen 35.1 - 35.920

Verbindungen der allgemeinen Formel Ib, in der R¹ Chlor und R² Methylsulfonyl, R⁵ Ethyl und R⁶ Methylcarbonyl bedeutet und die Substituente X¹ und Het für jede einzelne Verbindung jeweils einer Zeile der Tabelle A entsprechen.

### Tabelle 36: Verbindungen 36.1 - 36.920

Verbindungen der allgemeinen Formel Ib, in der R¹ Chlor und R² Methylsulfonyl, R⁵ n-Propyl und R⁶ Methylcarbonyl bedeutet und die Substituente X¹ und Het für jede einzelne Verbindung jeweils einer Zeile der Tabelle A entsprechen.

### Tabelle 37: Verbindungen 37.1 - 37.920

Verbindungen der allgemeinen Formel Ib, in der R¹ Chlor und R² Methylsulfonyl, R⁵ Methyl und R⁶ Ethylcarbonyl bedeutet und die Substituente X¹ und Het für jede einzelne Verbindung jeweils einer Zeile der Tabelle A entsprechen.

### Tabelle 38: Verbindungen 38.1 - 38.920

Verbindungen der allgemeinen Formel Ib, in der R¹ Chlor und R² Methylsulfonyl, R⁵ Ethyl und R⁶ Ethylcarbonyl bedeutet und die Substituente X¹ und Het für jede einzelne Verbindung jeweils einer Zeile der Tabelle A entsprechen.

### Tabelle 39: Verbindungen 39.1 - 39.920

Verbindungen der allgemeinen Formel Ib, in der R¹ Chlor und R² Methylsulfonyl, R⁵ n-Propyl und R⁶ Ethylcarbonyl bedeutet und die Substituente X¹ und Het für jede einzelne Verbindung jeweils einer Zeile der Tabelle A entsprechen.

### Tabelle 40: Verbindungen 40.1 - 40.920

Verbindungen der allgemeinen Formel Ib, in der R¹ Chlor und R² Methylsulfonyl, R⁵ Methyl und R⁶ Methylsulfonyl bedeutet und die Substituente X¹ und Het für jede einzelne Verbindung jeweils einer Zeile der Tabelle A entsprechen.

### Tabelle 41: Verbindungen 41.1 - 41.920

Verbindungen der allgemeinen Formel Ib, in der R¹ Chlor und R² Methylsulfonyl, R⁵ Ethyl und R⁶ Methylsulfonyl bedeutet und die Substituente X¹ und Het für jede einzelne Verbindung jeweils einer Zeile der Tabelle A entsprechen.

### Tabelle 42: Verbindungen 42.1 - 42.920

Verbindungen der allgemeinen Formel Ib, in der R¹ Chlor und R² Methylsulfonyl, R⁵ n-Propyl und R⁶ Methylsulfonyl bedeutet und die Substituente X¹ und Het für jede einzelne Verbindung jeweils einer Zeile der Tabelle A entsprechen.

### Tabelle 43: Verbindungen 43.1 - 43.920

Verbindungen der allgemeinen Formel Ib, in der R¹ Chlor und R² Methylsulfonyl, R⁵ Methyl und R⁶ Ethylsulfonyl bedeutet und die Substituente X¹ und Het für jede einzelne Verbindung jeweils einer Zeile der Tabelle A entsprechen.

### Tabelle 44: Verbindungen 44.1 - 44.920

Verbindungen der allgemeinen Formel Ib, in der R¹ Chlor und R² Methylsulfonyl, R⁵ Ethyl und R⁶ Ethylsulfonyl bedeutet und die Substituente X¹ und Het für jede einzelne Verbindung jeweils einer Zeile der Tabelle A entsprechen.

### Tabelle 45: Verbindungen 45.1 - 45.920

Verbindungen der allgemeinen Formel Ib, in der R¹ Chlor und R² Methylsulfonyl, R⁵ n-Propyl und R⁶ Ethylsulfonyl bedeutet und die Substituente X¹ und Het für jede einzelne Verbindung jeweils einer Zeile der Tabelle A entsprechen.

### Tabelle 46: Verbindungen 46.1 - 46.920

Verbindungen der allgemeinen Formel Ib, in der R¹ Chlor und R² Methylsulfonyl, R⁵ Methyl und R⁶ 4-Methylphenylsulfonyl bedeutet und die Substituente X¹ und Het für jede einzelne Verbindung jeweils einer Zeile der Tabelle A entsprechen.

### Tabelle 47: Verbindungen 47.1 - 47.920

Verbindungen der allgemeinen Formel Ib, in der R¹ Chlor und R² Methylsulfonyl, R⁵ Ethyl und R⁶ 4-Methylphenylsulfonyl bedeutet und die Substituente X¹ und Het für jede einzelne Verbindung jeweils einer Zeile der Tabelle A entsprechen.

### Tabelle 48: Verbindungen 48.1 - 48.920

Verbindungen der allgemeinen Formel Ib, in der R¹ Chlor und R² Methylsulfonyl, R⁵ n-Propyl und R⁶ 4-Methylphenylsulfonyl bedeutet und die Substituente X¹ und Het für jede einzelne Verbindung jeweils einer Zeile der Tabelle A entsprechen.

### Tabelle 49: Verbindungen 49.1 - 49.920

Verbindungen der allgemeinen Formel Ib, in der R¹ Chlor und R² Trifluormethyl, R⁵ Methyl und R⁶ Wasserstoff bedeutet und die Substituente X¹ und Het für jede einzelne Verbindung jeweils einer Zeile der Tabelle A entsprechen.

### Tabelle 50: Verbindungen 50.1 - 50.920

Verbindungen der allgemeinen Formel Ib, in der R¹ Chlor und R² Trifluormethyl, R⁵ Ethyl und R⁶ Wasserstoff bedeutet und die Substituente X¹ und Het für jede einzelne Verbindung jeweils einer Zeile der Tabelle A entsprechen.

### Tabelle 51: Verbindungen 51.1 - 51.920

Verbindungen der allgemeinen Formel Ib, in der R¹ Chlor und R² Trifluormethyl, R⁵ n-Propyl und R⁶ Wasserstoff bedeutet und die Substituente X¹ und Het für jede einzelne Verbindung jeweils einer Zeile der Tabelle A entsprechen.

### Tabelle 52: Verbindungen 52.1 - 52.920

Verbindungen der allgemeinen Formel Ib, in der R¹ Chlor und R² Trifluormethyl, R⁵ Methyl und R⁶ Methyl bedeutet und die Substituente X¹ und Het für jede einzelne Verbindung jeweils einer Zeile der Tabelle A entsprechen.

### Tabelle 53: Verbindungen 53.1 - 53.920

Verbindungen der allgemeinen Formel Ib, in der R¹ Chlor und R² Trifluormethyl, R⁵ Ethyl und R⁶ Methyl bedeutet und die Substituente X¹ und Het für jede einzelne Verbindung jeweils einer Zeile der Tabelle A entsprechen.

### Tabelle 54: Verbindungen 54.1 - 54.920

Verbindungen der allgemeinen Formel Ib, in der R¹ Chlor und R² Trifluormethyl, R⁵ *n*-Propyl und R⁶ Methyl bedeutet und die Substituente X¹ und Het für jede einzelne Verbindung jeweils Zeile der Tabelle A entsprechen.

### Tabelle 55: Verbindungen 55.1 - 55.920

Verbindungen der allgemeinen Formel Ib, in der R¹ Chlor und R² Trifluormethyl, R⁵ Methyl und R⁶ Ethyl bedeutet und die Substituente X¹ und Het für jede einzelne Verbindung jeweils einer Zeile der Tabelle A entsprechen.

### Tabelle 56: Verbindungen 56.1 - 56.920

Verbindungen der allgemeinen Formel Ib, in der R¹ Chlor und R² Trifluormethyl, R⁵ Ethyl und R⁶ Ethyl bedeutet und die Substituente X¹ und Het für jede einzelne Verbindung jeweils einer Zeile der Tabelle A entsprechen.

### Tabelle 57: Verbindungen 57.1 - 57.920

Verbindungen der allgemeinen Formel Ib, in der R¹ Chlor und R² Trifluormethyl, R⁵ n-Propyl und R⁶ Ethyl bedeutet und die Substituente X¹ und Het für jede einzelne Verbindung jeweils einer Zeile der Tabelle A entsprechen.

### Tabelle 58: Verbindungen 58.1 - 58.920

Verbindungen der allgemeinen Formel Ib, in der R¹ Chlor und R² Trifluormethyl, R⁵ Methyl und R⁶ Methylcarbonyl bedeutet und die Substituente X¹ und Het für jede einzelne Verbindung jeweils einer Zeile der Tabelle A entsprechen.

### Tabelle 59: Verbindungen 59.1 - 59.920

Verbindungen der allgemeinen Formel Ib, in der R¹ Chlor und R² Trifluormethyl, R⁵ Ethyl und R⁶ Methylcarbonyl bedeutet und die Substituente X¹ und Het für jede einzelne Verbindung jeweils einer Zeile der Tabelle A entsprechen.

### Tabelle 60: Verbindungen 60.1 - 60.920

Verbindungen der allgemeinen Formel Ib, in der R¹ Chlor und R² Chlor, R⁵ n-Propyl und R⁶ Methylcarbonyl bedeutet und die Substituente X¹ und Het für jede einzelne Verbindung jeweils einer Zeile der Tabelle A entsprechen.

### Tabelle 61: Verbindungen 61.1 - 61.920

Verbindungen der allgemeinen Formel Ib, in der R¹ Chlor und R² Trifluormethyl, R⁵ Methyl und R⁶ Ethylcarbonyl bedeutet und die Substituente X¹ und Het für jede einzelne Verbindung jeweils einer Zeile der Tabelle A entsprechen.

### Tabelle 62: Verbindungen 62.1 - 62.920

Verbindungen der allgemeinen Formel Ib, in der R¹ Chlor und R² Trifluormethyl, R⁵ Ethyl und R⁶ Ethylcarbonyl bedeutet und die Substituente X¹ und Het für jede einzelne Verbindung jeweils einer Zeile der Tabelle A entsprechen.

### Tabelle 63: Verbindungen 63.1 - 63.920

Verbindungen der allgemeinen Formel Ib, in der R¹ Chlor und R² Trifluormethyl, R⁵ *n*-Propyl und R⁶ Ethylcarbonyl bedeutet und die Substituente X¹ und Het für jede einzelne Verbindung jeweils einer Zeile der Tabelle A entsprechen.

### Tabelle 64: Verbindungen 64.1 - 64.920

Verbindungen der allgemeinen Formel Ib, in der R¹ Chlor und R² Trifluormethyl, R⁵ Methyl und R⁶ Methylsulfonyl bedeutet und die Substituente X¹ und Het für jede einzelne Verbindung jeweils einer Zeile der Tabelle A entsprechen.

### Tabelle 65: Verbindungen 65.1 - 65.920

Verbindungen der allgemeinen Formel Ib, in der R¹ Chlor und R² Trifluormethyl, R⁵ Ethyl und R⁶ Methylsulfonyl bedeutet und die Substituente X¹ und Het für jede einzelne Verbindung jeweils einer Zeile der Tabelle A entsprechen.

### Tabelle 66: Verbindungen 66.1 - 66.920

Verbindungen der allgemeinen Formel Ib, in der R¹ Chlor und R² Trifluormethyl, R⁵ n-Propyl und R⁶ Methylsulfonyl bedeutet und die Substituente X¹ und Het für jede einzelne Verbindung jeweils einer Zeile der Tabelle A entsprechen.

### Tabelle 67: Verbindungen 67.1 - 67.920

Verbindungen der allgemeinen Formel Ib, in der R¹ Chlor und R² Trifluormethyl, R⁵ Methyl und R⁶ Ethylsulfonyl bedeutet und die Substituente X¹ und Het für jede einzelne Verbindung jeweils einer Zeile der Tabelle A entsprechen.

### Tabelle 68: Verbindungen 68.1 - 68.920

Verbindungen der allgemeinen Formel Ib, in der R¹ Chlor und R² Trifluormethyl, R⁵ Ethyl und R⁶ Ethylsulfonyl bedeutet und die Substituente X¹ und Het für jede einzelne Verbindung jeweils einer Zeile der Tabelle A entsprechen.

### Tabelle 69: Verbindungen 69.1 - 69.920

Verbindungen der allgemeinen Formel Ib, in der R¹ Chlor und R² Trifluormethyl, R⁵ *n*-Propyl und R⁶ Ethylsulfonyl bedeutet und die Substituente X¹ und Het für jede einzelne Verbindung jeweils einer Zeile der Tabelle A entsprechen.

### Tabelle 70: Verbindungen 70.1 - 70.920

Verbindungen der allgemeinen Formel Ib, in der R¹ Chlor und R² Trifluormethyl, R⁵ Methyl und R⁶ 4-Methylphenylsulfonyl bedeutet und die Substituente X¹ und Het für jede einzelne Verbindung jeweils einer Zeile der Tabelle A entsprechen.

### Tabelle 71: Verbindungen 71.1 - 71.920

Verbindungen der allgemeinen Formel Ib, in der R¹ Chlor und R² Trifluormethyl, R⁵ Ethyl und R⁶ 4-Methylphenylsulfonyl bedeutet und die Substituente X¹ und Het für jede einzelne Verbindung jeweils einer Zeile der Tabelle A entsprechen.

### Tabelle 72: Verbindungen 72.1 - 72.920

Verbindungen der allgemeinen Formel Ib, in der R¹ Chlor und R² Trifluormethyl, R⁵ *n*-Propyl und R⁶ 4-Methylphenylsulfonyl bedeutet und die Substituente X¹ und Het für jede einzelne Verbindung jeweils einer Zeile der Tabelle A entsprechen.

### Tabelle 73: Verbindungen 73.1 - 73.920

Verbindungen der allgemeinen Formel Ib, in der R¹ Methyl und R² Chlor, R⁵ Methyl und R⁶ Wasserstoff bedeutet und die Substituente X¹ und Het für jede einzelne Verbindung jeweils einer Zeile der Tabelle A entsprechen.

### Tabelle 74: Verbindungen 74.1 - 74.920

Verbindungen der allgemeinen Formel Ib, in der R¹ Methyl und R² Chlor, R⁵ Ethyl und R⁶ Wasserstoff bedeutet und die Substituente X¹ und Het für jede einzelne Verbindung jeweils einer Zeile der Tabelle A entsprechen.

### Tabelle 75: Verbindungen 75.1 - 75.920

Verbindungen der allgemeinen Formel Ib, in der R¹ Methyl und R² Chlor, R⁵ n-Propyl und R⁶ Wasserstoff bedeutet und die Substituente X¹ und Het für jede einzelne Verbindung jeweils einer Zeile der Tabelle A entsprechen.

### Tabelle 76: Verbindungen 76.1 - 76.920

Verbindungen der allgemeinen Formel Ib, in der R¹ Methyl und R² Chlor, R⁵ Methyl und R⁶ Methyl bedeutet und die Substituente X¹ und Het für jede einzelne Verbindung jeweils einer Zeile der Tabelle A entsprechen.

### Tabelle 77: Verbindungen 77.1 - 77.920

Verbindungen der allgemeinen Formel Ib, in der R¹ Methyl und R² Chlor, R⁵ Ethyl und R⁶ Methyl bedeutet und die Substituente X¹ und Het für jede einzelne Verbindung jeweils einer Zeile der Tabelle A entsprechen.

### Tabelle 78: Verbindungen 78.1 - 78.920

Verbindungen der allgemeinen Formel Ib, in der R¹ Methyl und R² Chlor, R⁵ *n*-Propyl und R⁶ Methyl bedeutet und die Substituente X¹ und Het für jede einzelne Verbindung jeweils einer Zeile der Tabelle A entsprechen.

### Tabelle 79: Verbindungen 79.1 - 79.920

Verbindungen der allgemeinen Formel Ib, in der R¹ Methyl und R² Chlor, R⁵ Methyl und R⁶ Ethyl bedeutet und die Substituente X¹ und Het für jede einzelne Verbindung jeweils einer Zeile der Tabelle A entsprechen.

### Tabelle 80: Verbindungen 80.1 - 80.920

Verbindungen der allgemeinen Formel Ib, in der R¹ Methyl und R² Chlor, R⁵ Ethyl und R⁶ Ethyl bedeutet und die Substituente X¹ und Het für jede einzelne Verbindung jeweils einer Zeile der Tabelle A entsprechen.

### Tabelle 81: Verbindungen 81.1 - 81.920

Verbindungen der allgemeinen Formel Ib, in der R¹ Methyl und R² Chlor, R⁵ n-Propyl und R⁶ Ethyl bedeutet und die Substituente X¹ und Het für jede einzelne Verbindung jeweils einer Zeile der Tabelle A entsprechen.

### Tabelle 82: Verbindungen 82.1 - 82.920

Verbindungen der allgemeinen Formel Ib, in der R¹ Methyl und R² Chlor, R⁵ Methyl und R⁶ Methylcarbonyl bedeutet und die Substituente X¹ und Het für jede einzelne Verbindung jeweils einer Zeile der Tabelle A entsprechen.

### Tabelle 83: Verbindungen 83.1 - 83.920

Verbindungen der allgemeinen Formel Ib, in der R¹ Methyl und R² Chlor, R⁵ Ethyl und R⁶ Methylcarbonyl bedeutet und die Substituente X¹ und Het für jede einzelne Verbindung jeweils einer Zeile der Tabelle A entsprechen.

### Tabelle 84: Verbindungen 84.1 - 84.920

Verbindungen der allgemeinen Formel Ib, in der R¹ Methyl und R² Chlor, R⁵ *n*-Propyl und R⁶ Methylcarbonyl bedeutet und die Substituente X¹ und Het für jede einzelne Verbindung jeweils einer Zeile der Tabelle A entsprechen.

### Tabelle 85: Verbindungen 85.1 - 85.920

Verbindungen der allgemeinen Formel Ib, in der R¹ Methyl und R² Chlor, R⁵ Methyl und R⁶ Ethylcarbonyl bedeutet und die Substituente X¹ und Het für jede einzelne Verbindung jeweils einer Zeile der Tabelle A entsprechen.

### Tabelle 86: Verbindungen 86.1 - 86.920

Verbindungen der allgemeinen Formel Ib, in der R¹ Methyl und R² Chlor, R⁵ Ethyl und R⁶ Ethylcarbonyl bedeutet und die Substituente X¹ und Het für jede einzelne Verbindung jeweils einer Zeile der Tabelle A entsprechen.

### Tabelle 87: Verbindungen 87.1 - 87.920

Verbindungen der allgemeinen Formel Ib, in der R¹ Methyl und R² Chlor, R⁵ n-Propyl und R⁶ Ethylcarbonyl bedeutet und die Substituente X¹ und Het für jede einzelne Verbindung jeweils einer Zeile der Tabelle A entsprechen.

### Tabelle 88: Verbindungen 88.1 - 88.920

Verbindungen der allgemeinen Formel Ib, in der R¹ Methyl und R² Chlor, R⁵ Methyl und R⁶ Methylsulfonyl bedeutet und die Substituente X¹ und Het für jede einzelne Verbindung jeweils einer Zeile der Tabelle A entsprechen.

### Tabelle 89: Verbindungen 89.1 - 89.920

Verbindungen der allgemeinen Formel Ib, in der R¹ Methyl und R² Chlor, R⁵ Ethyl und R⁶ Methylsulfonyl bedeutet und die Substituente X¹ und Het für jede einzelne Verbindung jeweils einer Zeile der Tabelle A entsprechen.

### Tabelle 90: Verbindungen 90.1 - 90.920

Verbindungen der allgemeinen Formel Ib, in der R¹ Methyl und R² Chlor, R⁵ n-Propyl und R⁶ Methylsulfonyl bedeutet und die Substituente X¹ und Het für jede einzelne Verbindung jeweils einer Zeile der Tabelle A entsprechen.

### Tabelle 91: Verbindungen 91.1 - 91.920

Verbindungen der allgemeinen Formel Ib, in der R¹ Methyl und R² Chlor, R⁵ Methyl und R⁶ Ethylsulfonyl bedeutet und die Substituente X¹ und Het für jede einzelne Verbindung jeweils einer Zeile der Tabelle A entsprechen.

### Tabelle 92: Verbindungen 92.1 - 92.920

Verbindungen der allgemeinen Formel Ib, in der R¹ Methyl und R² Chlor, R⁵ Ethyl und R⁶ Ethylsulfonyl bedeutet und die Substituente X¹ und Het für jede einzelne Verbindung jeweils einer Zeile der Tabelle A entsprechen.

### Tabelle 93: Verbindungen 93.1 - 93.920

Verbindungen der allgemeinen Formel Ib, in der R¹ Methyl und R² Chlor, R⁵ *n*-Propyl und R⁶ Ethylsulfonyl bedeutet und die Substituente X¹ und Het für jede einzelne Verbindung jeweils einer Zeile der Tabelle A entsprechen.

### Tabelle 94: Verbindungen 94.1 - 94.920

Verbindungen der allgemeinen Formel Ib, in der R¹ Methyl und R² Chlor, R⁵ Methyl und R⁶ 4-Methylphenylsulfonyl bedeutet und die Substituente X¹ und Het für jede einzelne Verbindung jeweils einer Zeile der Tabelle A entsprechen.

### Tabelle 95: Verbindungen 95.1 - 95.920

Verbindungen der allgemeinen Formel Ib, in der R¹ Methyl und R² Chlor, R⁵ Ethyl und R⁶ 4-Methylphenylsulfonyl bedeutet und die Substituente X¹ und Het für jede einzelne Verbindung jeweils einer Zeile der Tabelle A entsprechen.

### Tabelle 96: Verbindungen 96.1 - 96.920

Verbindungen der allgemeinen Formel Ib, in der R¹ Methyl und R² Chlor, R⁵ n-Propyl und R⁶ 4-Methylphenylsulfonyl bedeutet und die Substituente X¹ und Het für jede einzelne Verbindung jeweils einer Zeile der Tabelle A entsprechen.

### Tabelle 97: Verbindungen 97.1 - 97.920

Verbindungen der allgemeinen Formel Ib, in der R¹ Methyl und R² Methylsulfonyl, R⁵ Methyl und R⁶ Wasserstoff bedeutet und die Substituente X¹ und Het für jede einzelne Verbindung jeweils einer Zeile der Tabelle A entsprechen.

### Tabelle 98: Verbindungen 98.1 - 98.920

Verbindungen der allgemeinen Formel Ib, in der R¹ Methyl und R² Methylsulfonyl, R⁵ Ethyl und R⁶ Wasserstoff bedeutet und die Substituente X¹ und Het für jede einzelne Verbindung jeweils einer Zeile der Tabelle A entsprechen.

### Tabelle 99: Verbindungen 99.1 - 99.920

Verbindungen der allgemeinen Formel Ib, in der R¹ Methyl und R² Methylsulfonyl, R⁵ *n*-Propyl und R⁶ Wasserstoff bedeutet und die Substituente X¹ und Het für jede einzelne Verbindung jeweils einer Zeile der Tabelle A entsprechen.

### Tabelle 100: Verbindungen 100.1 - 100.920

Verbindungen der allgemeinen Formel Ib, in der R¹ Methyl und R² Methylsulfonyl, R⁵ Methyl und R⁶ Methyl bedeutet und die Substituente X¹ und Het für jede einzelne Verbindung jeweils einer Zeile der Tabelle A entsprechen.

### Tabelle 101: Verbindungen 101.1 - 101.920

Verbindungen der allgemeinen Formel Ib, in der R¹ Methyl und R² Methylsulfonyl, R⁵ Ethyl und R⁶ Methyl bedeutet und die Substituente X¹ und Het für jede einzelne Verbindung jeweils einer Zeile der Tabelle A entsprechen.

### Tabelle 102: Verbindungen 102.1 - 102.920

Verbindungen der allgemeinen Formel Ib, in der R¹ Methyl und R² Methylsulfonyl, R⁵ n-Propyl und R⁶ Methyl bedeutet und die Substituente X¹ und Het für jede einzelne Verbindung jeweils einer Zeile der Tabelle A entsprechen.

### Tabelle 103: Verbindungen 103.1 - 103.920

Verbindungen der allgemeinen Formel Ib, in der R¹ Methyl und R² Methylsulfonyl, R⁵ Methyl und R⁶ Ethyl bedeutet und die Substituente X¹ und Het für jede einzelne Verbindung jeweils einer Zeile der Tabelle A entsprechen.

### Tabelle 104: Verbindungen 104.1 - 104.920

Verbindungen der allgemeinen Formel Ib, in der R¹ Methyl und R² Methylsulfonyl, R⁵ Ethyl und R⁶ Ethyl bedeutet und die Substituente X¹ und Het für jede einzelne Verbindung jeweils einer Zeile der Tabelle A entsprechen.

### Tabelle 105: Verbindungen 105.1 - 105.920

Verbindungen der allgemeinen Formel Ib, in der R¹ Methyl und R² Methylsulfonyl, R⁵ n-Propyl und R⁶ Ethyl bedeutet und die Substituente X¹ und Het für jede einzelne Verbindung jeweils einer Zeile der Tabelle A entsprechen.

### Tabelle 106: Verbindungen 106.1 - 106. 920

Verbindungen der allgemeinen Formel Ib, in der R¹ Methyl und R² Methylsulfonyl, R⁵ Methyl und R⁶ Methylcarbonyl bedeutet und die Substituente X¹ und Het für jede einzelne Verbindung jeweils einer Zeile der Tabelle A entsprechen.

### Tabelle 107: Verbindungen 107.1 - 107.920

Verbindungen der allgemeinen Formel Ib, in der R¹ Methyl und R² Methylsulfonyl, R⁵ Ethyl und R⁶ Methylcarbonyl bedeutet und die Substituente X¹ und Het für jede einzelne Verbindung jeweils einer Zeile der Tabelle A entsprechen.

### Tabelle 108: Verbindungen 108.1 - 108.920

Verbindungen der allgemeinen Formel Ib, in der R¹ Methyl und R² Methylsulfonyl, R⁵ *n*-Propyl und R⁶ Methylcarbonyl bedeutet und die Substituente X¹ und Het für jede einzelne Verbindung jeweils einer Zeile der Tabelle A entsprechen.

### Tabelle 109: Verbindungen 109.1 - 109.920

Verbindungen der allgemeinen Formel Ib, in der R¹ Methyl und R² Methylsulfonyl, R⁵ Methyl und R⁶ Ethylcarbonyl bedeutet und die Substituente X¹ und Het für jede einzelne Verbindung jeweils einer Zeile der Tabelle A entsprechen.

### Tabelle 110: Verbindungen 110.1 - 110.920

Verbindungen der allgemeinen Formel Ib, in der R¹ Methyl und R² Methylsulfonyl, R⁵ Ethyl und R⁶ Ethylcarbonyl bedeutet und die Substituente X¹ und Het für jede einzelne Verbindung jeweils einer Zeile der Tabelle A entsprechen.

### Tabelle 111: Verbindungen 111.1 - 111.920

Verbindungen der allgemeinen Formel Ib, in der R¹ Methyl und R² Methylsulfonyl, R⁵ n-Propyl und R⁶ Ethylcarbonyl bedeutet und die Substituente X¹ und Het für jede einzelne Verbindung jeweils einer Zeile der Tabelle A entsprechen.

### Tabelle 112: Verbindungen 112.1 - 112.920

Verbindungen der allgemeinen Formel Ib, in der R¹ Methyl und R² Methylsulfonyl, R⁵ Methyl und R⁶ Methylsulfonyl bedeutet und die Substituente X¹ und Het für jede einzelne Verbindung jeweils einer Zeile der Tabelle A entsprechen.

### Tabelle 113: Verbindungen 113.1 - 113.920

Verbindungen der allgemeinen Formel Ib, in der R¹ Methyl und R² Methylsulfonyl, R⁵ Ethyl und R⁶ Methylsulfonyl bedeutet und die Substituente X¹ und Het für jede einzelne Verbindung jeweils einer Zeile der Tabelle A entsprechen.

### Tabelle 114: Verbindungen 114.1 - 114.920

Verbindungen der allgemeinen Formel Ib, in der R¹ Methyl und R² Methylsulfonyl, R⁵ n-Propyl und R⁶ Methylsulfonyl bedeutet und die Substituente X¹ und Het für jede einzelne Verbindung jeweils einer Zeile der Tabelle A entsprechen.

### Tabelle 115: Verbindungen 115.1 - 115.920

Verbindungen der allgemeinen Formel Ib, in der R¹ Methyl und R² Methylsulfonyl, R⁵ Methyl und R⁶ Ethylsulfonyl bedeutet und die Substituente X¹ und Het für jede einzelne Verbindung jeweils einer Zeile der Tabelle A entsprechen.

### Tabelle 116: Verbindungen 116.1 - 116.920

Verbindungen der allgemeinen Formel Ib, in der R¹ Methyl und R² Methylsulfonyl, R⁵ Ethyl und R⁶ Ethylsulfonyl bedeutet und die Substituente X¹ und Het für jede einzelne Verbindung jeweils einer Zeile der Tabelle A entsprechen.

### Tabelle 117: Verbindungen 117.1 - 117.920

Verbindungen der allgemeinen Formel Ib, in der R¹ Methyl und R² Methylsulfonyl, R⁵ n-Propyl und R⁶ Ethylsulfonyl bedeutet und die Substituente X¹ und Het für jede einzelne Verbindung jeweils einer Zeile der Tabelle A entsprechen.

### Tabelle 118: Verbindungen 118.1 - 118.920

Verbindungen der allgemeinen Formel Ib, in der R¹ Methyl und R² Methylsulfonyl, R⁵ Methyl und R⁶ 4-Methylphenylsulfonyl bedeutet und die Substituente X¹ und Het für jede einzelne Verbindung jeweils einer Zeile der Tabelle A entsprechen.

### Tabelle 119: Verbindungen 119.1 - 119.920

Verbindungen der allgemeinen Formel Ib, in der R¹ Methyl und R² Methylsulfonyl, R⁵ Ethyl und R⁶ 4-Methylphenylsulfonyl bedeutet und die Substituente X¹ und Het für jede einzelne Verbindung jeweils einer Zeile der Tabelle A entsprechen.

### Tabelle 120: Verbindungen 120.1 - 120.920

Verbindungen der allgemeinen Formel Ib, in der R¹ Methyl und R² Methylsulfonyl, R⁵ *n*-Propyl und R⁶ 4-Methylphenylsulfonyl bedeutet und die Substituente X¹ und Het für jede einzelne Verbindung jeweils einer Zeile der Tabelle A entsprechen.

### Tabelle 121: Verbindungen 121.1 - 121.920

Verbindungen der allgemeinen Formel Ib, in der R¹ Methyl und R² Trifluormethyl, R⁵ Methyl und R⁶ Wasserstoff bedeutet und die Substituente X¹ und Het für jede einzelne Verbindung jeweils einer Zeile der Tabelle A entsprechen.

### Tabelle 122: Verbindungen 122.1 - 122.920

Verbindungen der allgemeinen Formel Ib, in der R¹ Methyl und R² Trifluormethyl, R⁵ Ethyl und R⁶ Wasserstoff bedeutet und die Substituente X¹ und Het für jede einzelne Verbindung jeweils einer Zeile der Tabelle A entsprechen.

### Tabelle 123: Verbindungen 123.1 - 123.920

Verbindungen der allgemeinen Formel Ib, in der R¹ Methyl und R² Trifluormethyl, R⁵ n-Propyl und R⁶ Wasserstoff bedeutet und die Substituente X¹ und Het für jede einzelne Verbindung jeweils einer Zeile der Tabelle A entsprechen.

### Tabelle 124: Verbindungen 124.1 - 124.920

Verbindungen der allgemeinen Formel Ib, in der R¹ Methyl und R² Trifluormethyl, R⁵ Methyl und R⁶ Methyl bedeutet und die Substituente X¹ und Het für jede einzelne Verbindung jeweils einer Zeile der Tabelle A entsprechen.

### Tabelle 125: Verbindungen 12.1 - 125.920

Verbindungen der allgemeinen Formel Ib, in der R¹ Methyl und R² Trifluormethyl, R⁵ Ethyl und R⁶ Methyl bedeutet und die Substituente X¹ und Het für jede einzelne Verbindung jeweils einer Zeile der Tabelle A entsprechen.

### Tabelle 126: Verbindungen 126.1 - 126.920

Verbindungen der allgemeinen Formel Ib, in der R¹ Methyl und R² Trifluormethyl, R⁵ *n*-Propyl und R⁶ Methyl bedeutet und die Substituente X¹ und Het für jede einzelne Verbindung jeweils einer Zeile der Tabelle A entsprechen.

### Tabelle 127: Verbindungen 127.1 - 127.920

Verbindungen der allgemeinen Formel Ib, in der R¹ Methyl und R² Trifluormethyl, R⁵ Methyl und R⁶ Ethyl bedeutet und die Substituente X¹ und Het für jede einzelne Verbindung jeweils einer Zeile der Tabelle A entsprechen.

### Tabelle 128: Verbindungen 128.1 - 128.920

Verbindungen der allgemeinen Formel Ib, in der R¹ Methyl und R² Trifluormethyl, R⁵ Ethyl und R⁶ Ethyl bedeutet und die Substituente X¹ und Het für jede einzelne Verbindung jeweils einer Zeile der Tabelle A entsprechen.

### Tabelle 129: Verbindungen 129.1 - 129.920

Verbindungen der allgemeinen Formel Ib, in der R¹ Methyl und R² Trifluormethyl, R⁵ n-Propyl und R⁶ Ethyl bedeutet und die Substituente X¹ und Het für jede einzelne Verbindung jeweils einer Zeile der Tabelle A entsprechen.

### Tabelle 130: Verbindungen 130.1 - 130.920

Verbindungen der allgemeinen Formel Ib, in der R¹ Methyl und R² Trifluormethyl, R⁵ Methyl und R⁶ Methylcarbonyl bedeutet und die Substituente X¹ und Het für jede einzelne Verbindung jeweils einer Zeile der Tabelle A entsprechen.

### Tabelle 131: Verbindungen 131.1 - 131.920

Verbindungen der allgemeinen Formel Ib, in der R¹ Methyl und R² Trifluormethyl, R⁵ Ethyl und R⁶ Methylcarbonyl bedeutet und die Substituente X¹ und Het für jede einzelne Verbindung jeweils einer Zeile der Tabelle A entsprechen.

### Tabelle 132: Verbindungen 132.1 - 132. 920

Verbindungen der allgemeinen Formel Ib, in der R¹ Methyl und R² Trifluormethyl, R⁵ *n*-Propyl und R⁶ Methylcarbonyl bedeutet und die Substituente X¹ und Het für jede einzelne Verbindung jeweils einer Zeile der Tabelle A entsprechen.

### Tabelle 133: Verbindungen 133.1 - 133.920

Verbindungen der allgemeinen Formel Ib, in der R¹ Methyl und R² Trifluormethyl, R⁵ Methyl und R⁶ Ethylcarbonyl bedeutet und die Substituente X¹ und Het für jede einzelne Verbindung jeweils einer Zeile der Tabelle A entsprechen.

### Tabelle 134: Verbindungen 134.1 - 134.920

Verbindungen der allgemeinen Formel Ib, in der R¹ Methyl und R² Trifluormethyl, R⁵ Ethyl und R⁶ Ethylcarbonyl bedeutet und die Substituente X¹ und Het für jede einzelne Verbindung jeweils einer Zeile der Tabelle A entsprechen.

### Tabelle 135: Verbindungen 135.1 - 135.920

Verbindungen der allgemeinen Formel Ib, in der R¹ Methyl und R² Trifluormethyl, R⁵ *n*-Propyl und R⁶ Ethylcarbonyl bedeutet und die Substituente X¹ und Het für jede einzelne Verbindung jeweils einer Zeile der Tabelle A entsprechen.

### Tabelle 136: Verbindungen 136.1 - 136.920

Verbindungen der allgemeinen Formel Ib, in der R¹ Methyl und R² Trifluormethyl, R⁵ Methyl und R⁶ Methylsulfonyl bedeutet und die Substituente X¹ und Het für jede einzelne Verbindung jeweils einer Zeile der Tabelle A entsprechen.

### Tabelle 137: Verbindungen 137.1 - 137.920

Verbindungen der allgemeinen Formel Ib, in der R¹ Methyl und R² Trifluormethyl, R⁵ Ethyl und R⁶ Methylsulfonyl bedeutet und die Substituente X¹ und Het für jede einzelne Verbindung jeweils einer Zeile der Tabelle A entsprechen.

### Tabelle 138: Verbindungen 138.1 - 138.920

Verbindungen der allgemeinen Formel Ib, in der R¹ Methyl und R² Trifluormethyl, R⁵ n-Propyl und R⁶ Methylsulfonyl bedeutet und die Substituente X¹ und Het für jede einzelne Verbindung jeweils einer Zeile der Tabelle A entsprechen.

### Tabelle 139: Verbindungen 139.1 - 139.920

Verbindungen der allgemeinen Formel Ib, in der R¹ Methyl und R² Trifluormethyl, R⁵ Methyl und R⁶ Ethylsulfonyl bedeutet und die Substituente X¹ und Het für jede einzelne Verbindung jeweils einer Zeile der Tabelle A entsprechen.

### Tabelle 140: Verbindungen 140.1 - 140.920

Verbindungen der allgemeinen Formel Ib, in der R¹ Methyl und R² Trifluormethyl, R⁵ Ethyl und R⁶ Ethylsulfonyl bedeutet und die Substituente X¹ und Het für jede einzelne Verbindung jeweils einer Zeile der Tabelle A entsprechen.

### Tabelle 141: Verbindungen 141.1 - 141.920

Verbindungen der allgemeinen Formel Ib, in der R¹ Methyl und R² Trifluormethyl, R⁵ n-Propyl und R⁶ Ethylsulfonyl bedeutet und die Substituente X¹ und Het für jede einzelne Verbindung jeweils einer Zeile der Tabelle A entsprechen.

### Tabelle 142: Verbindungen 142.1 - 142.920

Verbindungen der allgemeinen Formel Ib, in der R¹ Methyl und R² Trifluormethyl, R⁵ Methyl und R⁶ 4-Methylphenylsulfonyl bedeutet und die Substituente X¹ und Het für jede einzelne Verbindung jeweils einer Zeile der Tabelle A entsprechen.

### Tabelle 143: Verbindungen 143.1 - 143.920

Verbindungen der allgemeinen Formel Ib, in der R¹ Methyl und R² Trifluormethyl, R⁵ Ethyl und R⁶ 4-Methylphenylsulfonyl bedeutet und die Substituente X¹ und Het für jede einzelne Verbindung jeweils einer Zeile der Tabelle A entsprechen.

### Tabelle 144: Verbindungen 144.1 - 144.920

Verbindungen der allgemeinen Formel Ib, in der R¹ Methyl und R² Trifluormethyl, R⁵ n-Propyl und R⁶ 4-Methylphenylsulfonyl bedeutet und die Substituente X¹ und Het für jede einzelne Verbindung jeweils einer Zeile der Tabelle A entsprechen.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z.B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die substituierten 4-Benzoyl-pyrazole als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90% bis 100 %, vorzugsweise 95 % bis 100 % (nach NMR-Spektrum) eingesetzt.

Die erfindungsgemäßen Verbindungen I können beispielsweise wie folgt formuliert werden:
- I: 20 Gewichtsteile der Verbindung Nr. 26.39 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
- II: 20 Gewichtsteile der Verbindung Nr. 26.39 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
- III: 20 Gewichtsteile des Wirkstoffs Nr. 26.39 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
- IV: 20 Gewichtsteile des Wirkstoffs Nr. 26.39 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20000 Gewichtsteilen Wasser enthält man eine Spritzbrühe, die 0,1 Gew.-% des Wirkstoffs enthält.
- V: 3 Gewichtsteile des Wirkstoffs Nr. 26.39 werden mit 97 Gewichtsteilen feinteiligem Kaolin vermischt. Man erhält auf diese Weise ein Stäubemittel, das 3 Gew.-% des Wirkstoffs enthält.

- VI: 20 Gewichtsteile des Wirkstoffs Nr. 26.39 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
- VII: 1 Gewichtsteil der Verbindung Nr. 26.39 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
- 5 VIII: 1 Gewichtsteil der Verbindung Nr. 26.39 wird in einer Mischung gelöst, die aus 80 Gewichtsteilen Cyclohexanon und 20 Gewichtsteilen Wettol® EM 31 (nicht ionischer Emulgator auf der Basis von ethoxyliertem Ricinusöl). Man erhält ein stabiles Emulsionskonzentrat.

Zur Verbreiterung des Wirkungsspektrums und zur Erzielung synergistischer Effekte können die substituierten 4-Benzoylpyrazole mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF3-phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.)

Nachfolgend werden die Synthesen einiger Edukte und Produkte beschrieben.

### 2,4-Dichlor-3-((2-pyridyl)-(hydroxymethyl)-phenyl)-(1-ethyl-5-hydroxy-1H-pyrazol-4-yl)-methanon

### Stufe a: 2,4-Dichlor-3-((2-pyridyl)-(hydroxymethyl))-benzoesäure-tert-butylester

4.0 g (39.6 mmol) Diisopropylamin werden in 120 ml Tetrahydrofuran bei -20°C für 40 min mit 25,0 ml (40,0 mmol) 1,6 M n-Butyllithium-Lösung in Hexan gerührt. Bei -75°C wird eine Lösung von 10,0 g (40,5 mmol) 2,4-Dichlorbenzoesäure-*tert*-butylester in 30 ml Tetrahydrofuran zugetropft und 1,5 h gerührt. Man tropft eine Lösung aus 4,3 g (40,5 mmol) 2-Formylpyridin in 20 ml Tetrahydropyran zu und rührt 2.5 h bei Raumtemp. Die Mischung wird in 500 ml gesättigte, wäßrige Ammoniumchlorid-Lösung gegeben und mit Essigsäureethylester extrahiert. Die vereinigten Extrakte werden mit Wasser gewaschen, über Natriumsulfat getrocknet, filtriert und i. Vak. vom Lösungsmittel befreit. Das Rohprodukt wird durch Chromatographie an Kieselgel mit Cyclohexan/Essigsäureethylester gereinigt. Ausbeute: 7,5 g; ¹H-NMR, δ [ppm], (DMSO-d₆) : 1,5 (s), 6,5 (m), 7,2 (m) ; 7,5 (m), 7,9 (m), 8,4 (d)

### Stufe b: 2,4-Dichlor-3-((2-pyridyl)-(hydroxymethyl))-benzoesäure

3,5 g (9,9 mmol) 2,4-Dichlor-3-((2-pyridyl)-(hydroxymethyl))-benzoesäure-*tert*-butylester werden in 120 ml Toluol und 60 ml Wasser mit 1,9 g p-Toluolsulfonsäure 9 h am Rückfluß erhitzt. Nach Abkühlen trennt man die organische Phase ab und versetzt die wäßrige Phase mit einer Lösung aus 23,8 g Natriumdihydrogenphosphat in 280 ml Wasser und extrahiert mit Essigsäureethylester. Die vereinigten organischen Phasen werden über Natriumsulfat getrocknet, filtriert und i. Vak. vom Lösungsmittel befreit. Ausbeute: 1,9 g; ¹H-NMR, δ [ppm], (DMSO-d₆) : 6,5 (m), 7,2 (m), 7,5 (d), 7,6 (d), 7,95 (m), 8,4 (d), 13,5 (breites s)

### Stufe c: (2,4-Dichlor-3-((2-pyridyl)-(hydroxymethyl)-phenyl)-(1-ethyl-5-hydroxy-1H-pyrazol-4-yl)-methanon (Tabelle 2, Beispiel 319; Verb. Nr. 2.319)

1,7 g (5,7 mmol) 2,4-Dichlor-3-((2-pyridyl)-(hydroxymethyl))-benzoesäure, 0,6 g (5.7 mmol) 1-Ethyl-5-hydroxy-1*H*-pyrazol und 1,2 g (5,7 mmol) *N,N*-Dicyclohexylcarbodiimid werden in 25 ml Acetonitril 3 d bei Raumtemp. gerührt. Das Reaktionsgemisch wird in 50 ml 2 %iger, wäßriger Natriumhydrogencarbonat-Lösung aufgenommen, filtriert und mit Essigsäureethylester extrahiert. Die vereinigten, organischen Phasen werden über Natriumsulfat getrocknet, filtriert und i. Vak. vom Lösungsmittel befreit. Das Zwischenprodukt wird in 10 ml 1,4-Dioxan gelöst, mit 1,0 g (7,1 mmol) Kaliumcarbonat versetzt und 8 h am Rückfluß erhitzt. Nach dem Abkühlen wird das Reaktionsgemisch in 80 ml Wasser aufgenommen, mit Methyl-*tert*-butylether extrahiert. Das Produkt wird durch Ansäuern mit verdünnter, wäßriger Salzsäure aus der wäßrigen Phase gefällt. ¹H-NMR, δ [ppm], (DMSO-d₆): 1,3 (t), 4,0 (q), 6,6 (s), 7,2 (s), 7,3 (m), 7,4 (d), 7,5 (d), 7,8 (d), 7,9 (m), 8,0 (d)

### 2,4-Dichlor-3-((2-furyl)-(hydroxymethyl)-phenyl)-(1-ethyl-5-hydroxy-1H-pyrazol-4-yl)-methanon

Diese Verbindung wurde analog zu den oben angegebenen Arbeitsvorschriften dargestellt. ¹H-NMR, δ [ppm], (DMSO-d₆) : 1,3 (t), 3,9 (q), 6,3 (m), 6,4 (breites s); 6,4 (m), 6,5 (m), 7,3 (s), 7,4 (d), 7,6 (m)

### Stufe a: 2,4-Dichlor-3-((2-furyl)-(hydroxymethyl))-benzoesäure-tert-butylester

4,0 g (39,6 mmol) Diisopropylamin in 80 ml Tetrahydrofuran werden bei -20°C für 15 min mit 19 ml (30,4 mmol) 1,6 M *n*-ButyllithiumLösung in Hexan behandelt. Nach Abkühlen auf -75°C tropft man eine Lösung aus 7,5 g (30,4 mmol) 2,4-Dichlorbenzoesäure-*tert*-butylester in 20 ml Tetrahydrofuran zu und rührt für 12 h bei Raumtemp. Man versetzt das Reaktionsgemisch mit einer Lösung aus 2,9 g (30,2 mmol) 2-Formylfuran in 15 ml Tetrahydrofuran und rührt weitere 12 h bei Raumtemp. Die Mischung wird in 300 ml gesättigter, wäßriger Natriumchlorid-Lösung aufgenommen und mit Essigsäureethylester extrahiert. Die vereinigten, organischen Phasen werden über Natriumsulfat getrocknet, filtriert und i. Vak. vom Lösungsmittel befreit. Ausbeute: 9,2 g; ¹H-NMR, δ [ppm], (CDCl₃) : 1,6 (s), 3,6 (d), 6,2 (m), 6,3 (m), 7,4 (m), 7,7 (d), 7,4 (m), 7,5 (d)

### Stufe b: 2,4-Dichlor-3-((2-furyl)-(methoxymethyl))-benzoesäure-tert-butylester

3,0 g (8,8 mmol) 2,4-Dichlor-3-((2-furyl)-(hydroxymethyl))-benzoesäure-tert-butylester werden in 40 ml Tetrahydrofuran bei Raumtemp. 1 h mit 0,4 g (16,6 mmol) Natriumhydrid gerührt. Man tropft 6,3 g (43,8 mmol) Iodmethan zu und rührt weitere 3 h bei Raumtemp. Das Reaktionsgemisch wird in 100 ml gesättigter, wäßriger Natriumchlorid-Lösung aufgenommen, mit Methyl-*tert*-butylether extrahiert, über Natriumsulfat getrocknet, filtriert und i. Vak. vom Lösungsmittel befreit. Das Rohprodukt wird durch Chromatographie an Kieselgel mit Cyclohexan/Essigsäureethylester gereinigt. Ausbeute: 2,7 g; ¹H-NMR, δ [ppm], (CDCl₃) : 1,6 (s), 3,5 (s), 6,2 (m), 6,3 (m), 7,4 (m), 7,5 (d)

### Stufe c: 2,4-Dichlor-3-((2-furyl)-(methoxymethyl))-benzoesäure

2,3 g (6,4 mmol) 2,4-Dichlor-3-((2-furyl)-(methoxymethyl))-benzoesäure-tert-butylester werden in 50 ml Methanol und 15 ml Wasser mit 0,7 g (16,1 mmol) Natriumhydroxid 4 h am Rückfluß erhitzt. Man gibt 5,0 ml 10 %ige, wäßrige Natriumhydroxid-Lösung zu und erhitzt weitere 3 h. Nachdem das Reaktionsgemisch i. Vak. eingeengt worden ist, gibt man 50 ml Wasser zu und extrahiert das Reaktionsgemisch mit Dichlormethan. Die wäßrige Phase wird mit 10 %iger, wäßriger Salzsäure angesäuert und das Produkt mit Essigsäureethylester extrahiert. Die vereinigten organischen Phasen werden über Natriumsulfat getrocknet, filtriert und i. Vak. vom Lösungsmittel befreit. Ausbeute 1,8 g

### Stufe d: (2,4-Dichlor-3-((2-furyl)-(methoxymethyl)-phenyl)-(1-ethyl-5-hydroxy-1H-pyrazol-4-yl)-methanon (Tabelle 2, Beispiel 362; Verb. Nr. 2.362)

1,4 g (4,7 mmol) 2,4-Dichlor-3-((2-furyl)-(methoxymethyl))-benzoesäure, 0,5 g (4,7 mmol) 1-Ethyl-5-hydroxy-1*H*-pyrazol und 1,0 g (4,7 mmol) *N,N*-Dicyclohexylcarbodiimid werden in 10 ml Acetonitril 24 h bei Raumtemp. gerührt. Das Reaktionsgemisch wird in eine 2 %ige, wäßrige Natriumhydrogencarbonat-Lösung eingerührt, mit Essigsäureethylester extrahiert, über Natriumsulfat getrocknet, filtriert und i. Vak. vom Lösungsmittel befreit. Das Zwischenprodukt wird durch Chormatographie an Kieselgel mit Essigsäureethylester/Cyclohexan gereinigt (Ausbeute: 0,5 g), in 3 ml 1,4-Dioxan gelöst, mit 0,2 g (1,2 mmol) Kaliumcarbonat versetzt und 4 h am Rückfluß erhitzt. Das Reaktionsgemisch wird i. Vak. eingeengt, in 40 ml Wasser aufgenommen und mit Methylenchlorid extrahiert. Das Produkt wird durch Ansäuern der wäßrigen Phase mit 10 %iger, wäßriger Salzsäure gefällt. Ausbeute: 190 mg; Fp. 92 bis 93°C.

### (2,4-Dichlor-3-((3-furyl)-(hydroxymethyl)-phenyl)-(1-ethyl-5-hydroxy-1H-pyrazol-4-yl)-methanon (Tabelle 2, Beispiel 250; Verb. Nr. 2.250)

Diese verbindung wird analog zu den oben angegebenen Arbeitsvorschriften dargestellt. ¹H-NMR, δ [ppm], (DMSO-d₆): 1,3 (t), 3,9 (q), 6,1 (breites s), 6,4 (s), 6,5 (s), 7,3 (breites s.), 7,4 (d), 7,5 (s), 7,5 (d), 7,6 (s)

### (2,4-Dichlor-3-((3-furyl)-(methoxymethyl)-phenyl)-(1-ethyl-5-hydroxy-1H-pyrazol-4-yl)-methanon (Tabelle 2, Beispiel 365; Verb. Nr. 2.365)

Diese Verbindung wird analog zu den oben angegebenen Arbeitsvorschriften dargestellt. ¹H-NMR, δ [ppm], (DMSO-d₆): 1,3 (t), 3,3 (s), 3,9 (q), 6,1 (s), 6,4 (s), 7,3 (breites s), 7,4 (d), 7,5 (s), 7,6 (d), 7,6 (s)

### 2,4-Dichlor-3-(3-(5H-furanon)methyl)phenyl)-(1-ethyl-5-hydroxy-1H-pyrazol-4-yl)methanon

### Stufe a: 2,4-Dichlor-3-(3-(5H-furanon)methyl)benzoesäure

Die Lösung von 13 g (0,038 mol) 2,4-Dichlor-3-(3-furyl)hydroxy-methylbenzoesäure-tert-butylester (analog Bsp. 2.362 Stufe a) und 1,8 g p-Toluolsulfonsäure in 370 ml Toluol werden 6 h refluxiert. Anschließend wird auf 100 ml 10 % Natronlauge gegeben und mit Essigester extrahiert. Die wäßrige Phase wird mit Salzsäure angesäuert und mit Essigester mehrmals extrahiert. Die vereinigten organischen Phasen werden mit Wasser gewaschen, getrocknet und eingeengt. Es verbleiben 4,8 g (45 %) der Titelverbindung Fp. 196°C.

### Stufe b: 2,4-Dichlor-3-(3-(5H-furanon)methyl)phenyl)-(1-ethyl-5-hydroxy-1H-pyrazol-4-yl)methanon

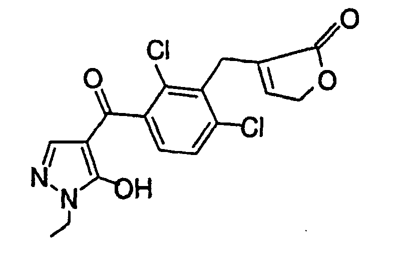

1,1 g (0,0035 mol) 2,4-Dichlor-3-(3-(5H-furanon)methyl)benzoesäure, 0,4 g (0,0035 mol) 1-Ethyl-5-hydroxy-1H-pyrazol und 0,72 g (0,0035 mol) Dicyclohexylcarbodiimid werden 12 h bei Raumtemperatur in 15 ml Acetonitril gerührt. Das Reaktionsgemisch wird auf 100 ml 2 %iger wäßriger Natriumhydrogencarbonat-Lösung gegeben und mit Essigester extrahiert. Die organische Phase wird getrocknet und eingeengt.

1 g des so erhaltenen Rückstandes und 0,5 g (0,0034 mol) Kaliumcarbonat in 5 ml Dioxan werden 5 h refluxiert. Nach dem Abkühlen wird mit 60 ml Wasser verdünnt und nacheinander mit Methylenchlorid und Methyl-tert-butylether extrahiert. Die wäßrige Phase wird abgetrennt, mit HCl angesäuert und der Niederschlag abgesaugt (23 %; Fp. 90-93°C).

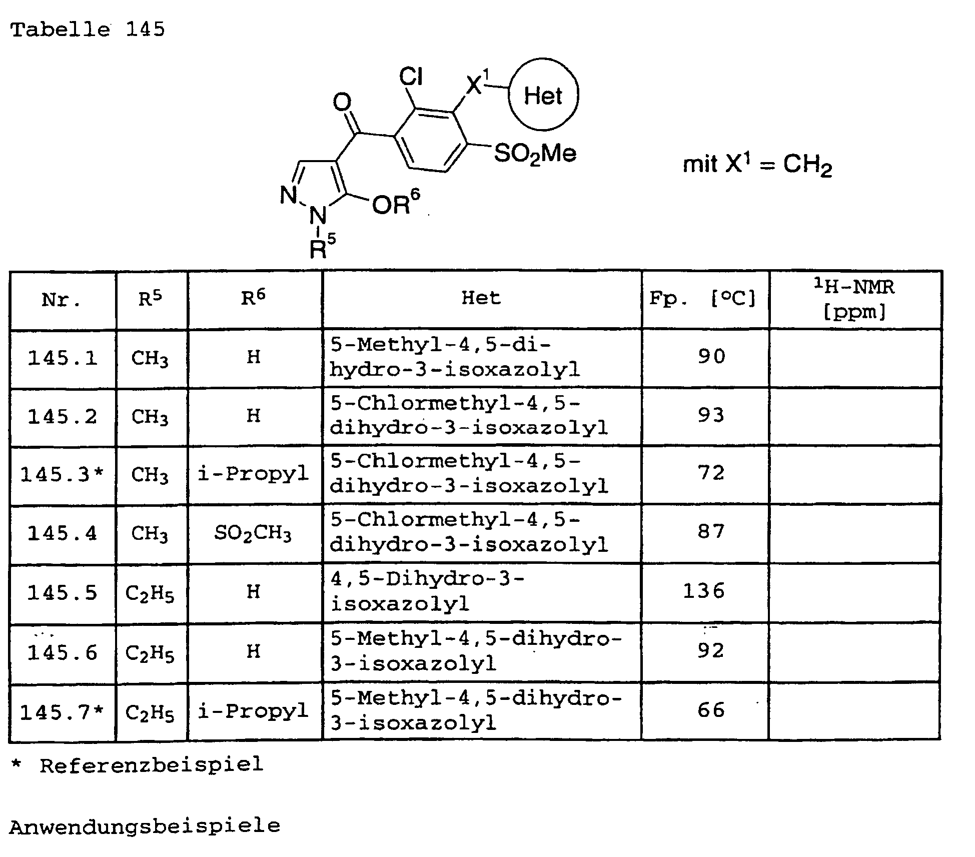

Die herbizide Wirkung der substituierten 4-Benzoyl-pyrazole der Formel I ließ sich durch Gewächshausversuche zeigen:

Als Kulturgefäße dienten Plastiktöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,5 bzw. 0,25 kg/ha a.S.

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Chenopodium album | Weißer Gänsefuß | lambsquarters(goosefoot) |
| Echinochloa crus-galli | Hühnerhirse | barnyardgrass |
| Setaria faberii | Borstenhirse | giant foxtail |
| Setaria viridis | Grüne Borstenhirse | green foxtail |
| Zea mays | Mais | corn |

Selektive herbizide Aktivität bei Nachauflauf anwendung im Gewächshaus

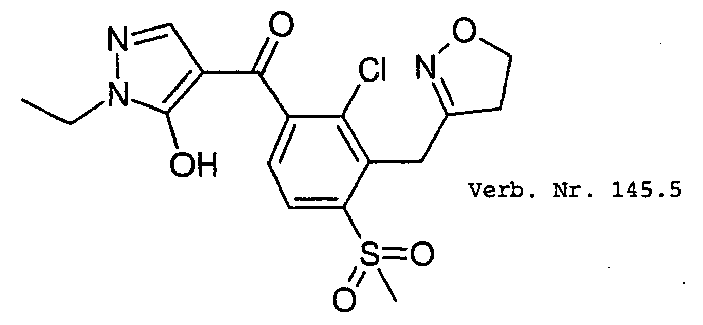

Oben genannte Unkräuter werden von Verbindung Nr. 145.5 im Nachauflauf bei Aufwandmengen von 0,5 bzw. 0,25 kg/ha a.S. sehr gut bekämpft.

## Patentansprüche

1. 4-Benzoyl-pyrazole der Formel I 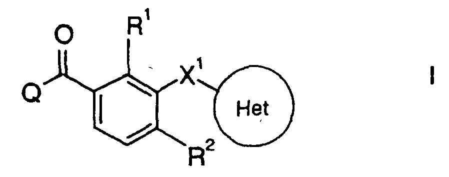 in der die Substituenten folgende Bedeutung haben:
R¹ Chlor oder Methyl;
R² Chlor, Methylsulfonyl oder Trifluormethyl;
Q ein in 4-Stellung verknüpftes Pyrazol der Formel II, 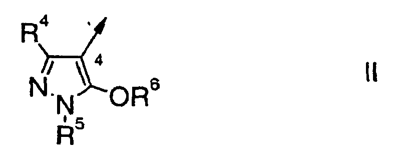 wobei
R⁴ für Wasserstoff;
R⁵ für Methyl,Ethyl oder n-Propyl;
R⁶ für Wasserstoff, Methyl, Ethyl, Methylcarbonyl, Ethylcarbonyl, Methylsulfonyl, Ethylsulfonyl oder 4-Methylphenylsulfonyl;
stehen;
X¹ CH₂, CHCH₃, CHOH, CHOCH₃, CHOCOCH₃, CHOSO₂CH₃, CH₂-CH₂ oder -C≡C-;
Het Oxiranyl, 3-Methyl-2-oxiranyl, 2-Oxetanyl, 3-Hydroxy-3-methyl-2-oxetanyl, 3-Hydroxy-3-ethyl-2-oxetanyl, 3-Hydroxy-3-propyl-2-oxetanyl, 3-Hydroxy-3-buthyl-2-oxetanyl, 3-Methoxy-3-methyl-2-oxetanyl, 3-Methoxy-3-ethyl-2-oxetanyl, 3-Methoxy-3-propyl-2-oxetanyl, 3-Methoxy-3-butyl-2-oxetanyl, 3-Trimethylsilyloxy-3-methyl-2-oxetanyl, 3-Trimethylsilyloxy-3-ethyl-2-oxetanyl, 3-Trimethyl-silyloxy-3-propyl-2-oxetanyl, 3-Trimethylsilyloxy-3-bütyl-2-oxeta- nyl, 3-Oxetanyl, 2-Furyl, 4,5-Dihydro-2-furyl, 2,3-Dihydro-2-furyl, 3-Furyl, 4,5-Dihydro-3-furyl, 2,3-Dihydro-3-furyl, 2-Thienyl, 4,5-Dihydro-2-thienyl, 2,3-Dihydro-2-thienyl, 5-Chlor-2-thienyl, 5-Methyl-2-thienyl, 3-Thienyl, 4,5-Dihydro-3-thienyl, 2,3-Dihydro-3-thienyl, 2-Pyrrolyl, 2,5-Dihydro-2-pyrrolyl, 3-Pyrrol, 2,5-Dihydro-3-pyrrolyl, 3-Isoxazolyl, 4-Methyl-3-isoxazolyl, 5-Methyl-3-isoxazolyl, 4,5-Dimethyl-3-isoxazolyl, 4,5-Dihydro-3-isoxazolyl, 4-Methyl-4,5-dihydro-3-isoxazolyl, 5-Methyl-4,5-dihydro-3-isoxazolyl, 4,5-Dimethyl-4,5-dihydro-3-isoxazolyl, 4-Isoxazolyl, 3-Methyl-4-isoxazolyl, 5-Methyl-4-isoxazolyl, 5-Cyclopropyl-4-isoxazolyl, 5-Phenyl-4-isoxazolyl, 3,5-Dimethyl-4-isoxazolyl, 4,5-Dihydro-4-isoxazolyl, 3-Methyl-4,5-dihydro-4-isoxazolyl, 5-Methyl-4,5-dihydro-4-isoxazolyl, 3,5-Dimethyl-4,5-dihydro-4-isoxazolyl, 5-Isoxazolyl, 3-Methyl-5-isoxazolyl, 4-Methyl-5-isoxazolyl, 3,4-Dimethyl-5-isoxazolyl, 4,5-Dihydro-5-isoxazolyl, 3-Methyl-4,5-dihydro-5-isoxazolyl, 4-Methyl-4,5-dihydro-5-isoxazolyl, 3,4-Dimethyl-4,5-dihydro-5-isoxazolyl, 3-Isothiazolyl, 4-Methyl-3-isothiazolyl, 5-Methyl-3-isothiazolyl, 4-Isothiazolyl, 3-Methyl-4-isothiazolyl, 5-Methyl-4-isothiazolyl, 5-Isothiazolyl, 3-Methyl-5-isothiazolyl, 4-Methyl-5-isothiazolyl, 2-Oxazolyl, 4-Oxazolyl, 5-Oxazolyl, 2-Thiazolyl, 4-Thiazolyl, 5-Thiazolyl, 3-Pyrazolyl, 4-Pyrazolyl, 1-Methyl-3-pyrazolyl, 1-Methyl-4-pyrazolyl, 1-Methyl-5-pyrazolyl, 2-Imidazolyl, 1-Methyl-2-imidazolyl, 5-Methyl-[1,3,4]-2-oxadiazolyl, 5-Methyl-[1,2,4]-3-oxadiazolyl, 5-Methyl-[1,3,4]-2-thiadiazolyl, 5-Methyl-[1,2,4]-3-thiadiazolyl, [1,2,4]-3-Triazolyl, [1,2,3]-4-Triazolyl, 2-Pyridyl, 6-Chlor-2-pyridyl, 6-Methoxy-2-pyridyl, 6-Trifluormethyl-2-pyridyl, 3-Pyridyl, 2-Chlor-3-pyridyl, 2-Methoxy-3-pyridyl, 4-Pyridyl, 2-Chlor-4-pyridyl, 2-Methoxy-4-pyridyl, 2-Ethoxy-4-pyridyl, 2-Methylthio-4-pyridyl, 2-Trifluormethyl-5-pyridyl, 2-Pyrimidinyl, 3-Pyrimidinyl, 4-Pyrimidinyl, 2-Pyrazinyl, 3-Pyridazinyl, 4-Pyridazinyl, 2-(2*H*-1,3-Oxazinyl), 2-(6*H*-1,3-Oxazinyl), 4-(6*H*-1,3-Oxazinyl), 6-(6*H*-1,3-Oxazinyl), [1,3,5]-2-Triazinyl, [1,2,4]-3-Triazinyl, [1,2,4]-5-Triazinyl, [1,2,4]-6-Triazinyl;
sowie deren landwirtschaftlich brauchbaren Salze.

2. Verfahren zur Herstellung von 4-Benzoyl-pyrazolen der Formel I gemäß Anspruch 1, **dadurch gekennzeichnet, daß** man ein Pyrazol der Formel IIa, in der die Substituenten R⁵ und R⁴ die unter Anspruch 1 genannte Bedeutung haben, 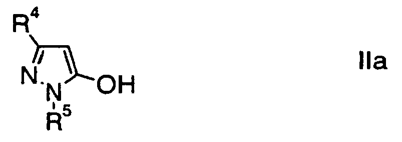 mit einer aktivierten Carbonsäure IIIa oder mit einer Carbonsäure IIIb, 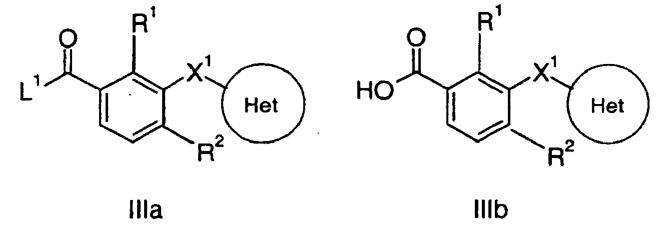 wobei die Substituenten R¹, R², X¹ und Het die in Anspruch 1 genannte Bedeutung haben und L¹ für Alkoxy, Phenoxy, Alkylthio, Phenylthio, Halogen, über N-gebundenes Heteroaryl, Amino oder Imino steht, acyliert und das Acylierungsprodukt gegebenenfalls in Gegenwart eines Katalysators zu den Verbindungen I umlagert und falls gewünscht zur Herstellung von 4-Benzoyl-pyrazolen der allgemeinen Formel I mit R⁶ ≠ H mit einer Verbindung der Formel IV,
L²-R⁶ IV
(mit R⁶≠ H)
in der R⁶ die unter Anspruch 1 genannte Bedeutung hat mit Ausnahme von Wasserstoff und L² für Halogen, Hetaryl, Carboxylat oder Sulfonat steht, umsetzt.

3. Carbonsäurederivate der Formel IIIa und Carbonsäuren der Formel IIIb gemäß Anspruch 2, wobei die Substituenten R¹, R², X¹ und Het die in Anspruch 1 genannte Bedeutung haben und L¹ für Alkoxy, Phenoxy, Alkylthio, Phenylthio, Halogen, über N-gebundenes Heteroaryl, Amino, Imino steht.

4. Mittel, enthaltend eine herbizid wirksame Menge mindestens eines 4-Benzoyl-pyrazols der Formel I oder eines landwirtschaftlich brauchbaren Salzes von I gemäß Anspruch 1 und für die Formulierung von Pflanzenschutzmitteln übliche Hilfsmittel.

5. Verfahren zur Herstellung von herbizid wirksamen Mitteln gemäß Anspruch 2, **dadurch gekennzeichnet, daß** man eine herbizid wirksame Menge mindestens eines 4-Benzoyl-pyrazols der Formel I oder eines landwirtschaftlich brauchbaren Salzes von I gemäß Anspruch 1 und für die Formulierung von Pflanzenschutzmitteln übliche Hilfsmittel mischt.

6. Verfahren zur Bekämpfung von unerwünschtem Pflanzenwuchs, **dadurch gekennzeichnet, daß** man eine herbizid wirksame Menge mindestens eines 4-Benzoyl-pyrazols der Formel I oder eines landwirtschaftlich brauchbaren Salzes von I gemäß Anspruch 1 auf Pflanzen, deren Lebensraum und/oder auf Samen einwirken läßt.

7. Verwendung der 4-Benzoyl-pyrazole der Formel I und deren landwirtschaftlich brauchbaren Salze gemäß Anspruch 1 als Herbizide.

## Revendications

1. 4-Benzoyl-pyrazoles de la formule I : 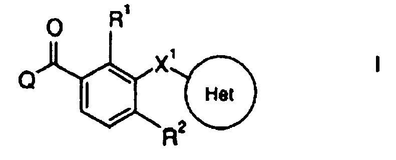 dans laquelle les substituants ont la signification suivante :
R ¹ est du chlore ou du méthyle,
R² est du chlore, du méthylsulfonyle ou du trifluorométhyle,
Q est un pyrazole de la formule II, enchaîné en position 4, 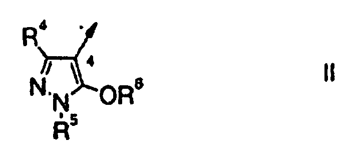 où
R⁴représente de l'hydrogène,
R⁵représente du méthyle, de l'éthyle ou du n-propyle,
R⁶ représente de l'hydrogène ou un groupe méthyle, éthyle, méthylcarbonyle, éthylcarbonyle, méthylsulfonyle, éthylsulfonyle ou 4-méthylphénylsulfonyle,
X¹ représente CH₂, CHCH₃, CHOH, CHOCH₃, CHOCOCH₃, CHOSO₂-CH₃, CH₂-CH₂ ou -C≡C-,
Het représente un groupe oxiranyle, 3-méthyl-2-oxiranyle, 2-oxétanyle, 3-hydroxy-3-méthyl-2-oxétanyle, 3-hydroxy-3-éthyl-2-oxétanyle, 3-hydroxy-3-propyl-2-oxétanyle, 3-hydroxy-3-butyl-2-oxétanyle, 3-méthoxy-3-méthyl-2-oxétanyle, 3-méthoxy-3-éthyl-2-oxétanyle, 3-méthoxy-3-propyl-2-oxétanyle, 3-méthoxy-3-butyl-2-oxétanyle, 3-triméthylsilyloxy-3-méthyl-2-oxétanyle, 3-triméthylsilyloxy-3-éthyl-2-oxétanyle, 3-triméthylsilyloxy-3-propyl-2-oxétanyle, 3-triméthylsilyloxy-3-butyl-2-oxétanyle, 3-oxétanyle, 2-furyle, 4,5-dihydro-2-furyle, 2,3-dihydro-2-furyle, 3-furyle, 4,5-dihydro-3-furyle, 2,3-dihydro-3-furyle, 2-thiényle, 4,5-dihydro-2-thiényle, 2,3-dihydro-2-thiényle, 5-chloro-2-thiényle, 5-méthyl-2-thiényle, 3-thiényle, 4,5-dihydro-3-thiényle, 2,3-dihydro-3-thiényle, 2-pyrrolyle, 2,5-dihydro-2-pyrrolyle, 3-pyrrole, 2,5-dihydro-3-pyrrolyle, 3-isoxazolyle, 4-méthyl-3-isoxazolyle, 5-méthyl-3-isoxazolyle, 4,5-diméthyl-3-isoxazolyle, 4,5-dihydro-3-isoxazolyle, 4-méthyl-4,5-dihydro-3-isoxazolyle, 5-méthyl-4,5-dihydro-3-isoxazolyle, 4,5-diméthyl-4,5-dihydro-3-isoxazolyle, 4-isoxazolyle, 3-méthyl-4-isoxazolyle, 5-méthyl-4-isoxazolyle, 5-cyclopropyl-4-isoxazolyle, 5-phényl-4-isoxazolyle, 3,5-diméthyl-4-isoxazolyle, 4,5-dihydro-4-isoxazolyle, 3-méthyl-4,5-dihydro-4-isoxazolyle, 5-méthyl-4,5-dihydro-4-isoxazolyle, 3,5-diméthyl-4,5-dihydro-4-isoxazolyle, 5-isoxazolyle, 3-méthyl-5-isoxazolyle, 4-méthyl-5-isoxazolyle, 3,4-diméthyl-5-isoxazolyle, 4,5-dihydro-5-isoxazolyle, 3-méthyl-4,5-dihydro-5-isoxazolyle, 4-méthyl-4,5-dihydro-5-isoxazolyle, 3,4-diméthyl-4,5-dihydro-5-isoxazolyle, 3-isothiazolyle, 4-méthyl-3-isothiazolyle, 5-méthyl-3-isothiazolyle, 4-isothiazolyle, 3-méthyl-4-isothiazolyle, 5-méthyl-4-isothiazolyle, 5-isothiazolyle, 3-méthyl-5-isothiazolyle, 4-méthyl-5-isothiazolyle, 2-oxazolyle, 4-oxazolyle, 5-oxazolyle, 2-thiazolyle, 4-thiazolyle, 5-thiazolyle, 3-pyrazolyle, 4-pyrazolyle, 1-méthyl-3-pyrazolyle, 1-méthyl-4-pyrazolyle, 1-méthyl-5-pyrazolyle, 2-imidazolyle, 1-méthyl-2-imidazolyle, 5-méthyl-[1,3,4]-2-oxadiazolyle, 5-méthyl-[1,2,4]-2-oxadiazolyle, 5-méthyl-[1,3,4]-2-thiadiazolyle, 5-méthyl-[1,2,4]-3-thiadiazolyle, [1,2,4]-3-triazolyle, [1,2,3]-4-triazolyle, 2-pyridyle, 6-chloro-2-pyridyle, 6-méthoxy-2-pyridyle, 6-trifluorométhyl-2-pyridyle, 3-pyridyle, 2-chloro-3-pyridyle, 2-méthoxy-3-pyridyle, 4-pyridyle, 2-chloro-4-pyridyle, 2-méthoxy-4-pyridyle, 2-éthoxy-4-pyridyle, 2-méthylthio-4-pyridyle, 2-trifluorométhyl-5-pyridyle, 2-pyrimidinyle, 3-pyrimidinyle, 4-pyrimidinyle, 2-pyrazinyle, 3-pyridazinyle, 4-pyridazinyle, 2-(2H-1,3-oxazinyle), 2-(6H-1,3-oxazinyle), 4-(6H-1,3-oxazinyle), 6-(6H-1,3-oxazinyle), [1,3,5]-2-triazinyle, [1,2,4]-3-triazinyle, [1,2,4]-5-triazinyle, [1,2,4]-6-triazinyle,
ainsi que leurs sels utilisables en agriculture.

2. Procédé de préparation de 4-benzoyl-pyrazoles de la formule I suivant la revendication 1, **caractérisé en ce qu'**on acyle un pyrazole de la formule IIa, dans laquelle les substituants R⁵ et R⁴ ont la signification donnée dans la revendication 1 : 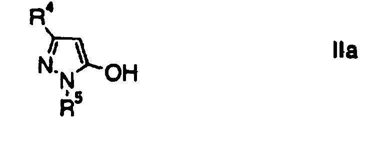 avec un acide carboxylique activé IIIa ou un acide carboxylique IIIb : 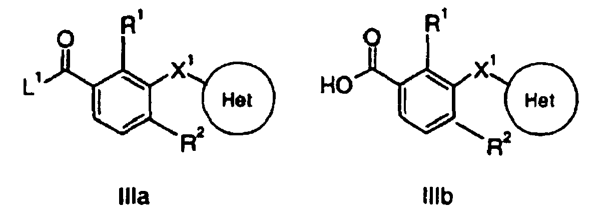 où les substituants R¹, R², X¹ et Het ont la signification donnée dans la revendication 1 et L¹ représente un groupe alcoxy, phénoxy, alkylthio, phénylthio, halogène, hétéroaryle lié par du N, amino ou imino, et on transpose le produit d'acylation, éventuellement en présence d'un catalyseur, en les composés I et, dans le cas où cela est souhaité pour la préparation de 4-benzoyl-pyrazoles de la formule générale I où R⁶ ≠ H, on les fait réagir avec un composé de la formule IV :
L² ― R⁶ IV
(avec R⁶ ≠ H)
dans laquelle R⁶ a la signification donnée dans la revendication 1, à l'exception de l'hydrogène, et L² représente de l'halogène ou un groupe hétaryle, carboxylate ou sulfonate.

3. Dérivés d'acide carboxylique de la formule IIIa et acides carboxyliques de la formule IIIb suivant la revendication 2, dans lesquels les substituants R¹, R², X¹ et Het ont la signification donnée dans la revendication 1 et L¹ représente un groupe alcoxy, phénoxy, alkylthio, phénylthio, halogène, hétéroaryle lié par du N, amino, imino.

4. Produit contenant une quantité efficace du point de vue herbicide d'au moins un 4-benzoyl-pyrazole de la formule I ou d'un sel utilisable en agriculture de I suivant la revendication 1 et des adjuvants courants pour la formulation de produits phytosanitaires.

5. Procédé de préparation de produits efficaces du point de vue herbicide suivant la revendication 2, **caractérisé en ce qu'**on mélange une quantité efficace du point de vue herbicide d'au moins un 4-benzoyl-pyrazole de la formule I ou d'un sel utilisable en agriculture de I suivant la revendication 1 et des adjuvants courants pour la formulation de produits phytosanitaires.

6. Procédé pour lutter contre la croissance non désirée de plantes, **caractérisé en ce qu'**on laisse agir une quantité efficace du point de vue herbicide d'au moins un 4-benzoyl-pyrazole de la formule I ou d'un sel utilisable en agriculture de I suivant la revendication 1 sur des plantes, leur environnement et/ou sur des semences.

7. Utilisation des 4-benzoyl-pyrazoles de la formule I et de leurs sels utilisables en agriculture suivant la revendication 1, comme herbicides.

## Claims

1. A 4-benzoylpyrazole of the formula I 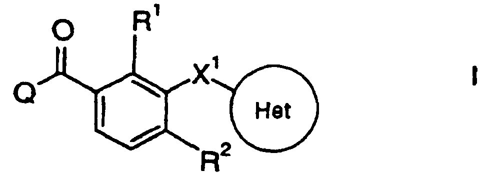 in which the substituents have the following meaning:
R¹ is chlorine or methyl;
R² is chlorine, methylsulfonyl or trifluoromethyl;
Q is a pyrazole of the formula II which is attached in position 4, 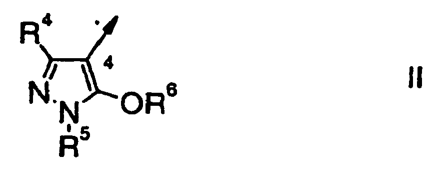 where
R⁴ is hydrogen;
R⁵ is methyl, ethyl or n-propyl;
R⁶ is hydrogen, methyl, ethyl, methylcarbonyl, ethylcarbonyl, methylsulfonyl, ethylsulfonyl or 4-methylphenylsulfonyl;
X¹ is CH₂, CHCH₃, CHOH, CHOCH₃, CHOCOCH₃, CHOSO₂CH₃, CH₂-CH₂ or -C=C-;
Het is oxiranyl, 3-methyl-2-oxiranyl, 2-oxetanyl, 3-hydroxy-3-methyl-2-oxetanyl, 3-hydroxy-3-ethyl-2-oxetanyl, 3-hydroxy-3-propyl-2-oxetanyl, 3-hydroxy-3-butyl-2-oxetanyl, 3-methoxy-3-methyl-2-oxetanyl, 3-methoxy-3-ethyl-2-oxetanyl, 3-methoxy-3-propyl-2-oxetanyl, 3-methoxy-3-butyl-2-oxetanyl, 3-trimethylsilyloxy-3-methyl-2-oxetanyl, 3-trimethylsilyloxy-3-ethyl-2-oxetanyl, 3-trimethylsilyloxy-3-propyl-2-oxetanyl, 3-trimethylsilyloxy-3-butyl-2-oxetanyl, 3-oxetanyl, 2-furyl, 4,5-dihydro-2-furyl, 2,3-dihydro-2-furyl, 3-furyl, 4,5-dihydro-3-furyl, 2,3-dihydro-3-furyl, 2-thienyl, 4,5-dihydro-2-thienyl, 2,3-dihydro-2-thienyl, 5-chloro-2-thienyl, 5-methyl-2-thienyl, 3-thienyl, 4,5-dihydro-3-thienyl, 2,3-dihydro-3-thienyl, 2-pyrrolyl, 2,5-dihydro-2-pyrrolyl, 3-pyrrole, 2,5-dihydro-3-pyrrolyl, 3-isoxazolyl, 4-methyl-3-isoxazolyl, 5-methyl-3-isoxazolyl, 4,5-dimethyl-3-isoxazolyl, 4,5-dihydro-3-isoxazolyl, 4-methyl-4,5-dihydro-3-isoxazolyl, 5-methyl-4,5-dihydro-3-isoxazolyl, 4,5-dimethyl-4,5-dihydro-3-isoxazolyl, 4-isoxazolyl, 3-methyl-4-isoxazolyl, 5-methyl-4-isoxazolyl, 5-cyclopropyl-4-isoxazolyl, 5-phenyl-4-isoxazolyl, 3,5-dimethyl-4-isoxazolyl, 4,5-dihydro-4-isoxazolyl, 3-methyl-4,5-dihydro-4-isoxazolyl, 5-methyl- 4,5-dihydro-4-isoxazolyl, 3,5-dimethyl-4,5-dihydro-4-isoxazolyl, 5-isoxazolyl, 3-methyl-5-isoxazolyl, 4-methyl-5-isoxazolyl, 3,4-dimethyl-5-isoxazolyl, 4,5-dihydro-5-isoxazolyl, 3-methyl-4,5-dihydro-5-isoxazolyl, 4-methyl-4,5-dihydro-5-isoxazolyl, 3,4-dimethyl-4,5-dihydro-5-isoxazolyl, 3-isothiazolyl, 4-methyl-3-isothiazolyl, 5-methyl-3-isothiazolyl, 4-isothiazolyl, 3-methyl-4-isothiazolyl, 5-methyl-4-isothiazolyl, 5-isothiazolyl, 3-methyl-5-isothiazolyl, 4-methyl-5-iso-thiazolyl, 2-oxazolyl, 4-oxazolyl, 5-oxazolyl, 2-thiazolyl, 4-thiazolyl, 5-thiazolyl, 3-pyrazolyl, 4-pyrazolyl, 1-methyl-3-pyrazolyl, 1-methyl-4-pyrazolyl, 1-methyl-5-pyrazolyl, 2-imidazolyl, 1-methyl-2-imidazolyl, 5-methyl-[1,3,4]-2-oxadiazolyl, 5-methyl-[1,2,4]-3-oxadiazolyl, 5-methyl-[1,3,4]-2-thiadiazolyl, 5-methyl-[1,2,4]-3-thiadiazolyl, [1,2,4]-3-triazolyl, [1,2,3]-4-triazolyl, 2-pyridyl, 6-chloro-2-pyridyl, 6-methoxy-2-pyridyl, 6-trifluoromethyl-2-pyridyl, 3-pyridyl, 2-chloro-3-pyridyl, 2-methoxy-3-pyridyl, 4-pyridyl, 2-chloro-4-pyridyl, 2-methoxy-4-pyridyl, 2-ethoxy-4-pyridyl, 2-methylthio-4-pyridyl, 2-trifluoromethyl-5-pyridyl, 2-pyrimidinyl, 3-pyrimidinyl, 4-pyrimidinyl, 2-pyrazinyl, 3-pyridazinyl, 4-pyridazinyl, 2-(2*H*-1,3-oxazinyl), 2-(6*H*-1,3-oxazinyl), 4-(6*H*-1,3-oxazinyl), 6-(6*H*-1,3-oxazinyl), [1,3,5]-2-triazinyl, [1,2,4]-3-triazinyl, [1,2,4]-5-triazinyl, [1,2,4]-6-triazinyl;
or an agriculturally useful salt thereof.

2. A process for preparing 4-benzoylpyrazoles of the formula I according to claim 1, which comprises acylating a pyrazole of the formula IIa in which the substituents R⁵ and R⁴ are each as defined under claim 1 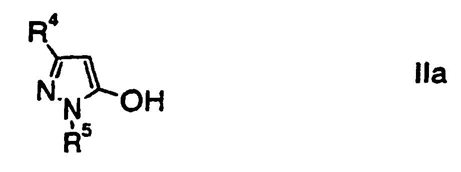 with an activated carboxylic acid IIIa or with a carboxylic acid IIIb, 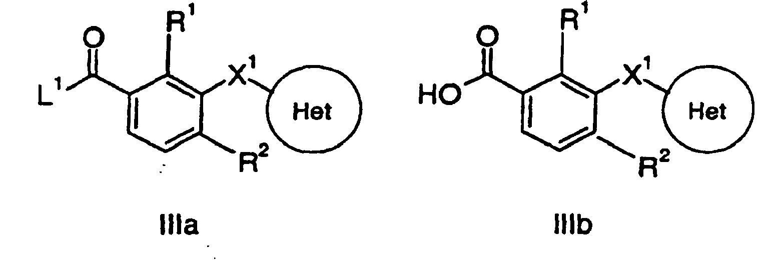 where the substituents R¹, R², X¹ and Het are each as defined in claim 1 and L¹ is alkoxy, phenoxy, alkylthio, phenylthio, halogen, heteroaryl bonded via N, amino or imino, and rearranging the acylation product, if appropriate in the presence of a catalyst, to the compounds I and, if desired, reacting it with a compound of the formula IV, 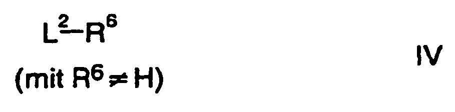 in which R⁶ is as defined in claim 1, except for hydrogen, and L² is halogen, hetaryl, carboxylate or sulfonate, for preparing 4-benzoylpyrazoles of the formula I where R⁶ ≠ H.

3. A carboxylic acid derivative of the formula IIIa or a carboxylic acid of the formula IIIb according to claim 2, where the substituents R¹, R², X¹ and Het are each as defined in claim 1 and L¹ is alkoxy, phenoxy, alkylthio, phenylthio, halogen, heteroaryl bonded via N, amino or imine.

4. A composition comprising a herbicidally effective amount of at least one 4-benzoylpyrazole of the formula I or an agriculturally useful salt of I according to claim 1 and auxiliaries customary for the formulation of crop protection agents.

5. A process for preparing herbicidally effective compositions according to claim 2, which comprises mixing a herbicidally effective amount of at least one 4-benzoylpyrazole of the formula I or an agriculturally useful salt of I according to claim 1 and auxiliaries customary for the formulation of crop protection agents.

6. A method for controlling undesirable vegetation, which comprises allowing a herbicidally effective amount of at least one 4-benzoylpyrazole of the formula I or an agriculturally useful salt of I according to claim 1 to act on plants, their habitat and/or on seeds.

7. The use of the 4-benzoylpyrazoles of the formula I and agriculturally useful salts thereof according to claim 1 as herbicides.
